# EUROPEAN PATENT SPECIFICATION

(11) **EP 2 991 975 B1**
(45) Date of publication and mention of the grant of the patent: **19.07.2017**
(21) Application number: 14728327.9
(22) Date of filing: 29.04.2014
(51) Int. Cl.: C07D 401/04, A61K 31/4439, A61K 31/4406, A61K 31/4427, A61P 29/00, A61P 17/00, A61P 35/00, A61P 3/10

(54) **INHIBITORS OF NICOTINAMIDE PHOSPHORIBOSYLTRANSFERASE, COMPOSITIONS, PRODUCTS AND USES THEREOF**
HEMMER DES ENZYMS NIKOTINAMID-PHOSPHORIBOSYLTRANSFERASE, ZUSAMMENSETZUNGEN SOWIE PRODUKTE UND IHRE VERWENDUNG
INHIBITEURS DE NICOTINAMIDE PHOSPHORIBOSYLTRANSFÉRASE, COMPOSITIONS ET PRODUITS ET UTILISATIONS DE CELLES-CI

(30) Priority: 03.05.2013 IT TO20130356
(43) Date of publication of application: 09.03.2016
(73) Proprietor: Università degli Studi del Piemonte Orientale "Amedeo Avogadro", 13100 Vercelli (IT)
(72) Inventor: GENAZZANI, Armando A., 28100 Novara (IT); TRON, Gian Cesare, 28060 San Pietro Mosezzo (Novara) (IT); GALLI, Ubaldina, 10090 Castagneto Po (Torino) (IT); TRAVELLI, Cristina, 28047 Oleggio (Novara) (IT); CUZZOCREA, Salvatore, 98100 Messina (IT); GROSA, Giorgio, 10146 Torino (IT); SORBA, Giovanni, 10090 San Giusto Canavese (Torino) (IT); CANONICO, Pier Luigi, 28100 Novara (IT)
(74) Representative: Freyria Fava, Cristina
(86) International application number: PCT/IB2014/061080
(87) International publication number: WO 2014/178001

(56) References cited:
- GIAMPIERO COLOMBANO ET AL: "A novel potent nicotinamide phosphoribosyltransferase inhibitor synthesized via click chemistry", JOURNAL OF MEDICINAL CHEMISTRY, AMERICAN CHEMICAL SOCIETY, US, vol. 53, no. 2, 1 January 2010 (2010-01-01), pages 616-623, XP002638251, ISSN: 0022-2623, DOI: 10.1021/JM9010669 [retrieved on 2009-12-04] cited in the application
- OLESEN ET AL: "Anticancer agent CHS-828 inhibits cellular synthesis of NAD", BIOCHEMICAL AND BIOPHYSICAL RESEARCH COMMUNICATIONS, ACADEMIC PRESS INC. ORLANDO, FL, US, vol. 367, no. 4, 15 January 2008 (2008-01-15), pages 799-804, XP022449876, ISSN: 0006-291X, DOI: 10.1016/J.BBRC.2008.01.019
- HASMANN MAX ET AL: "FK866, a highly specific noncompetitive inhibitor of nicotinamide phosphoribosyltransferase, represents a novel mechanism for induction of tumor cell apoptosis", CANCER RESEARCH, AMERICAN ASSOCIATION FOR CANCER RESEARCH, US, vol. 63, no. 21, 1 November 2003 (2003-11-01), pages 7436-7442, XP002432917, ISSN: 0008-5472

## Description

### Field of the invention

This disclosure concerns new compounds able to inhibit nicotinamide phosphoribosyltransferase (NAMPT).

The disclosure also relates to the use of these new compounds for treatment of pathological conditions in which NAMPT inhibition might be beneficial, such as acute and chronic inflammation, cancer and metabolic disorders.

### Background of the invention

A number of metabolic pathways leading to the formation of nicotinamide adenine dinucleotide (NAD) are present in cells. The principal precursors of NAD are nicotinic acid/nicotinamide, tryptophan, and the recently described nicotinamide riboside. Furthermore, a rescue mechanism exists in cells to re-use nicotinamide released by NAD-metabolizing enzymes. This pathway, known as the "NAD salvage pathway" is the quantitatively more important mechanism of NAD synthesis in mammalian cells and is schematically composed of two enzymes: nicotinamide phosphoribosyltransferase (NAMPT) and nicotinamide mononucleotide adenylyltransferase (NMNAT) which leads to NAD from NMN and ATP.

NAMPT is a homodimeric enzyme that catalyzes the synthesis of nicotinamide mononucleotide (NMN) from nicotinamide (NM) and 5'-phosphoribosyl-1'-pyrophosphate (PRPP), thus playing an important role in the cyclic biosynthetic pathway of NAD.

NAD is an essential cofactor in a number of fundamental intracellular processes, including cellular respiration, by virtue its ability to transfer electrons during redox reactions, to modulate the activity of key regulators of cellular longevity, and to serve as a substrate for the generation of other biologically important molecules. Indeed, NAD is a substrate for poly and mono-ADP-ribosylation reactions, acetylation reactions, as well as being the precursor for a number of molecules involved in calcium signaling (*e*.g. ADPR, cADPR, NAADP). Furthermore, the possibility that NAD (or precursors and metabolites) can also have a role in the extracellular space has been postulated by a number of Authors. Indeed, NAD can be detected at nanomolar levels in human serum, and there is increasing evidence that extracellular NAD can modulate the function of the innate immune system. (Billington et al. Mol. Med. 2006, 12, 324-7; Imai, Curr. Pharm. Des. 2009, 15. 20-8). Indeed, NAMPT is also known as visfatin when it is secreted and present in plasma.

In this respect, intracellular or extracellular NAMPT-dependent recycling of nicotinamide to NAD may represent a physiologically important homeostatic mechanism to avoid depletion of intracellular NAD pool during its active use as a substrate. Indeed increased levels of NAMPT reflect the increased activity of several NAD-degrading enzymes, thus providing a molecular link between NAD metabolism and the regulation of aspects of cell physiology. In line with this hypothesis, it has been shown that NAMPT activity is able to enhance cellular proliferation and to tip the balance toward cell survival following genotoxic insult. Moreover, NAMPT plays a central role in controlling circadian clock machinery, by dictating the periodical oscillations of some of its key transcription factors.

NAMPT has also been identified as an extracellular proinflammatory cytokine, known as pre-B-cell colony-enhancing factor (PBEF), a growth factor for early stage B cells able to promote myeloid and lymphoid differentiation and to induce cellular expression of inflammatory cytokines such as TNF-α, IL-1β and IL-6. NAMPT has also been shown to be functional on other cells of the immune system. This suggests that NAMPT, either via the "NAD salvage pathway" or by other mechanisms, may modulate innate or acquired immune functions.

Moreover, NAMPT has recently further been identified as an adipokine, known as visfatin, a cytokine that is secreted from visceral fat tissues that is able to regulate a variety of physiological and pathological processes, including immunity and inflammation. Furthermore, visfatin has been postulated to act on the insulin receptor (*IR*), although at a site different from insulin, and to exert a insulin-mimetic effects, including enhancing glucose uptake and increasing triglyceride synthesis. Recently, several reports showed that visfatin is involved in thrombin-induced lung endothelial barrier dysregulation and promotes the efficient acquisition of a mature smooth muscle cell phenotype that is essential for remodelling of blood vessel, indicating a possible role in vascular homeostasis.

Emerging data implicate PBEF/NAMPT/Visfatin in the pathogenesis of a number of different human diseases, in particular in the field of cancer and inflammation. Alterations of NAMPT expression and/or activity have been shown in inflammatory disease, including chronic diseases such as rheumatoid arthritis (RA) and Type 2 diabetes and also acute, life-threatening processes such as acute lung injury and sepsis. It was also suggested that NAMPT has potential implications in the pathogenesis of acute lung injury, spinal cord injury, Crohn's disease (CD) and ulcerative colitis (UC). Indeed, a recent study showed elevated plasma visfatin levels also in patients with type 2 diabetes mellitus (Sethi, Curr. Hypertens Rep. 2007, 9, 33-8). Furthermore, NAMPT is upregulated during activation of immune cells such as monocytes, macrophages, dendritic cells, T cells and B cells (Rongvaux et al. Eur. J. Immunol. 2002, 32, 3225-34). A cytokine-like activity of visfatin has been implicated also in the pathogenesis of unstable atherosclerosis. Indeed, visfatin expression is up-regulated in plaque from the cariotid artery of patients with symptoms of stroke and with acute myocardial infarction, suggesting that visfatin plays an important role also in atherogenesis and plaque destabilization. Furthermore, recent studies demonstrated that increase visfatin expression and plasma levels of visfatin are associated with obesity in animals and humans.

Furthermore, NAMPT has also been implicating in tumorigenesis. The link between NAMPT and cancer therapy is rapidly strengthening. First, NAMPT has been shown to be up-regulated in a number of solid and liquid tumours (Bi and Che, Cancer Biol. Ther. 2010, 10, 119-25), including Prostate cancer, ovarian cancer, lung cancer, breast cancer, colon cancer, pancreatic adenocarcinoma, melanoma, neuroblastoma, leukemia and Lymphoma. Second, NAMPT has been shown to be involved in angiogenesis by inducing VEGF and MMP2/9 expression. Moreover the human nampt gene is transcriptionally activated in response to hypoxia, which can mainly trigger molecular determinants involved in different steps of angiogenesis (Drevs et al., Anticancer Res. 2003, 23, 4853-58; Kim et al., Biochem. Biophys. Res. Commun. 2007, 357,150-156). NAMPT is also able to increase TNF-α release, this increase in TNF-α in the tumour microenvironment is likely to stimulate tumour growth thus promoting cancer cells proliferation. Third, an important role in tumorigenesis or tumour treatment has been postulated or proven for a number of NAD-utilizing enzymes (*e*.g. PARP, sirtuins; Rouleau et al., Nat. Rev. Cancer 2010, 10,293-301). Finally, most cancer cells predominantly produce energy by a high rate of glycolysis followed by lactic acid fermentation in the cytosol, rather than by a comparatively low rate of glycolysis followed by oxidation of pyruvate in mitochondria like most normal cells. Intracellular NAMPT is mainly expressed in the cytosolic compartment rather than in the mitochondrial compartment, suggesting that NAMPT may play an important role in energy production and thus in tumoral cells proliferation.

In this respect, two inhibitors of NAMPT have been identified named FK866 (also known as APO866) and CHS828. These compounds are able to inhibit NAMPT activity by binding the enzyme in the dimer interface. The crystal structures of the complexes with either NMN or FK866 have shown that the enzymatic active site of NAMPT is optimized for nicotinamide binding and that the nicotinamide-binding site is important for inhibition of FK866 (Kang et al. Mol. Cells 2009, 27,667-71).

These compounds have potent antitumoral activity *in vitro* and *in vivo* by inducing cancer cell death, apoptosis or autophagy depending on cell type (Hasmann and Schemainda, Cancer Res. 2003, 63,7436-42; Olesen et al., Biochem. Biophys Res. Commun. 2008, 367, 799-804; Colombano et al. J Med. Chem. 2010, 53:616-23). Indeed, both drugs have entered clinical trials for cancer treatment and are now in phase I/II (www.clinicaltrials.gov). It is now emerging that these agents may also be useful in inflammatory and autoimmune diseases (Busso et al., Plos One 2008, 3,e2267; Bruzzone et al., Plos One 2009, 4,e7897). Indeed, in contrast to other organs, immune cells seem to rely exclusively on the NAMPT salvage pathway for NAD biosynthesis, moreover actively proliferating cells (such as tumours and proliferating immune cells) display increased PARP activity, rendering these cells highly dependent upon NAMPT activity for adequate survival (Rongvaux et al. J. Immunol. 2008, 181,4685-95).

In the light of these evidences, since NAMPT seems to be involved in a large number of pathological conditions, NAMPT inhibitors might be useful in the treatment of a large number of diseases, in particular in cancer, metabolic disease and inflammatory pathologies.

### Summary of the invention

The object of this disclosure is to provide new compounds which exhibit inhibitory activity against nicotinamide phosphoribosyltransferase.

According to the invention, the above object is achieved thanks to the subject matter recalled specifically in the ensuing claims, which are understood as forming an integral part of this disclosure.

The present invention provides a class of 3-((1-substituted)-1*H*-1,2,3-triazol-4-yl)pyridine compounds as a novel class of nicotinamide phosphoribosyl transferase inhibitors and to their use in therapy.

More particularly, the invention provides a family of 1,4-disubstituted triazoles with at 1-position a substituted alkyl/aromatic chain and at 4-position a pyridine ring.

The present disclosure provides a compound of formula (I): 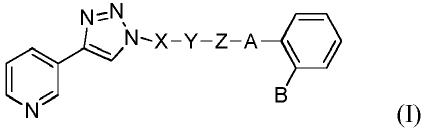 wherein
X is (CH₂)ₙ;
Y is selected from (CH₂)ₘ, *para* substituted phenyl;
Z is (CH₂)ₚ;
n is an integer 0 to 4;
m is an integer 1 to 8;
p is an integer 0 to 4;
A is selected from the following groups: 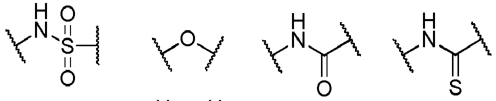 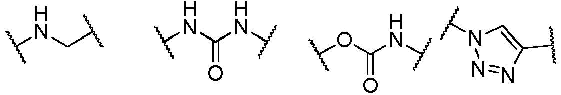
B is selected from Br, Cl, I, F, methyl, isopropyl, tert-butyl, substituted or unsubstituted aryl or heteroaryl (Ar) group,
wherein Ar is selected from benzene, furan, thiophene, pyrrolidine, pyrrole, 1,2,3-triazole, pyrazole, imidazole, oxazole, isooxazole, thiazole, isothiazole, 1,2,3-oxadiazole, 1,2,4-oxadiazole, 1,2,5-oxadiazole, pyridine, pyridazine, pyrimidine, pyrazine, naphthalene, indole, 1*H*-indazole, 1*H*-benzo[d]imidazole, benzofuran, benzothiophene, quinoline, isoquinoline, quinoxaline, or carbazole, and
wherein when B is selected from a substituted aryl or heteroaryl (Ar) group, the one or more substituents are independently selected from -COOH, -NH₂, - COOR¹, -CONH₂, -CONHR¹, -CONR¹R², -R³;
wherein
R¹ and R² are identical or different from each other and independently selected from -H, straight or branched, unsubstituted C₁₋₈ alkyl, unsubstituted C₃₋₆ cycloalkyl,
R³ is selected from straight or branched, unsubstituted C₁₋₈ alkyl, unsubstituted C₃₋₆ cycloalkyl;
pharmaceutically acceptable, hydrate, solvate or salt thereof.

The disclosure relates to 1,4-disubstituted triazoles compounds of formula (I) with a specific nicotinamide phosphoribosyltransferase inhibitory activity and a cytostatic and/or cytotoxic effect on tumor cells.

The disclosure also relates to the use of the 3-((1-substituted)-1*H*-1,2,3-triazol-4-yl)pyridine compounds of formula (I) for *in vivo* treatment of pathological conditions in which NAMPT inhibition might be beneficial, such as acute and chronic inflammation, cancer and metabolic disorders.

The disclosure also provides pharmaceutical compositions comprising at least one 1,4-disubstituted triazoles compound of formula (I) and a pharmaceutically acceptable carrier. The pharmaceutical composition may further comprise one or more additional therapeutic agents.

The therapeutic potential of formula (I) compounds can also be achieved by additive effects with anti-cancer drugs as a combined preparation for simultaneous, separate or sequential use in tumor therapy or additive effects with anti-inflammatory drugs (e.g. methotrexate, corticosteroids). Example of anti-cancer agents which can be advantageously comprised in this preparation are, for example, DNA-alkylating agents (e.g. cis-platin), DNA intercalator agents (e.g. doxorubicin), topoisomerase inhibitors (e.g. etoposide) and also autophagy inhibitors, such as cloroquine and its analogues and PI3K inhibitors.

### Detailed description of the invention

In the following description, numerous specific details are given to provide a thorough understanding of embodiments. The embodiments can be practiced without one or more of the specific details, or with other methods, components, materials, etc. In other instances, well-known structures, materials, or operations are not shown or described in detail to avoid obscuring aspects of the embodiments.

Reference throughout this specification to "one embodiment" or "an embodiment" means that a particular feature, structure, or characteristic described in connection with the embodiment is included in at least one embodiment. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments.

The headings provided herein are for convenience only and do not interpret the scope or meaning of the embodiments.

An embodiment of the present disclosure provides compounds of formula (I): 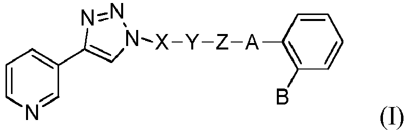 wherein
X is (CH₂)ₙ;
Y is selected from (CH₂)ₘ, *para* substituted phenyl;
Z is (CH₂)ₚ;
n is an integer 0 to 4;
m is an integer 1 to 8;
p is an integer 0 to 4;
A is selected from the following groups: 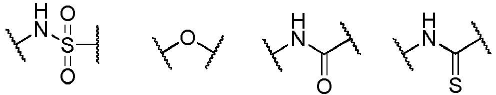 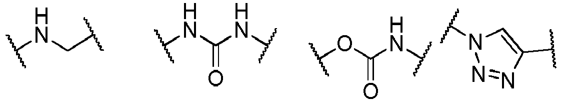
B is selected from Br, Cl, I, F, Methyl, Isopropyl, tert-butyl, substituted or unsubstituted aryl or heteroaryl (Ar) group,
wherein Ar is selected frombenzene, furan, thiophene, pyrrolidine, pyrrole, 1,2,3-triazole, pyrazole, imidazole, oxazole, isooxazole, thiazole, isothiazole, 1,2,3-oxadiazole, 1,2,4-oxadiazole, 1,2,5-oxadiazole, pyridine, pyridazine, pyrimidine, pyrazine, naphthalene, indole, 1*H-*indazole, 1*H-*benzo[d]imidazole, benzofuran, benzothiophene, quinoline, isoquinoline, quinoxaline, or carbazole, and
wherein when B is selected from a substituted aryl or heteroaryl (Ar) group, the one or more substituents are independently selected from -COOH, -NH₂, - COOR¹, -CONH₂, -CONHR¹, -CONR¹R², -R³;
wherein
R¹ and R² are identical or different from each other and independently selected from -H, straight or branched, unsubstituted C₁₋₈ alkyl, unsubstituted C₃₋₆ cycloalkyl,
R³ is selected from straight or branched, unsubstituted C₁₋₈ alkyl, unsubstituted C₃₋₆ cycloalkyl;pharmaceutically acceptable, hydrate, solvate or salt thereof.

In an embodiment, any of R¹, R² and R³ groups, if present, are independently selected from methyl, ethyl, n-propyl, isopropyl, n-butyl, isobutyl, tert-butyl, cyclopropyl, cyclobutyl, cyclopentyl, cyclohexyl, n-pentyl, n-hexyl; n-heptyl,n-octyl.

The presence of a reversed amide or a thioamide linkage or an ether linkage or a triazole linkage or an urea linkage or a carbamate linkage or sulfonamide linkage or a methylen amine linkage in compounds of formula (I), compared with previously reported structures (J. Med. Chem. 2010, 53, 616-623 see for example compound 8), surprisingly generates more metabolically stable analogues avoiding the generation of toxic and suspected cancerogenic aromatic amines.

Compounds of formula (I) herein described are useful for the treatment of disease conditions depending on increased presence or activity of NAMPT. Such diseases have been identified with inflammation, altered metabolism or cancer, or neuroprotection from injury.

Accordingly, the compounds of formula (I) are useful for the prevention or treatment of i) autoimmune diseases, preferably selected from lupus erythematosus, psoriasis, rheumatoid arthritis, Crohn's disease, autoimmune enchephalitis; ii) acute and/or chronic inflammation disorders, preferably selected from ulcerative colitis, asthma, chronic obstructive pulmonary disease (COPD), acute respiratory distress syndrome (ARDS), allergic rhinitis, anaphylaxis, cystic fibrosis, myocardial infarction, heart failure, ischemic diseases and atherosclerosis, acute lung injury, graft vs host disease, spinal cord injury and sepsis; (iii) metabolic-associated disorders such as obesity and type II diabetes. The compounds of the formula (I) are useful for the treatment of tumor and cancer types that have been demonstrated to have increased levels of NAMPT as well as those that have normal levels, as inhibition of this enzyme will result in
reduced energetic currency. The compounds of the formula (I) are useful in both solid and liquid tumours, including Prostate cancer, ovarian cancer, lung cancer, breast cancer, colon cancer, pancreatic adenocarcinoma, melanoma, neuroblastoma, leukemia and Lymphoma.

The therapeutic potential of compound of formula (I) can also be achieved by synergic effects with other drugs. For example, DNA-alkylating agents (such as platin-containing drugs, including cisplatin), DNA intercalator agents (such as doxorubicin) and topoisomerase inhibitors (such as etoposide) can be used as anticancer agents together with the nicotinamide phosphoribosyltransferase inhibitors disclosed herein. Similarly, autophagy inhibitors, such as cloroquine, can also be used to potentiate the therapeutic effect of nicotinamide phosphoribosyltransferase inhibitors disclosed herein in the cancer therapy. Also, immunomodulating drugs, such as 1-methyltryphtophan can also be used to potentiate the therapeutic effect of nicotinamide phosphoribosyltransferase inhibitors disclosed herein in cancer and inflammation therapy.

In clinical practice, in fact, it is frequent to combine different drugs in order to improve the therapeutic effect on the target cells. Thanks to the solution provided herein, it is possible to combine the effect of formula (I) compounds which are able to inhibit the nicotinamide phosphoribosyltransferase activity with the effect of agents which are able, for example, to damage DNA or to inhibit its replication in rapidly growing cancer cells. This solution, therefore, allows to achieve a contemporary action in counteracting the cancer cell proliferation and tumor growth.

Compounds of formula (I) can be administered in various routes appropriate to the condition to be treated.

Suitable routes include oral, parenteral (including subcutaneous, intramuscular, intravenous, intraarterial, intradermal, intrathecal and epidural), transdermal, rectal, nasal, topical (including buccal and sublingual), vaginal, intraperitoneal, intrapulmonary and intranasal. When the compound/s is/are administered orally, it/they may be formulated as pills, tables, capsules, ingested daily or less frequently for a specific period of time.

The dosage depends on a variety of factors including the age, weight and condition of the patient and the route of administration. Although daily dosage can vary from one individual to another, the compound/s will be administered to an adult human in a range of 0.0001-50 mg/kg of body weight as daily single dose or 0.01 to 1 mg/kg as daily repeated doses.

Compounds of formula (I) can be formulated as a pharmaceutical composition in the form of tablet, capsule, aqueous solution, granule, powder, suspension, cream, syrup, gel, emulsion, etc.

Tablets contain the compound/s of formula (I) in a mixture with non-toxic pharmaceutically excipients suitable for the manufacture of tablets. Exemplary excipients could be: inert diluents, such as sodium carbonate, lactose, dextrose, cellulose etc.; granulating and disintegrating agents as maize starch, glycolate, alginic acid; binding agents as gelatin or acacia; lubricating agents, for example silica magnesium or calcium stearate, stearic acid or talc.

For preparing suppositories, a mixture of for example fatty acid glycerides or cocoa butter is first melted and the compound/s of formula (I) is/are dissolved homogenously by stirring. The homogenous mixture is then cooled into convenient sized molds.

Liquid preparations, which include solutions, suspensions and emulsions, contain the formula (I) compound/s in a mixture of excipients suitable for the manufacture of aqueous suspension such as sodium carboxymethylcellulose, methylcellulose, resin, sodium alginate and natural or synthetic gums. Eventually the liquid preparation may contain suitable colorants, flavors, stabilizers, preservatives and thickening agents as desired.

Preferred 1,4-disubstituted triazoles compounds of formula (I) are shown in Table 1.

**Table 1**

| **Name** | **Structure** |
|---|---|
| 3-(1-(8-([1,1'-biphenyl]-2-yloxy)octyl)-1H-1,2,3-triazol-4-yl)pyridine | 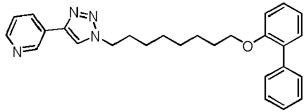 |
| 3-(1-(7-([1,1'-biphenyl]-2-yloxy)heptyl)-1H-1,2,3-triazol-4-yl)pyridine | 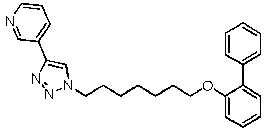 |
| 3-(1-(6-([1,1'-biphenyl]-2-yloxy)hexyl)-1H-1,2,3-triazol-4-yl)pyridine | 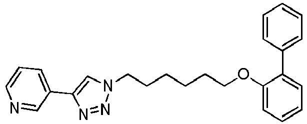 |
| 3-(1-(8-phenoxyoctyl)-1H-1,2,3-triazol-4-yl)pyridine | 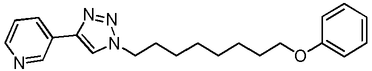 |
| 3-(1-(7-phenoxyheptyl)-1H-1,2,3-triazol-4-yl)pyridine | 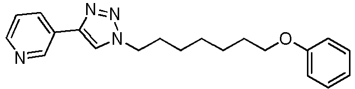 |
| 3-(1-(6-phenoxyhexyl)-1H-1,2,3-triazol-4-yl)pyridine | 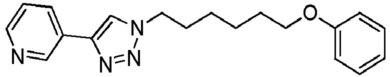 |
| 3-(1-(6-(2-iodophenoxy)hexyl)-1H-1,2,3-triazol-4-yl)pyridine | 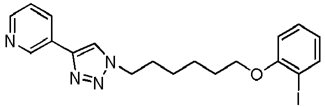 |
| 2'-((6-(4-(pyridin-3-yl)-1H-1,2,3-triazol-1-yl)hexyl)oxy)-[1,1'-biphenyl]-3-carboxylic acid | 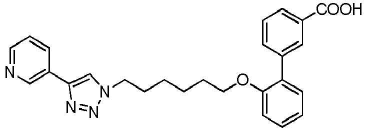 |
| 2'-((6-(4-(pyridin-3-yl)-1H-1,2,3-triazol-1-yl)hexyl)oxy)-[1,1'-biphenyl]-4-carboxylic acid | 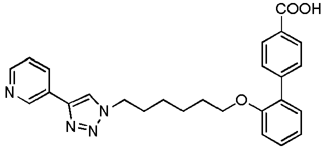 |
| 3-(2-((6-(4-(pyridin-3-yl)-1H-1,2,3-triazol-1-yl)hexyl)oxy)phenyl)pyridine | 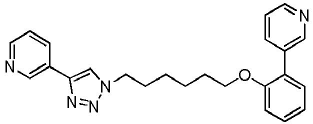 |
| 3-(1-(6-((2'-methyl-[1,1'-biphenyl]-2-yl)oxy)hexyl)-1H-1,2,3-triazol-4-yl)pyridine | 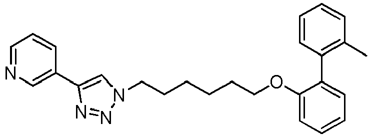 |
| 8-(4-(pyridin-3-yl)-1H-1,2,3-triazol-1-yl)octyl[1,1'-biphenyl]-2-ylcarbamate | 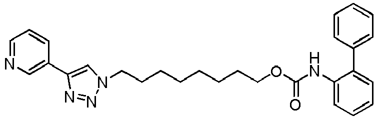 |
| 7-(4-(pyridin-3-yl)-1H-1,2,3-triazol-1-yl)heptyl[1,1'-byphenyl]-2-ylcarbamate | 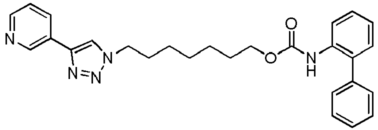 |
| 6-(4-(pyridin-3-yl)-1H-1,2,3-triazol-1-yl)hexyl[1,1'-byphenyl]-2-ylcarbamate | 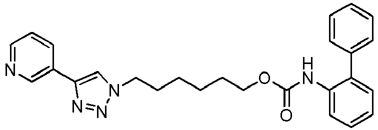 |
| 3-(1-(7-(4-phenyl-1H-1,2,3-triazol-1-yl)heptyl)-1H-1,2,3-triazol-4-yl)pyridine | 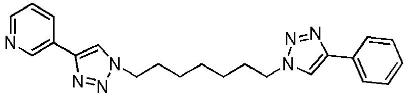 |
| 3-(1-(6-(4-phenyl-1H-1,2,3-triazol-1-yl)hexyl)-1H-1,2,3-triazol-4-yl)pyridine | 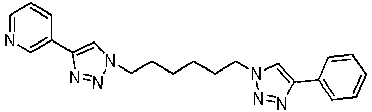 |
| 3-(1-(8-(4-([1,1'-byphenyl]-2-yl)-1H-1,2,3-triazol-1-yl)octyl)-1H-1,2,3-triazol-4-yl)pyridine | 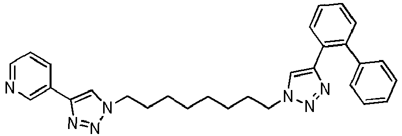 |
| 3-(1-(7-(4-([1,1'-byphenyl]-2-yl)-1H-1,2,3-triazol-1-yl)heptyl)-1H-1,2,3-triazol-4-yl)pyridine | 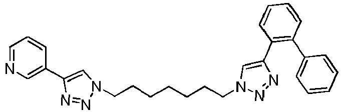 |
| 3-(1-(6-(4-([1,1'-byphenyl]-2-yl)-1H-1,2,3-triazol-1-yl)hexyl)-1H-1,2,3-triazol-4-yl)pyridine | 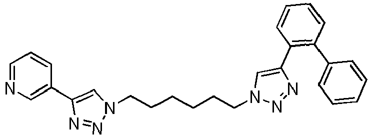 |
| N-(8-(4-(pyridin-3-yl)-1H-1,2,3-triazol-1-yl)octyl)-[1,1'-biphenyl]-2-carboxamide | 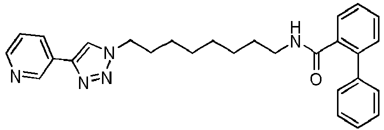 |
| N-(7-(4-(pyridin-3-yl)-1H-1,2,3-triazol-1-yl)heptyl)-[1,1'-biphenyl]-2-carboxamide | 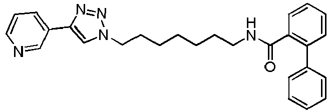 |
| N-(6-(4-(pyridin-3-yl)-1H-1,2,3-triazol-1-yl)hexyl)-[1,1'-biphenyl]-2-carboxamide | 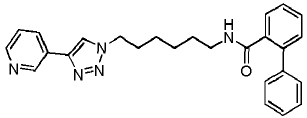 |
| N-(7-(4-(pyridin-3-yl)-1H-1,2,3-triazol-1-yl)heptyl)benzamide | 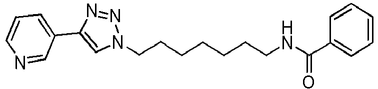 |
| methyl 2'-((7-(4-(pyridin-3-yl)-1H-1,2,3-triazol-1-yl)heptyl)carbamoyl)-[1,1'-biphenyl]-2-carboxylate | 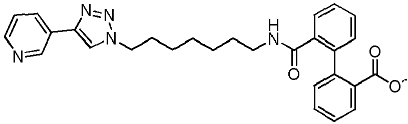 |
| 2'-((7-(4-(pyridin-3-yl)-1H-1,2,3-triazol-1-yl)heptyl)carbamoyl)-[1,1'-biphenyl]-2-carboxylic acid | 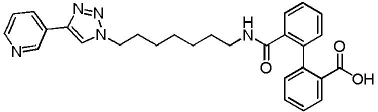 |
| N-(7-(4-(pyridin-3-yl)-1H-1,2,3-triazol-1-yl)heptyl)-[1,1'-biphenyl] -2-carbothioamide | 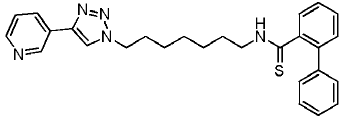 |
| N²-(7-(4-(pyridin-3-yl)-1H-1,2,3-triazol-1-yl)heptyl)-[1,1'-biphenyl]-2,2'-dicarboxamide | 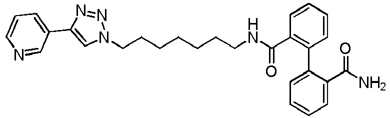 |
| 2-iodo-N-(7-(4-(pyridin-3-yl)-1H-1,2,3-triazol-1-yl)heptyl)benzamide | 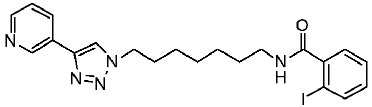 |
| 2'-((7-(4-(pyridin-3 -yl)-1H-1,2,3-triazol-1-yl)heptyl)carbamoyl)-[1,1'-biphenyl]-3-carboxylic acid | 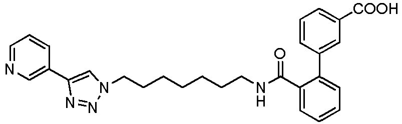 |
| 2'-((7-(4-(pyridin-3-yl)-1H-1,2,3-triazol-1-yl)heptyl)carbamoyl)-[1,1'-biphenyl]-4-carboxylic acid | 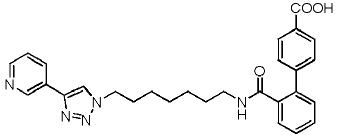 |
| 2-(pyridin-3-yl)-N-(7-(4-(pyridin-3-yl)-1H-1,2,3-triazol-1-yl)heptyl)benzamide | 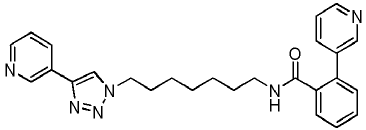 |
| 2'-methyl-N-(7-(4-(pyridin-3-yl)-1H-1,2,3-triazol-1-yl)heptyl)-[1,1'-biphenyl]-2-carboxamide | 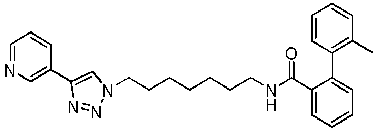 |
| 2-bromo-N-(8-(4-(pyridin-3-yl)-1H-1,2,3-triazol-1-yl)octyl)benzenesulfonamide | 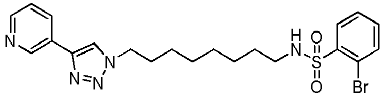 |
| 2-bromo-N-(7-(4-(pyridin-3-yl)-1H-1,2,3-triazol-1-yl)heptyl)benzenesulfonamide | 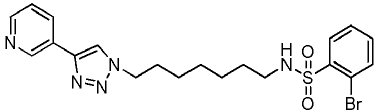 |
| 2-bromo-N-(6-(4-(pyridin-3-yl)-1H-1,2,3-triazol-1-yl)hexyl)benzenesulfonamide | 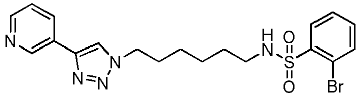 |
| N-(8-(4-(pyridin-3-yl)-1H-1,2,3-triazol-1-yl)octyl)biphenyl-2-sulfonamide | 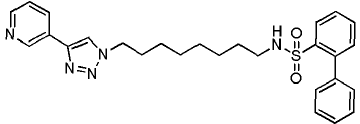 |
| N-(7-(4-(pyridin-3-yl)-1H-1,2,3-triazol-1-yl)heptyl)biphenyl-2-sulfonamide | 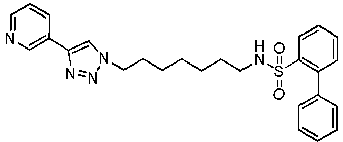 |
| N-(6-(4-(pyridin-3-yl)-1H-1,2,3-triazol-1-yl)hexyl)biphenyl-2-sulfonamide | 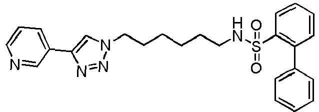 |
| N-([1,1'-biphenyl]-2-ylmethyl)-8-(4-(pyridin-3-yl)-1H-1,2,3-triazol-1-yl)octan-1-amine | 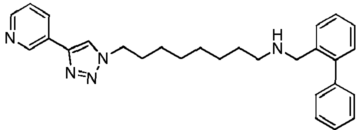 |
| N-([1,1'-biphenyl]-2-ylmethyl)-7-(4-(pyridin-3-yl)-1H-1,2,3-triazol-1-yl)heptan-1-amine | 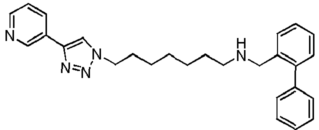 |
| N-([1,1'-biphenyl]-2-ylmethyl)-6-(4-(pyridin-3-yl)-1H-1,2,3-triazole-1-yl)hexan-1-amine | 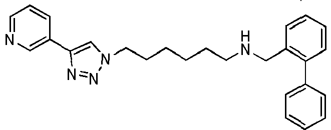 |
| 1-([1,1'-biphenyl]-2-il)-3-(8-(4-(pyridin-3-yl)-1H-1,2,3-triazole-1-yl)octyl)urea | 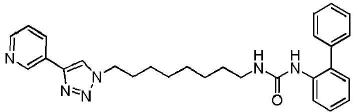 |
| 1-([1,1'-biphenyl]-2-yl)-3-(7-(4-(pyridin-3-yl)-1H-1,2,3-triazol-1-yl)heptyl)urea | 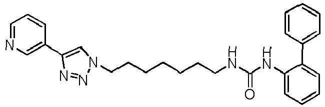 |
| 1-([1,1'-biphenyl]-2-yl)-3-(6-(4-(pyridin-3-yl)-1H-1,2,3-triazol-1-yl)hexyl)urea | 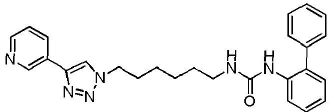 |
| N-(4-((4-(pyridin-3-yl)-1H-1,2,3-triazol-1-yl)methyl) phenethyl)-[1,1',-biphenyl]-2-carboxamide | 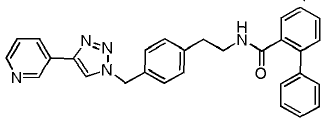 |
| 3-(1-(4-(2-([1,1'-biphenyl]-2-yloxy)ethyl)benzyl)-1H-1,2,3 -triazol-4-yl)pyridine | 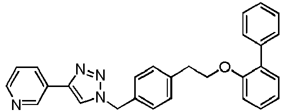 |
| 4-((4-(pyridin-3-yl)-1H-1,2,3-triazol-1-yl)methyl)phenethyl [1,1'-biphenyl]-2-ylcarbamate | 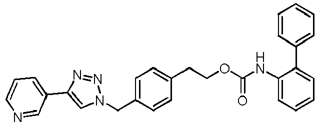 |
| 3-(1-(4-(2-(4-([1,1'-biphenyl]-2-yl)-1H-1,2,3-triazol-1-yl)ethyl)benzyl)-1H-1,2,3-triazol-4-yl)pyridine | 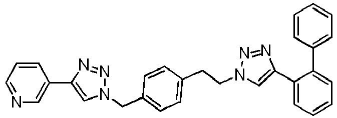 |
| 1-([1,1'-biphenyl]-2-yl)-3-(4-((4-(pyridin-3-yl)-1H-1,2,3-triazole-1-yl)methyl)phenethyl)urea | 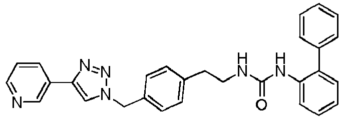 |
| N-([1,1'-biphenyl]-2-ylmethyl)-2-(4-((4-(pyridin-3-yl)-1H-1,2,3-triazole-1-yl)methy)phenyl)ethanamine | 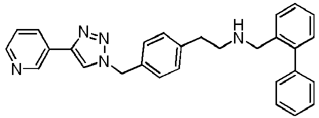 |
| 2-bromo-N-(4-((4-(pyridin-3-yl)-1H-1,2,3-triazol-1-yl)methyl)phenethyl)benzenesulfonami de | 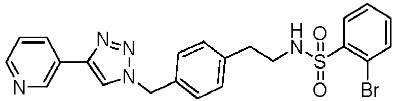 |
| N-(4-((4-(pyridin-3-yl)-1H-1,2,3-triazol-1-yl)methyl)phenethyl)-[1,1'-biphenyl]-2-sulfonamide | 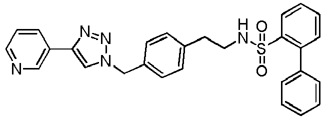 |
| N-(4-(2-(4-(pyridin-3-yl)-1H-1,2,3-triazol-1-yl)ethyl)benzyl)-[1,1'-biphenyl]-2-carboxamide | 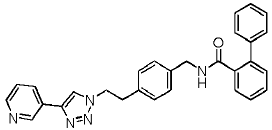 |
| 3-(1-(4-(([1,1'-biphenyl]-2-yloxy)methyl)phenethyl)-1H-1,2,3-triazol-4-yl)pyridine | 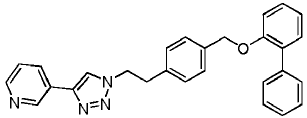 |
| N-(3-(4-(4-(pyridin-3-yl)-1H-1,2,3-triazol-1-yl)phenyl)propyl)-[1,1'-biphenyl]-2-carboxamide | 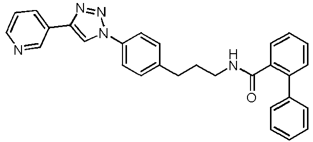 |
| 3-(1-(4-(3-([1,1'-biphenyl]-2-yloxy)propyl)phenyl)-1H-1,2,3-triazol-4-yl)pyridine | 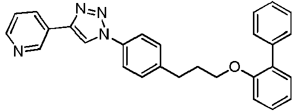 |
| N-(4-(3-(4-(pyridin-3-yl)-1H-1,2,3-triazol-1-yl)propyl)phenyl)-[1,1'-biphenyl]-2-carboxamide | 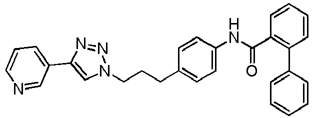 |
| 3-(1-(3-(4-([1,1'-biphenyl]-2-yloxy)phenyl)propyl)-1H-1,2,3-triazol-4-yl)pyridine | 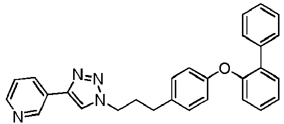 |

### Synthesis of compounds of formula (I)

The following schemes show a method for preparing the compounds of the present description.

For a more detailed description of the individual reaction steps, see the Examples herein below.

Those skilled in the art will appreciate that other synthetic routes may be used to synthesize the compounds of the invention. Although specific starting materials and reagents are depicted in the scheme and discussed below, other starting materials and reagents can be easily substituted to provide a variety of derivatives and/or reaction conditions. In addition, many of the compounds prepared by the methods described below can be further modified in light of this disclosure using conventional chemistry well known to those skilled in the art.

Compounds of formula (I) can be prepared as outlined in the **Schemes a-k** below.

Intermediates useful for the synthesis of compounds of formula (I) represented in **Scheme a** are identified as compounds (**4**), (**5**), and (**6**).

The azides (**1**) were prepared applying synthetic methodology known in the art starting from the corresponding bromide derivatives, while the 1,4-disubstituted triazoles (**2**) were prepared by a 1,3-dipolar cycloaddition between the corresponding azides and the 3-ethynylpyridine catalyzed by the active copper (I) specie generated *in situ* by sodium ascorbate and copper (II) sulfate (Angew. Chem. Int. Ed. 2002, 41, 2596). The intermediates **2**, were then esterified to give **3,** which were reduced to the alcoholic key intermediates **4**. These intermediates can be transformed in the corresponding azides **5**, which were reduced to amines **6** with triphenylphosphine and water.

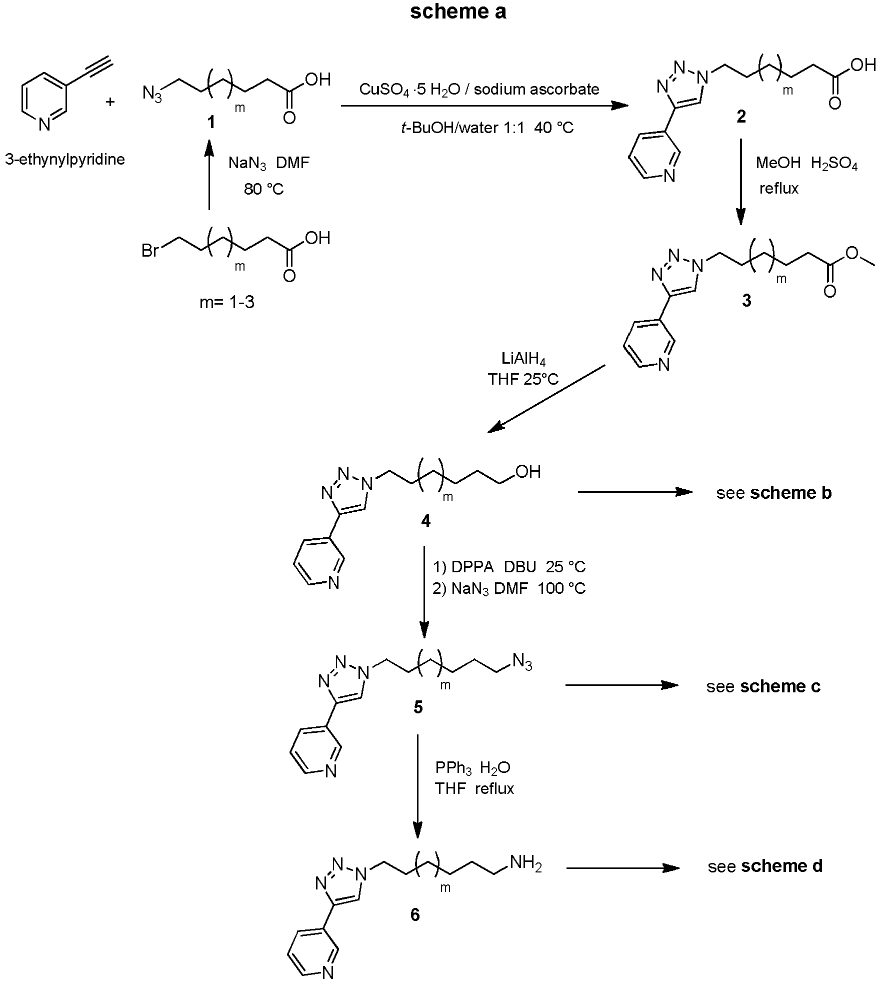

Compounds of formula (I) can be prepared as outlined in the **Scheme b** below. Compounds of formula (I) represented in **Scheme b** are identified as compounds (**8**), (**9**), (**10**) and (**11**).

To prepare compounds **8**, intermediates **4** were converted into their corresponding mesylates 7, which reacted with different phenolic compounds under basic conditions. Intermediates 7 can also react with 2-iodophenol in the presence of sodium hydride and DMF to give compounds **9**. These latter can undergo a Suzuki reaction to generate compounds **10.** Finally, compounds **11** were prepared by reacting intermediates **4** with biphenyl-2-isocyanate.

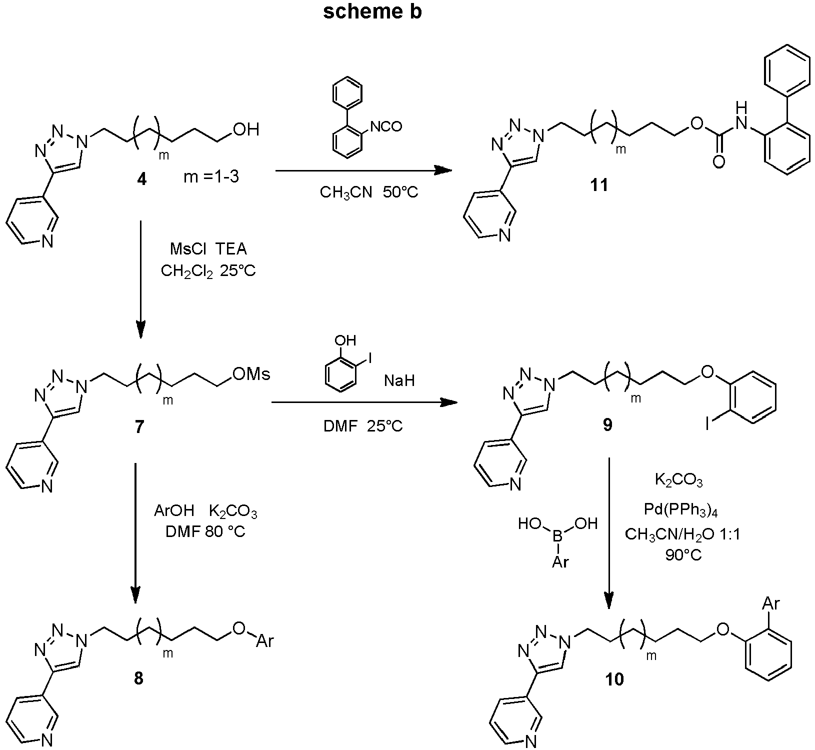

Compounds of formula (I) can be prepared as outlined in the **Scheme c** below. Bis-triazoles of formula (I) represented in **Scheme c** are identified as compounds (**12**).

Bis-triazoles (**12**) were prepared by reacting intermediates **5** with phenylacetylene or 2-ethynylbiphenyle under the Sharpless-Fokin reaction conditions (Copper sulfate, sodium ascorbate, tert-butanol/water).

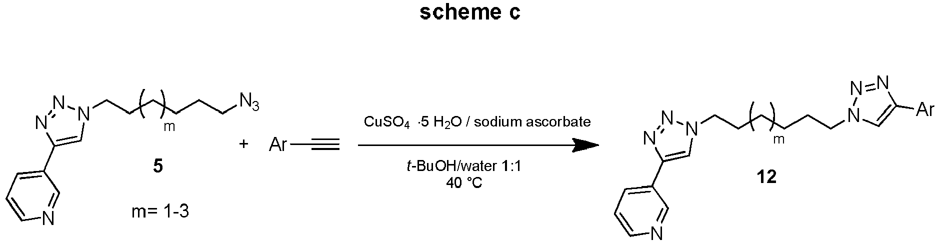

Compounds of formula (I) can be prepared as outlined in the **Scheme d** below. Compounds of formula (I) represented in **Scheme d** are identified as compounds (**13**), (**14**), (**15**), (**16**), (**17**), (**18**), (**19**) and (**20**).

Amidation of 6 with different carboxylic acids in the presence of coupling agents gave amides **13.** Amides **13** can be further transformed in thioamides **14** by using Lawesson's reagent. Intermediates **6** can also coupled with 2-iodobenzoic acid to give compounds **15**, which can undergo a Suzuki reaction with different boronic acids to give compounds **16**. Intermediates **6** react with 2-bromobenzen-1-sulfonyl chloride to give compounds **17**, which can undergo a Suzuki reaction with different boronic acids to give compounds **18**. Intermediates **6** can undergo a two steps reductive amination by reacting at first with biphenyl-2-carboxyaldehyde in dichloromethane and then with sodium borohydride in methanol to give compounds **19**. Finally, compounds **20** were prepared by reacting intermediates **6** with biphenyl-2-isocyanate.

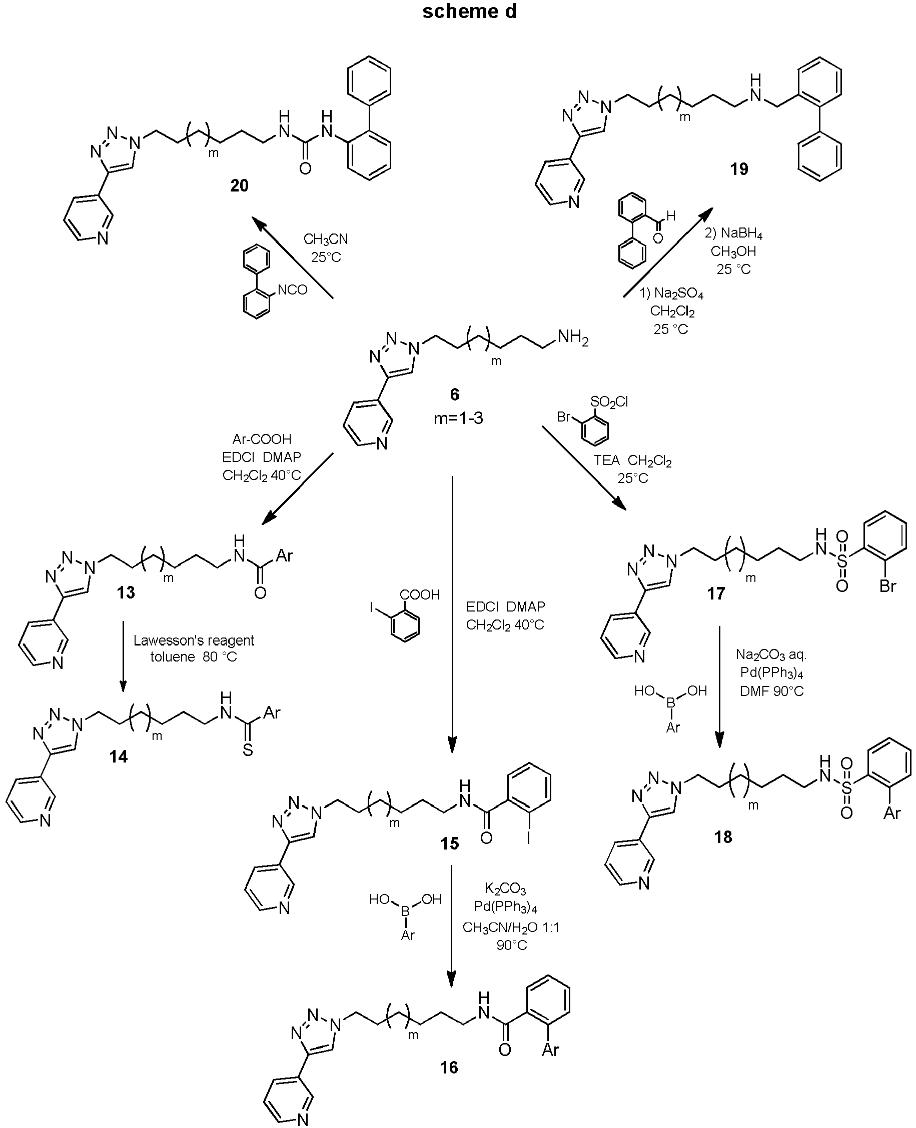

Compounds of formula (I) can be prepared as outlined in the **Scheme e** below. Compounds of formula (I) represented in **Scheme e** are identified as compounds (**27**) and (**28**).

Commercially available 2-(4-(bromomethyl)phenyl)acetic acid was converted into its methylester **21** by using catalytic thionyl chloride in methanol. Compound **21** reacted with sodium azide in DMF to give the azido derivative **22**. This compound underwent a 1,3 dipolar cycloaddition with 3-ethynylpyridine in the presence of copper sulfate and sodium ascorbate. The methylester of the 1,4-disubstituted triazole **23** was reduced to alcohol **24** with lithium aluminum hydride. The alcoholic group of intermediate **24**, can be converted to amine **26** via azide **25**. The amine **26** was finally coupled with 2-biphenylcarboxylic acid to give **27**. Intermediate **24** was furthermore coupled with 2-phenylphenol under Mitsunobu conditions (DIAD, triphenylphosphine) to give compound **28**.

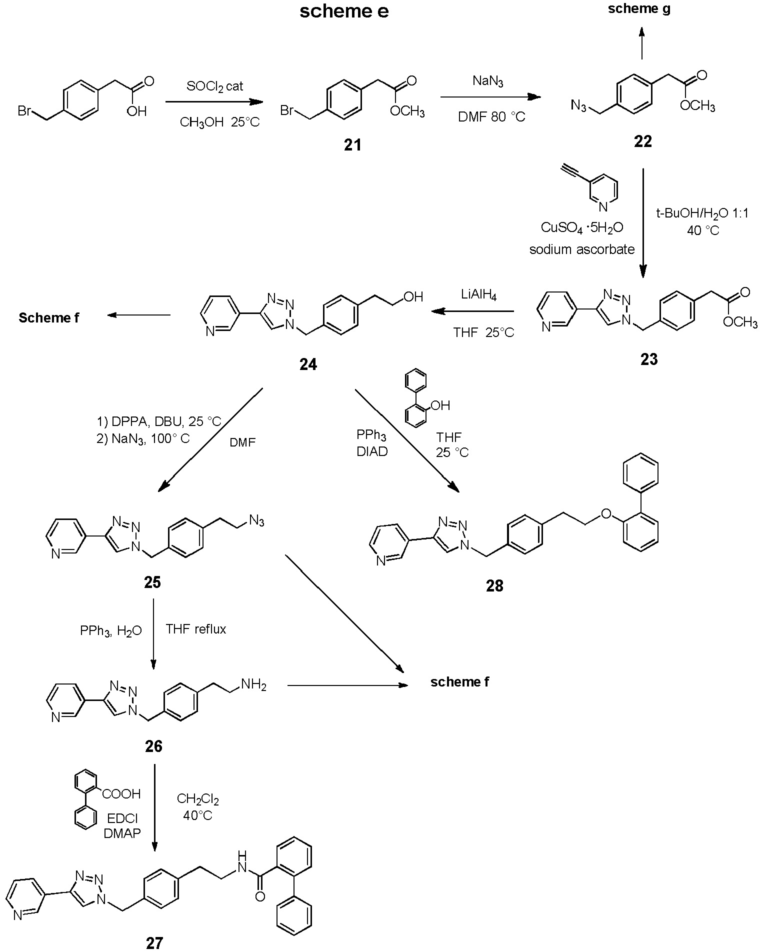

Compounds of formula (I) can be prepared as outlined in the **Scheme f** below. Compounds of formula (I) represented in **Scheme f** are identified as compounds (**29**), (**30**), (**31**), (**32**), (**33**) and (**34**).

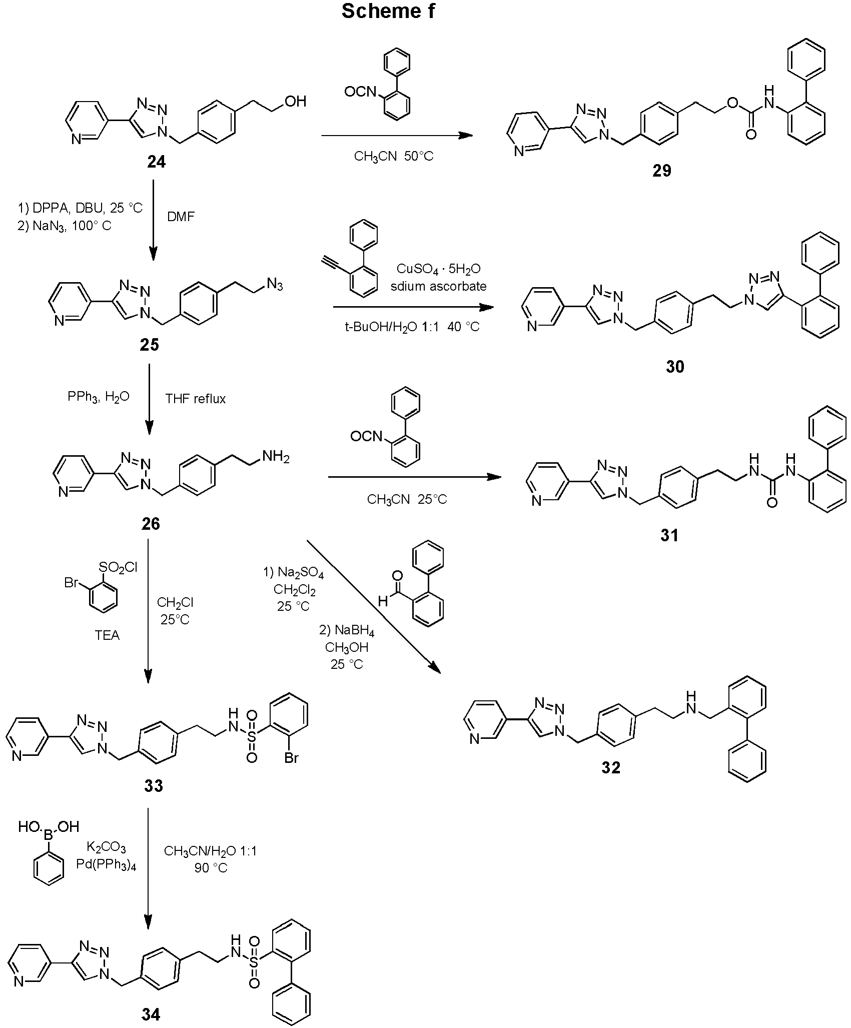

Intermediate **24** reacted with biphenyl-2-isocyanate to give compound **29**. Intermediate **25** underwent a 1,3-dipolar cycloaddition with 2-ethynylbiphenyle under classical conditions (copper sulfate, sodium ascorbate) to give the bis-triazolyl derivative **30**. Intermediate **26** was used to prepare compounds **31**, **32**, **33**, and **34**.

It can undergo reaction with biphenyl-2-isocyanate to give the urea derivative **31**. Alternatively, it can undergo a two steps reductive amination by reacting at first with biphenyl-2-carboxyaldehyde in dichloromethane and then with sodium borohydride in methanol to give compounds **32**. Alternatively, **26** can react with 2-bromobenzene-1-sulfonyl chloride to give **33**. **33** reacted with phenylboronic acid in a Suzuki reaction yielding compound **34**.

Compounds of formula (I) can be prepared as outlined in the **Scheme g** below. Compound of formula (I) represented in **Scheme g** is identified as compound (**38**).

Intermediate **22** underwent a lithium aluminum hydride-mediated reduction to give the amino-alcohol derivative **35**. A chemoselective coupling with biphenyl-2-carboxylic acid, using propane phosphonic acid anhydride (PPAA), as coupling agent, afforded derivative **36**, which was converted into the azide intermediate **37** by using diphenylphosporyl azide (DPPA), DBU and sodium azide. 1,3-dipolar cycloaddition with 3-ethynylpyridine afforded the final compound **38**.

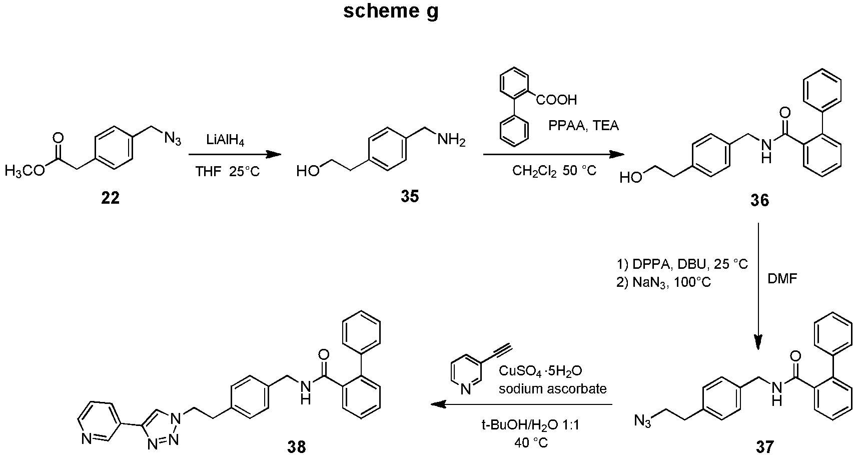

Compounds of formula (I) can be prepared as outlined in the **Scheme h** below. Compound of formula (I) represented in **Scheme h** is identified as compound (**43**).

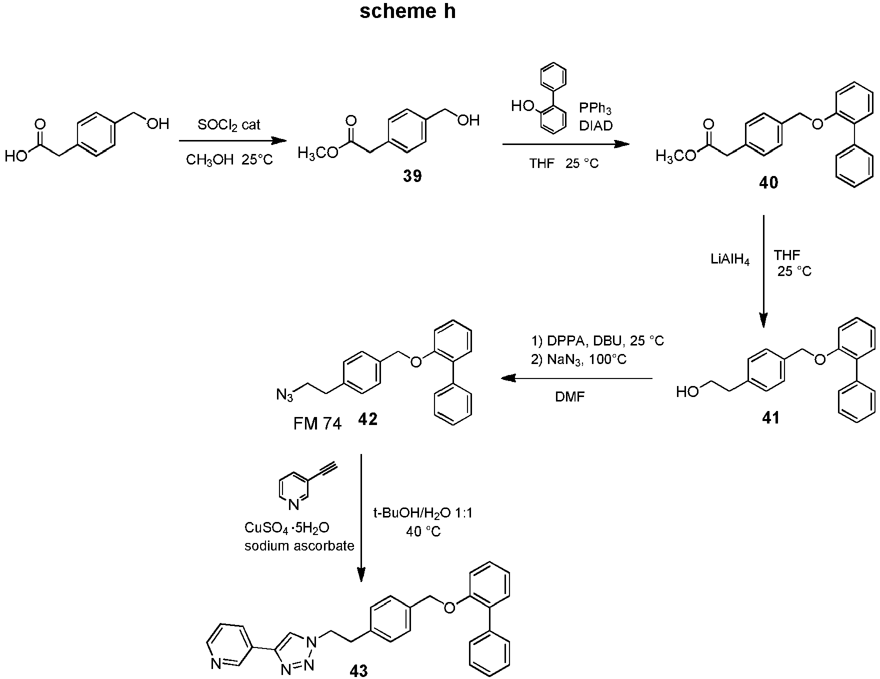

Commercially available 2-(4-(hydroxymethyl)phenyl-acetic acid was esterified (MeOH, SOCl₂ cat) to give intermediate **39**, which underwent a Mitsunobu reaction with 2-phenylphenol to give compound **40**. The ester of this latter was reduced to alcohol using lithium aluminum hydride and the alcohol **41** transformed into the azide **42** (DPPA, DBU, sodium azide). 1,3-dipolar cycloaddition with 3-ethynylpyridine afforded the final compound **43**.

Compounds of formula (I) can be prepared as outlined in the **Scheme i** below. Compounds of formula (I) represented in **Scheme i** are identified as compounds (**49**) and (**50**).

The synthetic scheme commences with the commercially available (*E*)-3-(4-nitrophenyl)prop-2-en-1-ol which underwent a double bond reduction by using Nickel Raney to afford compound **44**. A diazotation-azidation protocol (a: NaNO₂; HCl conc; 0 °C / b: sodium azide 25 °C) gave the azide derivative **45** which was then coupled with 3-ethynylpyridine to give the triazole derivative **46**. This intermediate can be converted into its azide derivative **47** by using diphenylphosporyl azide (DPPA), DBU and sodium azide. Staudinger reduction (triphenylphosphine, water) was used to reduce the azide group into the amine **48**. **48** was coupled with biphenyl-2-carboxylic acid to give the final compound **49**. Intermediate **46** can also undergo a Mitsunobu reaction with 2-phenylphenol to yield final compound **50**.

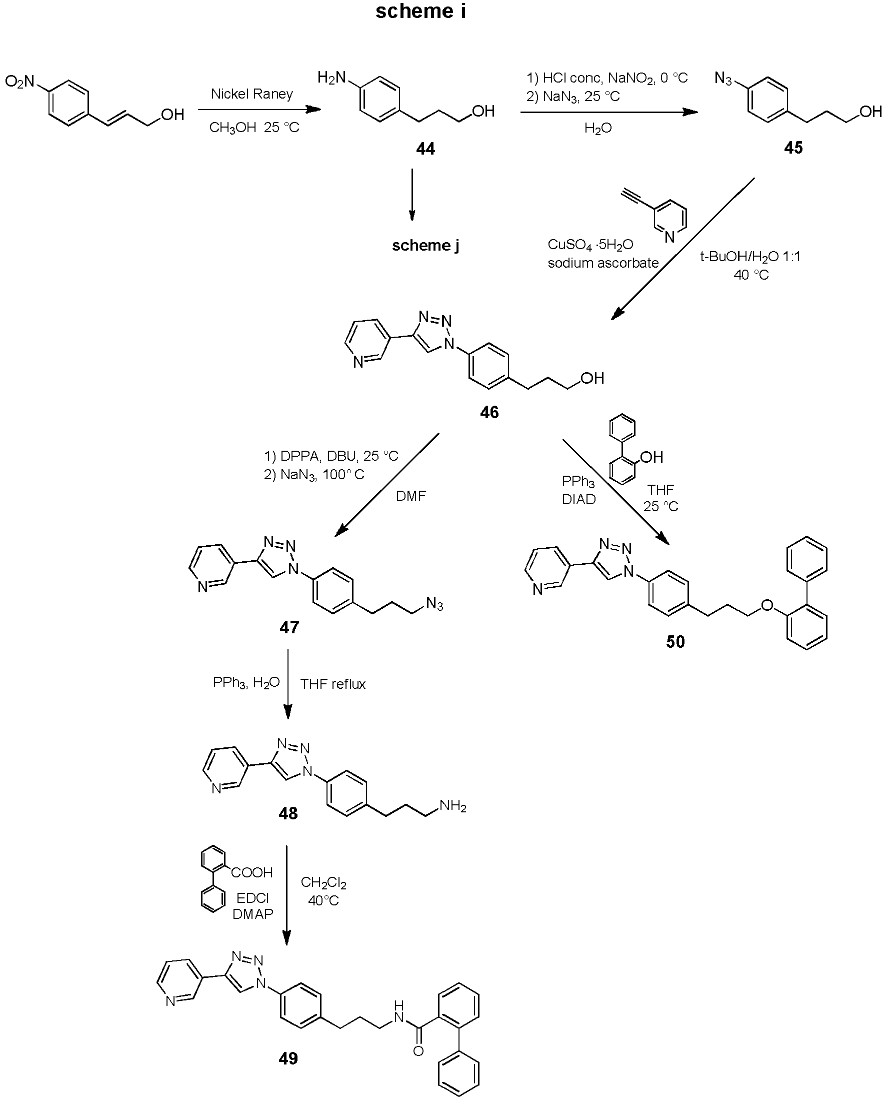

Compounds of formula (I) can be prepared as outlined in the **Scheme j** below. Compound of formula (I) represented in **Scheme j** is identified as compound (**53**).

Compound **44** was converted into its azide derivative **51** via DPPA, DBU, and sodium azide. Compound **51** was then coupled with biphenyl-2-carboxylic acid to give compound **52**. Finally, a 1,3-dipolar cycloaddition between **52** and the 3-ethynylpyridine afforded the final compound **53**.

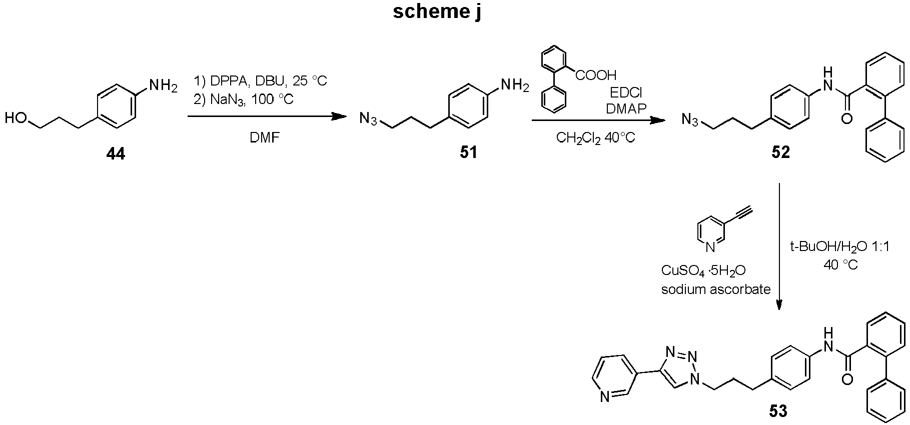

Compounds of formula (I) can be prepared as outlined in the **Scheme k** below. Compound of formula (I) represented in **Scheme k** is identified as compounds (**57**).

The commercially available 3-(4-bromophenyl)propanoic acid was reduced to alcohol **54**, which was converted into the azide **55**. A 1,3-dipolar cycloaddition between **55** and the 3-ethynylpyridine afforded the 1,4-disubstituted triazole **56**, which underwent a Ullmann-like coupling (CuI; K₂CO₃; 1-butylimidazole) with 2-phenylphenol to give the final compound **57**.

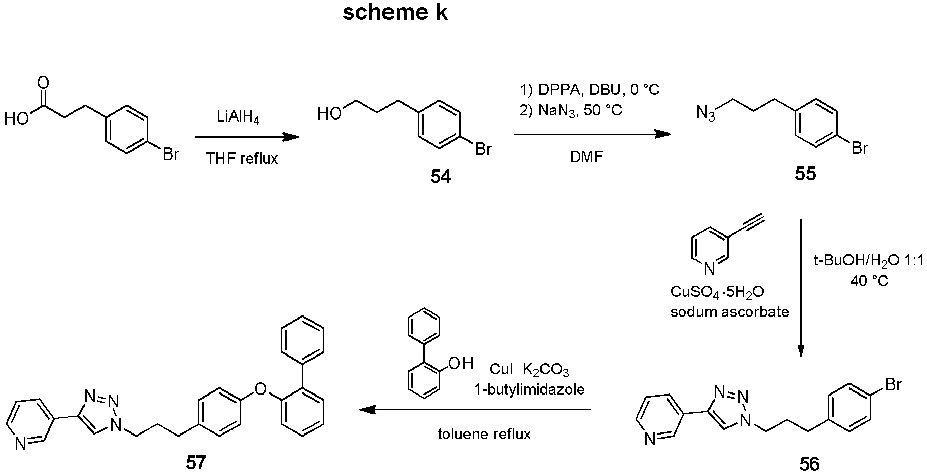

The chemical reactions described in the examples below may be readily adapted to prepare a number of other intermediates and nicotinamide phosphoribosyltransferase inhibitors of the invention, and alternative methods for preparing the compounds of this invention are deemed to be within the scope of this invention.

For example, the synthesis of non-exemplified compounds according to the invention may be successfully performed by modifications apparent to those skilled in the art, e.g., by appropriately protecting interfering groups, by utilizing other suitable reagents known in the art other than those described, and/or by making routine modifications of reaction conditions. Alternatively, other reactions disclosed herein or known in the art will be recognized as having applicability for preparing other compounds of the invention.

### Synthesis of intermediates (4), (5), (6) - (Scheme a)

### Example 1. General Procedure for the Preparation of Azides (1)

The corresponding bromo derivative (1 eq) was dissolved in *N,N'-*dimethylformamide (0.4 M). While stirring at room temperature sodium azide was added (1.2 eq) and the reaction was heated at 80 °C overnight. After completion of the reaction water was added and the desired product was extracted with diethyl ether (x3). The combined organic layers were washed with water (x3), brine (x1), dried over sodium sulfate and *concentrated in vacuo* to give the desired azide, which was used in the next step without further purification.

### 8-azidooctanoic acid

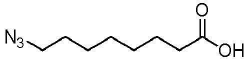

Yellowish oil; yield 87%; IR (neat) 2955, 2093, 1716, 1652, 1540, 1506, 1457, 667 cm⁻¹; ¹H-NMR (300 MHz, CDCl₃) *δ* 11.8 (br s, 1-H), 3.21 (m, 2-H), 2.31 (m, 2-H), 1.31 (s, 10-H) ppm; ¹³C-NMR (75 MHz, CDCl₃) *δ* 180.6, 51.4, 34.1, 28.9 (3-C), 28.8, 24.5 ppm.

### 7-azidoheptanoic acid

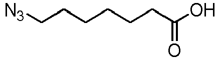

Colorless oil; yield 94%; IR (KBr) 2950, 2870, 2095, 1712, 1424, 1258, 665 cm⁻¹; ¹H-NMR (300 MHz, CDCl₃) *δ* 11.55 (br s, 1-H), 3.14 (t, *J* = 6.8 Hz, 2-H), 2.22 (t, *J* = 7.2 Hz, 2-H), 1.56-1.45 (m, 4-H), 1.30-1.24 (m, 4-H) ppm; ¹³C-NMR (75 MHz, CDCl₃) *δ* 178.3, 51.1, 33.7, 28.5, 28.4, 26.2, 24.4 ppm.

### 6-azidohexanoic acid

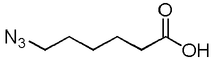

Colorless oil; yield 62%; IR (KBr) 2940, 2866, 2092, 1704, 1412, 1256, 668 cm⁻¹; ¹H NMR (300 MHz, CDCl₃) *δ* 11.13 (br s, 1-H), 3.21 (td, *J* = 6.7/1.8 Hz, 2-H), 2.31 (td, *J* = 7.4/2.1 Hz, 2-H), 1.72-1.50 (m, 4-H), 1.41-1.34 (m, 2-H) ppm; ¹³C-NMR (75 MHz, CDCl₃) *δ* 181.5, 52.7, 35.4, 30.0, 27.7, 25.7 ppm.

### Example 2. General Procedure for the Preparation of the 1,4-disubstituted Triazoles (2)

3-ethynylpyridine (1 eq) and the corresponding azide (**1**) (1 eq) were suspended in a mixture of water/*tert*-butanol (1:1) Sodium ascorbate (0.1 eq), of a freshly prepared 1 M solution in water, was added, followed by the addition of copper (II) sulfate pentahydrate (0.01 eq). The resulting mixture was heated to 40 °C and vigorously stirred for 24 h. The reaction mixture was then diluted with water, cooled, and the precipitate was collected by filtration, washed with diethyl ether and dried *in vacuo.*

When addition of water failed to precipitate the triazole, the reaction mixture was extracted with EtOAc (x3). The combined organic layers were washed with brine (x1) dried over sodium sulfate and concentrated *in vacuo.* Finally the crude was purified by column chromatography to give the desired triazole.

### 8-(4-(pyridin-3-yl)-1H-1,2,3-triazol-1-yl)octanoic acid

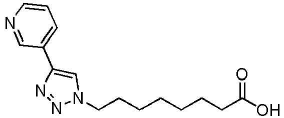

The compound precipitated as a yellowish solid; yield 87%; m.p. 142.1-142.6 °C; IR (KBr) 2092, 1705, 1219, 892, 772, 632 cm⁻¹; ¹H-NMR (300 MHz, (CD₃)₂SO) *δ* 8.71 (s, 1-H), 8.20 (d, *J* = 7.6 Hz, 1-H), 7.49 (br s, 1-H), 4.39 (t, *J* = 7.1 Hz, 2-H), 1.85 (m, 2-H), 1.26 (s, 8-H) ppm; ¹³C-NMR (75 MHz, (CD₃)₂SO) *δ* 183.1, 147.9, 147.5, 145.9, 134.1, 133.1, 130.3, 124.1, 52.4, 37.6, 29.1 (2-C), 28.4, 27.1, 25.7 ppm.

### 7-(4-(pyridin-3-yl)-1H-1,2,3-triazol-1-yl)heptanoic acid

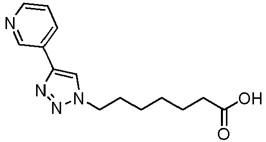

The compound precipitated as a yellowish amorphous solid; yield 90%; IR (KBr) 3560, 2975, 1715, 1420, 1055, 1030, 815 cm⁻¹; ¹H-NMR (300 MHz, (CD₃)₂SO) *δ* 11.80 (br s, 1-H), 9.03 (br s, 2-H), 8.60 (s, 1-H), 8.25 (d, *J* = 7.3 Hz, 1-H), 7.60 (br s, 1-H), 4.40 (t, *J* = 6.7 Hz, 2-H), 2.30 (br s, 2-H), 1.85 (m, 2-H), 1.53 (br s, 2-H), 1.25 (br s, 4-H) ppm; ¹³C-NMR (75 MHz, (CD₃)₂SO) *δ* 182.5, 147.9, 147.5, 145.8, 134.3, 133.2, 130.3, 124.2, 51.9, 37.6, 31.6, 29.9, 27.8, 25.6 ppm.

### 6-(4-(pyridin-3-yl)-1H-1,2,3-triazol-1-yl)hexanoic acid

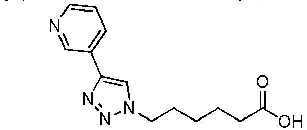

The compound precipitated as a white solid; yield 95%; m.p. 171-173 °C dec.; IR (KBr) 3679, 2972, 1711, 1410, 1053, 1032, 819 cm⁻¹; ¹H-NMR (300 MHz, (CD₃)₂SO) *δ* 12.03 (br s, 1-H), 9.00 (br s, 2-H), 8.69 (s, 1-H), 8.22 (d, *J* = 7.3 Hz, 1-H), 7.58 (br s, 1-H), 4.40 (t, *J* = 6.7 Hz, 2-H), 2.25 (br s, 2-H), 1.86 (m, 2-H), 11.53 (br s, 2-H), 1.27 (br s, 2-H) ppm; ¹³C-NMR (75 MHz, (CD₃)₂SO) *δ* 181.5, 147.9, 147.5, 145.6, 134.1, 133.1, 130.3, 124.1, 51.6, 37.6, 31.4 (2-C), 27.5 ppm.

### Example 3. General Procedure for the Preparation of Methyl Esters (3)

The corresponding carboxylic acid (**2**) (1 eq) dissolved in CH₃OH (0.4 M), and H₂SO₄ 96% w/w (1.1 eq) was heated at reflux. After 2 h. the solvent was evaporated under reduced pressure. The crude material was diluted with sat. aq. NaHCO₃ and extracted with EtOAc (x3). The combined organic layers were washed with brine, dried over sodium sulfate and concentrated *in vacuo* to give the desired methyl ester, which was used in the next step without further purification.

### methyl 8-(4-(pyridin-3-yl)-1H-1,2,3-triazol-1-yl) octanoate

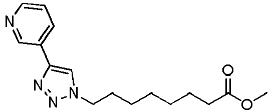

White solid; yield 72%; m.p. 82-83 °C; IR (KBr) 2933, 2925, 1730, 1452, 1055, 1031, 675 cm⁻¹; ¹H-NMR (300 MHz, CDCl₃) *δ* 8.79 (br s, 1-H), 8.28 (br s, 1-H), 7.91 (d, *J* = 8.0 Hz, 1-H), 7.84 (s, 1H), 7.10-7.06 (m, 1H), 4.15 (t, *J* = 7.0 Hz, 2-H), 3.36 (s, 3-H), 2.00 (t, *J* = 7.4 Hz, 2-H), 1.69-1.65 (m, 2-H), 1.35-1.28 (m, 2-H), 1.05-0.93 (m, 6-H) ppm; ¹³C-NMR (75 MHz, CDCl₃) *δ* 175.3, 150.2, 148.2, 145.7, 134.1, 128.3, 125.1, 121.8, 52.7, 51.7, 35.1, 31.4, 30.0, 29.8, 27.5, 26.0 ppm.

### methyl 7-(4-(pyridin-3-yl)-1H-1,2,3-triazol-1-yl) heptanoate

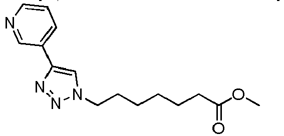

White solid; yield 85%; m.p. 74-75 °C; IR (KBr) 2942, 1732, 1442, 1053, 1032, 806 cm⁻¹; ¹H-NMR (300 MHz, CDCl₃) *δ* 8.90 (d, *J* = 2.2 Hz, 1-H), 8.43 (d, *J* = 3.6 Hz, 1-H), 8.06 (d, *J* = 7.7 Hz, 1-H), 7.84 (s, 1H), 7.24 (dd, *J* = 8.0/5.0 Hz, 1H), 4.30 (t, *J* = 7.1 Hz, 2-H), 3.52 (s, 3-H), 2.17 (t, *J* = 7.2 Hz, 2-H), 1.84 (br quint, 2-H), 1.49 (br quint, 2-H), 1.27-1.22 (m, 4-H) ppm; ¹³C-NMR (75 MHz, CDCl₃) *δ* 175.2, 150.6, 148.4, 146.1, 134.5, 128.4, 125.3, 121.6, 53.0, 51.9, 35.3, 31.6, 29.9, 27.6, 26.1 ppm.

### methyl 6-(4-(pyridin-3-yl)-1H-1,2,3-triazol-1-yl) hexanoate

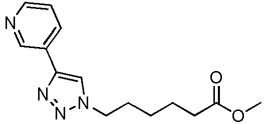

White solid; yield 96%; m.p. 95-95.5 °C; IR (KBr) 2937, 2922, 1732, 1455, 1054, 1033, 668 cm⁻¹; ¹H-NMR (300 MHz, CDCl₃) *δ* 9.01 (br s, 1-H), 8.58 (br s, 1-H), 8.28 (d, *J* = 7.0 Hz, 1-H), 7.91 (s, 1-H), 7.42 (br s, 1-H), 4.43 (t, *J* = 7.0 Hz, 2-H), 3.64 (s, 3-H), 2.32 (t, *J* = 7.4 Hz, 2-H), 1.98 (quint, *J* = 7.3 Hz, 2-H), 1.69 (quint, *J* = 7.3 Hz, 2-H), 1.39 (quint, *J* = 7.3 Hz, 2-H) ppm; ¹³C-NMR (75 MHz, CDCl₃) *δ* 175.3, 150.8, 148.6, 146.3, 134.5, 128.4, 125.4, 121.6, 53.1, 51.8, 35.2, 31.6, 27.5, 25.7 ppm.

### Example 4. General Procedure for the Preparation of Alcohols (4)

Under a nitrogen atmosphere LiAlH₄ (1.5 eq) was added portionwise to a stirred solution of the corresponding methylester (**3**) (1 eq) in THF dry (0.2 M) at 0 °C. After 0.5 h, the reaction was quenched by dropwise addition of NaOH 2 M solution under stirring and a white precipitate was formed. The suspension was filtered and the precipitated material was washed with MeOH. The filtrate obtained was concentrated *in vacuo* and the residue was purified by column chromatography using CH₂Cl₂/MeOH 98:2 and CH₂Cl₂/MeOH 95:5 as eluants to give the desired alcohol.

### 8-(4-(pyridin-3-yl)-1H-1,2,3-triazol-1-yl)octan-1-ol

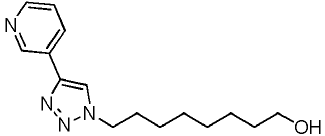

White solid; yield 84%; m.p. 86-88 °C; IR (KBr) 3370, 2922, 1457, 1032, 810 cm⁻¹; ¹H-NMR (300 MHz, CDCl₃) *δ* 8.87 (d, *J* = 1.6 Hz, 1-H), 8.40 (dd, *J* = 4.6/1.5 Hz, 1-H), 8.06 (dt, *J* = 8.3/1.8 Hz, 1-H), 7.86 (s, 1-H), 7.24 (dd, *J* = 8.0/4.9 Hz, 1-H), 4.28 (t, *J* = 7.0 Hz, 2-H), 3.49 (t, *J* = 6.4 Hz, 2-H), 1.83-1.78 (m, 2-H), 1.44-1.39 (m, 2-H), 1.28-1.14 (m, 8-H) ppm; ¹³C-NMR (75 MHz, CDCl₃) *δ* 150.3, 148.2, 145.9, 134.6, 128.4, 125.3, 121.7, 63.8, 52.0, 34.1, 31.6, 30.6, 30.3, 27.8, 27.1 ppm.

### 7-(4-(pyridin-3-yl)-1H-1,2,3-triazol-1-yl)heptan-1-ol

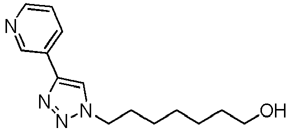

White solid; yield 98%; m.p. 96-97 °C; IR (KBr) 3268, 2933, 1455, 1056, 705 cm⁻¹; ¹H-NMR (300 MHz, CDCl₃) *δ* 8.93 (d, *J* = 1.5 Hz, 1-H), 8.48 (dd, *J* = 4.6/1.5 Hz, 1-H), 8.13 (dt, *J* = 8.0/1.8 Hz, 1-H), 7.85 (s, 1-H), 7.31 (dd, *J* = 8.0/4.9 Hz, 1-H), 4.35 (t, *J* = 7.4 Hz, 2-H), 3.57 (t, *J* = 6.4 Hz, 2-H), 2.94 (br s, 1-H), 1.90 (br quint 2-H), 1.49 (br quint, 2-H) 1.31 (br s, 6-H) ppm; ¹³C-NMR (75 MHz, CDCl₃) *δ* 150.5, 148.4, 146.2, 134.6, 128.5, 125.4, 121.6, 64.0, 52.1, 34.1, 31.7, 30.3, 27.9, 27.1 ppm.

### 6-(4-(pyridin-3-yl)-1H-1,2,3-triazol-1-yl)hexan-1-ol

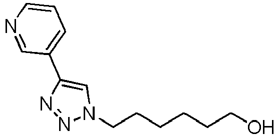

Yellowish solid; yield 96%; m.p. 79-79.5 °C; IR (KBr) 3450, 2937, 1408, 1033, 704 cm⁻¹; ¹H-NMR (300 MHz, CDCl₃) *δ* 8.91 (d, *J* = 2.1 Hz, 1-H), 8.46 (dd, *J* = 4.6/1.2 Hz, 1-H), 8.12 (dt, *J* = 8.0/1.5 Hz, 1-H), 7.88 (s, 1-H), 7.29 (dd, *J* = 8.0/4.9 Hz, 1-H), 4.34 (t, *J* = 7.0 Hz, 2-H), 3.55 (t, *J* = 6.4 Hz, 2-H), 3.30 (br s, 1-H), 1,89 (quint, *J* = 7.0 Hz, 2-H), 1.49 (quint, *J* = 6.8 Hz, 2-H), 1.39-1.28 (m, 4-H) ppm; ¹³C-NMR (75 MHz, CDCl₃) *δ* 150.5, 148.4, 146.1, 134.7, 128.5, 125.4, 121.8, 63.7, 52.0, 34.0, 31.8, 27.8, 26.8 ppm.

### Example 5. General Procedure for the Preparation of Azides (5)

Under a nitrogen atmosphere, DPPA (diphenylphosphoryl azide) (2 eq) and 1,8-Diazabicycloundec-7-ene (DBU) (2 eq) were subsequently added to a cooled (0° C) solution of the corresponding alcohol (**4**) (1 eq) in dry *N,N'-*dimethylformamide (0.4 M). After 0.5 h. NaN₃ (2 eq) was added and the reaction mixture was heated for 3 h. at 100 °C. After cooling to room temperature, the reaction was worked up by dilution with water and extraction with diethyl ether (x5). The combined organic layers were washed with water (x2), brine (x1), dried over sodium sulfate and concentrated *in vacuo.* Finally the crude material was purified by column chromatography to give the desired azide

### 3-(1-(8-azidooctyl)-1H-1,2,3-triazol-4-yl)pyridine

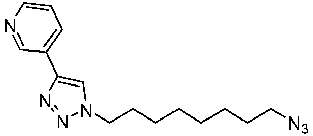

The crude material was purified by column chromatography using PE/EtOAc 2:8 and EtOAc as eluants to give a white solid; yield 86%; m.p. 62-62.5 °C; IR (KBr) 2972, 2095, 1456, 1346, 1188, 972 cm⁻¹; ¹H-NMR (300 MHz, CDCl₃) *δ* 8.95 (br s, 1-H), 8.50 (br s, 1-H), 8.13 (dt, *J* = 8.0/1.5 Hz, 1-H), 7.83 (s, 1-H), 7.33-7.28 (m, 1-H), 4.35 (td, *J* = 7.0/2.8 Hz, 2-H), 3.17 (td, *J* = 6.8/2.8 Hz, 2-H), 1.89 (br s, 2-H), 1.50 (br s, 2-H), 1.28 (br s, 8-H) ppm; ¹³C-NMR (75 MHz, CDCl₃) *δ* 150.5, 148.4, 146.1, 134.6, 128.4, 125.3, 121.5, 52.9, 52.0, 31.8, 30.4, 30.3, 30.2, 28.1, 27.9 ppm.

### 3-(1-(7-azidoheptyl)-1H-1,2,3-triazol-4-yl)pyridine

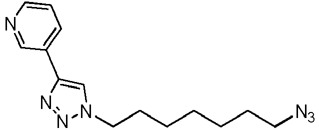

The crude material was purified by column chromatography using PE/EtOAc 2:8 and EtOAc as eluants to give a yellowish oil; yield 90 %; IR (KBr) 2934, 2858, 2094, 1589, 1488, 1187, 970 cm⁻¹; ¹H-NMR (300 MHz, CDCl₃) *δ* 8.96 (d, *J* = 1.5 Hz, 1-H), 8.48 (dd, *J* = 3.4/1.2 Hz, 1-H), 8.14 (dt, *J* = 8.0/1.8 Hz, 1-H), 7.88 (s, 1-H), 7.30 (dd, *J* = 8.0/4.9 Hz, 1-H), 4.33 3 (t, *J* = 7.4 Hz, 2-H), 3.15 (t, *J* = 6.7 Hz, 2-H), 1.87 (quint, *J* = 6.7 Hz, 2-H), 1.48 (quint, *J* = 7.0 Hz, 2-H), 1.28 (br s, 6-H) ppm; ¹³C-NMR (75 MHz, CDCl₃) *δ* 150.1, 148.1, 145.9, 134.9, 128.7, 125.5, 121.8, 52.8, 52.0, 31.7, 30.2, 30.0, 28.0, 27.8 ppm.

### 3-(1-(6-azidohexyl)-1H-1,2,3-triazol-4-yl)pyridine

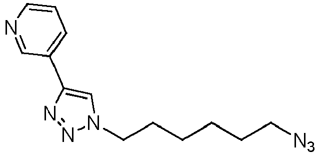

The crude material was purified by column chromatography using PE/EtOAc 2:8 and EtOAc as eluants to give a yellowish oil; yield 88%; IR (KBr) 2937, 2863, 2096, 1454, 1032, 709 cm⁻¹; ¹H-NMR (300 MHz, CDCl₃) *δ* 8.79 (d, *J* = 1.5 Hz, 1-H), 8.27 (d, *J* = 4.3 Hz, 1-H), 7.90 (d, *J* = 7.6 Hz, 1-H), 7.83 (s, 1-H), 7.08 (dd, *J* = 7.6/4.6 Hz, 1-H), 4.15 (t, *J* = 6.9 Hz, 2-H), 2.96 (t, *J* = 6.8 Hz, 2-H), 1.68 (quint, *J* = 7.0 Hz, 2-H), 1.28 (quint, *J* = 7.0 Hz, 2-H), 1.11-1.09 (m, 4-H) ppm; ¹³C-NMR (75 MHz, CDCl₃) *δ* 148.4, 146.3, 143.9, 132.4, 126.5, 123.3, 120.1, 50.6, 49.8, 29.5, 28.0, 25.5, 25.4 ppm.

### Example 6. General Procedure for the Preparation of Amines (6)

Water (6 eq) and triphenylphosphine (1.5 eq) were added to a solution of the corresponding azide (**5**)(1 eq) in tetrahydrofuran (0.3 M). The resulting mixture was heated at reflux for 3 h under magnetic stirring. The solvent was evaporated *in vacuo* and the residue was purified by column chromatography using EtOAc and MeOH/NH₄OH 98:2 as eluants to give the desired amine.

### 8-(4-(pyridin-3-yl)-1H-1,2,3-triazol-1-yl)octan-1-amine

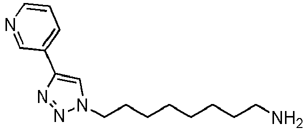

White solid; yield 69%; m.p. 71-72 °C; IR (KBr) 3244, 3123, 2922, 1574, 1455, 1051, 807, 706 cm⁻¹; ¹H-NMR (300 MHz, CD₃OD) *δ* 8.98 (br s, 1-H), 8.50-8.47 (m, 2-H), 8.26-8.23 (m, 1-H), 7.52-7.46 (m, 1-H), 4.44 (td, *J* = 7.0/3.4 Hz, 2-H), 2.56 (t, *J* = 7.3 Hz, 2-H), 1.93 (br s, 2-H), 1.42 (br s, 2-H), 1.33-1.30 (m, 8-H) ppm; ¹³C-NMR (75 MHz, CD₃OD) *δ* 149.7, 147.4, 145.5, 135.0, 128.9, 125.8, 123.2, 51.7, 42.7, 33.9, 31.4, 30.5, 30.2, 28.0, 27.6 ppm.

### 7-(4-(pyridin-3-yl)-1H-1,2,3-triazol-1-yl)heptan-1-amine

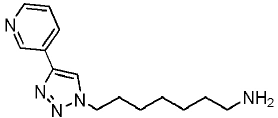

White solid; yield 64%; m.p. 89-91 °C; IR (KBr) 2926, 2849, 1560, 1474, 1027, 808 cm⁻¹; ¹H-NMR (300 MHz, CDCl₃) *δ* 8.96 (d, *J* = 0.9 Hz, 1-H), 8.51 (dd, *J* = 4.9/1.5 Hz, 1-H), 8.16 (dt, *J* = 8.0/1.5 Hz, 1-H), 7.84 (s, 1-H), 7.34-7.31 (m, 1-H), 4.37 (t, *J* = 7.4 Hz, 2-H), 2.64 (t, *J* = 7.0 Hz, 2-H), 2.34 (br s, 2-H), 1.91 (br quint, 2-H), 1.40 (br quint, 2-H), 1.30 (br s, 6-H) ppm; ¹³C-NMR (75 MHz, CDCl₃) *δ* 148.9, 146.8, 144.5, 132.9, 126.8, 123.7, 120.1, 50.4, 41.6, 32.6, 30.1, 28.6, 26.5, 26.3 ppm.

### 6-(4-(pyridin-3-yl)-1H-1,2,3-triazol-1-yl)hexan-1-amine

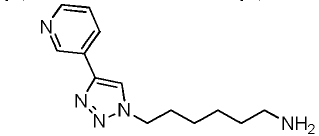

Off-white solid; yield 83%; m.p. 128-129 °C; IR (KBr) 2921, 2844, 1458, 1055, 1033, 809 cm⁻¹; ¹H-NMR (300 MHz, CD₃OD) *δ* 8.99 (d, *J* = 1.5 Hz, 1-H), 8.49 (s, 1-H), 8.48 (dd, *J* = 5.8/1.2 Hz, 1-H), 8.22 (d, *J* = 8.0 Hz, 1-H), 7.47 (dd, *J* = 7.7/4.9 Hz, 1-H), 4.44 (t, *J* = 7.0 Hz, 2-H), 2.68 (t, *J* = 6.7 Hz, 2-H), 1.94 (m, 2-H), 1.50 (m, 2-H), 1.42-1.34 (m, 4-H) ppm; ¹³C-NMR (75 MHz, CD₃OD) *δ* 149.5, 147.2, 145.2, 134.7, 128.7, 125.5, 123.0, 51.4, 41.8, 31.9, 31.1, 27.1, 27.0 ppm.

### Synthesis of alcoholic derivatives (8), (9), (10), (11) - (Scheme b)

### Example 7. General Procedure for the Preparation of Methanesulfonyl Esters (7)

Under a nitrogen atmosphere the corresponding alcohol (**4**) (1 eq) was dissolved in dry dichloromethane (0.3 M). To the cooled (0 °C) solution, TEA (2 eq) and Methanesulfonyl chloride (1.8 eq) were added. The resulting mixture was stirred for 0.5 h at room temperature and quenched by addition of sat. aq. NaHCO₃. The two phases were separated and the aqueous layer was extracted with dichloromethane (x2). The combined organic layers were washed with brine, dried over sodium sulfate and concentrated *in vacuo.* The crude was purified by column chromatography using PE/EtOAc 5:5 and PE/EtOAc 2:8 as eluants to give the desired methanesulfonyl ester.

### 8-(4-(pyridin-3-yl)-1H-1,2,3-triazol-1-yl)octyl methanesulfonate

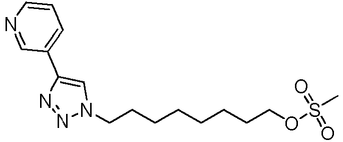

Yellowish oil; yield 95%; IR (KBr) 2940, 1552, 1480, 1189, 1060, 780 cm⁻¹; ¹H-NMR (300 MHz, CDCl₃) *δ* 8.87 (d, *J* = 1.5 Hz, 1-H), 8.37 (dd, *J* = 4.6/1.5 Hz, 1-H), 7.99 (dt, *J* = 8.0/1.8 Hz, 1-H), 7.85 (s, 1-H), 7.18 (dd, *J* = 7.6/4.6 Hz, 1-H), 4.24 (t, *J* = 7.0 Hz, 2-H), 4.02 (t, *J* = 6.4 Hz, 2-H), 2.83 (s, 3-H), 1.79-1.75 (m, 2-H), 1.55-1.51 (m, 2-H), 1.15 (br s, 8-H) ppm; ¹³C-NMR (75 MHz, CDCl₃) *δ* 150.5, 148.4, 145.9, 134.3, 128.3, 125.2, 121.8, 71.7, 51.8, 38.7, 31.6, 30.4, 30.1 (2-C), 27.6, 26.6 ppm.

### 7-(4-(pyridin-3-yl)-1H-1,2,3-triazol-1-yl)heptyl methanesulfonate

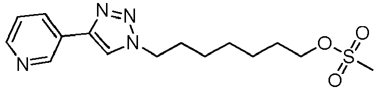

Yellow oil; yield 90%; IR (KBr) 3064, 2933, 1636, 1457, 1173, 1089, 770, 685 cm⁻¹; ¹H-NMR (300 MHz, CDCl₃) *δ* 8.89 (s, 1-H), 8.40 (d, *J* = 4.3 Hz, 1-H), 8.04 (d, *J* = 8.0 Hz, 1-H), 7.87 (s, 1-H), 7.22 (dd, *J* = 7.6/4.9 Hz, 1-H), 4.27 (t, *J* = 7.0 Hz, 2-H), 4.05 (t, *J* = 6.4 Hz, 2-H), 2.87 (s, 3-H), 1,81 (m, 2-H), 1.57 (m, 2-H), 1.23 (br s, 6-H) ppm; ¹³C-NMR (75 MHz, CDCl₃) *δ* 150.4, 148.3, 146.0, 134.5, 128.5, 125.3, 121.9, 71.7, 51.8, 38.8, 31.5, 30.4, 29.7, 27.6, 26.6 ppm.

### 6-(4-(pyridin-3-yl)-1H-1,2,3-triazol-1-yl)hexyl methanesulfonate

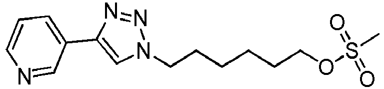

Yellow oil; yield 88%; IR (KBr) 2935, 1554, 1485, 1194, 1057, 784 cm⁻¹; ¹H-NMR (300 MHz, CDCl₃) *δ* 8.93 (d, *J* = 2.2 Hz, 1-H), 8.45 (d, *J* = 4.9/1.5 Hz, 1-H), 8.08 (dt, *J* = 8.0/1.8 Hz, 1-H), 7.86 (s, 1-H), 7.27 (dd, *J* = 8.0/4.9 Hz, 1-H), 4.33 (t, *J* = 7.4 Hz, 2-H), 4.10 (t, *J* = 6.4 Hz, 2-H), 2.90 (s, 3-H), 1,88 (quint, *J* = 7.4 Hz, 2-H), 1.64 (quint, *J* = 7.6 Hz, 2-H), 1.37-1.29 (m, 4-H) ppm; ¹³C-NMR (75 MHz, CDCl₃) *δ* 150.7, 148.6, 146.2, 134.5, 128.5, 125.4, 121.9, 71.5, 51.9, 38.9, 31.6, 30.4, 27.4, 26.4 ppm.

### Example 8. General Procedure for the Preparation of Ether Analogues (8)

Under a nitrogen atmosphere, the corresponding methanesulfonyl ester (7) (1 eq) was dissolved in dry *N,N*'-dimethylformamide (0.4 M). While stirring at room temperature, the appropriate phenol derivative (1.1 eq) and K₂CO₃ (1.1 eq) were added and the reaction was heated at 80 °C overnight. After completion of the reaction, water was added and the desired product was extracted with EtOAc (x3).The combined organic layers were washed with water (x1), brine (x1), dried over sodium sulfate and concentrated *in vacuo.* Finally the crude material was purified by column chromatography using PE/EtOAc 5:5 and PE/EtOAc 2:8 as eluants to give the desired ether.

### 3-(1-(8-([1,1'-biphenyl]-2-yloxy)octyl)-1H-1,2,3-triazol-4-yl)pyridine

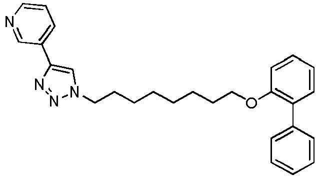

White solid; yield 53%; m.p. 69-70 °C; IR (KBr) 2939, 1582, 1471, 1222, 1055, 1031, 753 cm⁻¹; ¹H-NMR (300 MHz, CDCl₃) *δ* 8.99 (d, *J* = 1.8 Hz, 1-H), 8.54 (dd, *J* = 3.4/1.5 Hz, 1-H), 8.16 (dt, *J* = 8.0/1.8 Hz, 1-H), 7.78 (s, 1-H), 7.54-7.51 (m, 2-H), 7.38-7.24 (m, 6-H), 7.01-6.92 (m, 2-H), 4.34 (t, *J* = 7.0 Hz, 2-H), 3.91 (t, *J* = 6.4 Hz, 2-H), 1.91-1.87 (m, 2-H), 1.71-1.62 (m, 2-H), 1.36-1.21 (m, 8-H) ppm; ¹³C-NMR (75 MHz, CDCl₃) *δ* 157.5, 150.7, 148.6, 146.1, 140.1, 134.4, 132.4, 132.3, 131.1, 130.1, 129.3, 128.3, 128.2, 125.3, 122.3, 121.4, 114.1, 69.8, 52.0, 31.8, 30.6, 30.4, 30.3, 27.8, 27.4 ppm; MS (ESI) *m*/*z* 427 (M+H)⁺.

### 3-(1-(7-([1,1'-biphenyl]-2-yloxy)heptyl)-1H-1,2,3-triazol-4-yl)pyridine

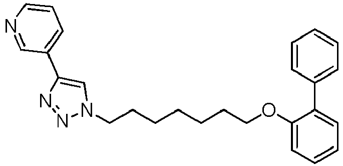

Yellowish solid; yield 44%; m.p. 79-80 °C; IR (KBr) 3125, 2931, 2852, 1577, 1259, 700 cm⁻¹; ¹H-NMR (300 MHz, CDCl₃) *δ* 9.01 (br s, 1-H), 8.56 (br s, 1-H), 8.23 (dt, *J=* 6.3/1.6 Hz, 1-H), 7.80 (s, 1-H), 7.53-7.50 (m, 2-H), 7.38-7.25 (m, 6-H), 7.03-6.93 (m, 2-H), 4.36 (t, *J* = 7.1 Hz, 2-H), 3.93 (t, *J* = 6.0 Hz, 2-H), 1,89 (quint, *J* = 6.8 Hz, 2-H), 1.68 (quint, *J* = 6.8 Hz, 2-H), 1.39-1.32 (m, 6-H) ppm; ¹³C-NMR (75 MHz, CDCl₃) *δ* 157.5, 150.2, 148.2, 146.1, 140.2, 134.9, 132.5, 132.4, 131.2, 130.1, 129.3, 129.1, 128.3, 125.5, 122.4, 121.4, 114.1, 69.8, 52.1, 31.6, 30.5, 30.1, 27.8, 27.4 ppm; MS (ESI) *m*/*z* 414 (M+H)⁺.

### 3-(1-(6-([1,1'-biphenyl]-2-yloxy)hexyl)-1H-1,2,3-triazol-4-yl)pyridine

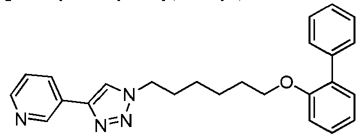

Off-white solid; yield 50%; m.p. 87-88 °C; IR (KBr) 3134, 2937, 1583, 1502, 1262, 749 cm⁻¹; ¹H-NMR (300 MHz, CDCl₃) *δ* 8.93 (br s, 1-H), 8.50 (br s, 1-H), 8.10 (d, *J* = 8.0 Hz, 1-H), 7.69 (s, 1-H), 7.48-7.45 (m, 2-H), 7.33-7.19 (m, 6-H), 6.97-6.87 (m, 2-H), 4.23 (t, *J* = 7.3 Hz, 2-H), 3.87 (t, *J* = 6.1 Hz, 2-H), 1,81 (quint, *J* = 7.4 Hz, 2-H), 1.63 (quint, *J* = 7.6 Hz, 2-H), 1.35 (quint, *J* = 7.6 Hz, 2-H) 1.24 (quint, *J* = 7.0 Hz, 2-H), ppm; ¹³C-NMR (75 MHz, CDCl₃) *δ* 154.9, 148.0, 145.9 143.5, 137.7, 132.0, 129.9, 129.8, 128.6, 127.6, 126.8, 126.0, 125.8, 122.8, 120.0, 119.0, 111.6, 67.1, 49.3, 29.1, 27.8, 24.9, 24.4 ppm; MS (ESI) *m*/*z* 399 (M+H)⁺.

### 3-(1-(8-phenoxyoctyl)-1H-1,2,3-triazol-4-yl)pyridine

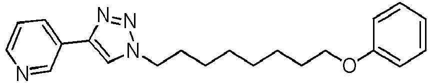

Pale yellowish solid; yield 42%; m.p. 102-103 °C; IR (KBr) 3131, 2928, 2852, 1602, 1500, 1249, 1175, 809, 753 cm⁻¹; ¹H-NMR (300 MHz, CDCl₃) *δ* 8.96 (d, *J* = 1.8 Hz, 1-H), 8.52 (d, *J* = 3.7 Hz, 1-H), 8.15 (m, 1-H), 7.80 (s, 1-H), 7.35-7.21 (m, 3-H), 6.91-6.84 (m, 3-H), 4.40-4.32 (m, 2-H), 3.93-3.85 (m, 2-H), 1.90 (br.s, 2-H), 1.71 (br s, 2-H), 1.32 (br s, 8-H) ppm; ¹³C-NMR (75 MHz, CDCl₃) *δ* 160.4, 150.6, 148.5, 146.1, 134.4, 130.9, 128.4, 125.2, 122.0, 121.3, 115.9, 69.2, 52.0, 31.8, 30.7, 30.6, 30.4, 27.9, 27.4 ppm; MS (ESI) *m*/*z* 351 (M+H)⁺.

### 3-(1-(7-phenoxyheptyl)-1H-1,2,3-triazol-4-yl)pyridine

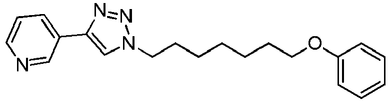

White solid; yield 56%; m.p. 90.5-91 °C; IR (KBr) 3129, 2933, 2853, 1597, 1407, 1236, 754 cm⁻¹; ¹H-NMR (300 MHz, CDCl₃) *δ* 9.00 (br s, 1-H), 8.56 (d, *J* = 4.0 Hz, 1-H), 8.24 (d, *J* = 8.0 Hz, 1-H), 7.85 (s, 1-H), 7.38 (dd, *J* = 7.6/4.9 Hz, 1-H), 7.28-7.22 (m, 2-H), 6.93-6.85 (m, 3-H), 4.41 (t, *J* = 7.0 Hz, 2-H), 3.92 (t, *J* = 6.4 Hz, 2-H), 1.97 (quint, *J* = 7.0 Hz, 2-H), 1.76 (quint, *J* = 7.0 Hz, 2-H), 1.46-1.39 (m, 6-H) ppm; ¹³C-NMR (75 MHz, CDCl₃) *δ* 160.6, 150.2, 148.2, 146.0, 134.9, 131.0, 128.7, 125.5, 122.1, 121.5 116.0, 69.2, 52.1, 31.8, 30.7, 30.3, 28.0, 27.4 ppm; MS (ESI) *m*/*z* 337 (M+H)⁺.

### 3-(1-(6-phenoxyhexyl)-1H-1,2,3-triazol-4-yl)pyridine

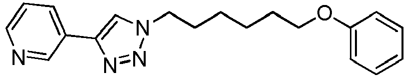

White solid; yield 48%; m.p. 74-75 °C; IR (KBr) 3118, 2931, 2853, 1601, 1498, 1245, 755 cm⁻¹; ¹H-NMR (300 MHz, CDCl₃) *δ* 8.97 (d, *J* = 1.6 Hz, 1-H), 8.52 (dd, *J* = 4.9/1.5 Hz, 1-H), 8.15 (dt, *J* = 7.6/1.8 Hz, 1-H), 7.82 (s, 1-H), 7.32 (dd, *J* = 8.0/4.9 Hz, 1-H), 7.25-7.20 (m, 2-H), 6.91-6.82 (m, 3-H), 4.38 (t, *J* = 7.0 Hz, 2-H), 3.89 (t, *J* = 6.1 Hz, 2-H), 1.95 (quint, *J* = 7.4 Hz, 2-H), 1.74 (quint, *J* = 6.4 Hz, 2-H), 1.53-1.39 (m, 4-H) ppm; ¹³C-NMR (75 MHz, CDCl₃) *δ* 159.0, 149.0, 146.9, 144.6, 133.0, 129.4, 126.9, 123.8, 120.6, 120.0 114.5, 67.4, 50.4, 30.2, 29.0, 26.2, 25.5 ppm; MS (ESI) *m*/*z* 323 (M+H)⁺.

### Example 9. General Procedure for the Preparation of 2-Iodophenyl Ethers (9)

Under a nitrogen atmosphere NaH (60% in mineral oil, 2 eq) was added portionwise to a stirred solution of 2-iodophenol (1.3 eq) in DMF dry (0.35 M) at 0 °C. After 20 minutes, a solution of the corresponding Methanesulfonyl Esters (7) (1 eq) in DMF dry (0.35 M) was added slowly.
The reaction mixture was vigorously stirred at 25 °C for 24 h.
After completion of the reaction water was added dropwise and the product was extracted with EtOAc (x3). The combined organic layers were washed with water (x1), brine (x1), dried over sodium sulfate and concentrated *in vacuo.* Finally the crude material was purified by column chromatography using PE/EtOAc 7:3 and PE/EtOAc 2:8 as eluants to afford the desired 2-iodophenylether.

### 3-(1-(6-(2-iodophenoxy)hexyl)-1H-1,2,3-triazol-4-yl)pyridine

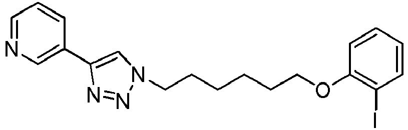

Pale yellow oil; yield 60%; IR (KBr) 2945, 2861, 1580, 1466, 1437, 1283, 1249, 1047, 807, 757 cm⁻¹; ¹H-NMR (300 MHz, CDCl₃) *δ* 8.97 (s, 1-H), 8.54 (d, *J* = 4.9 Hz, 1-H), 8.16 (dd, *J* = 7.9/1.8 Hz, 1-H), 7.83 (s, 1-H), 7.73 (dt, *J* = 7.6/1.5 Hz, 1-H), 7.36 (dd, *J* = 7.6/4.6 Hz, 1-H), 7.24 (t, *J* = 8.0 Hz, 1-H), 6.75 (d, *J* = 8.3 Hz, 1-H), 6.67 (t, *J* = 7.4 Hz, 1-H), 4.43 (t, *J* = 6.7 Hz, 2-H), 3.97 (t, *J* = 4.9 Hz, 2-H), 2.00 (quint, *J* = 7.4 Hz, 2-H), 1.81 (quint, *J* = 7.0 Hz, 2-H), 1.61 (quint, *J* = 7.0 Hz, 2-H), 1.44 (quint, *J* = 7.0 Hz, 2-H) ppm; ¹³C-NMR (75 MHz, CDCl₃) *δ* 157.5, 149.2, 147.1, 144.7, 139.5, 133.1, 129.6, 126.9, 123.9, 122.6, 120.1, 112.1, 86.7, 68.7, 50.5, 30.3, 28.8, 26.1, 25.7 ppm; MS (ESI) *m*/*z* 449 (M+H)⁺.

### Example 10. General Procedure for the Preparation of 2-Arylphenyl Ether Analogues (10)

Under a nitrogen atmosphere, the appropriate commercially boronic acid (1.3 eq), K₂CO₃ (3 eq) e Pd(PPh₃)₄ (0.1 eq) were added to a stirred solution of the corresponding 2-iodophenylether derivative (**9**) (1 eq) in CH₃CN/H₂O 1:1 (0,15 M) at 25 °C. The mixture was heated to 90 °C for 24-48 h. After completion of the reaction the mixture was filtered.
The solvent of the filtrate was removed by rotatory evaporation and the resulting aqueous layer was extracted with EtOAc (x3).
(When 3-carboxyphenylboronic acid or 4-carboxyphenylboronic acid were used for the reaction, the resulting aqueous layer was acidified dropwise with HCl 3 M to pH 4 before extraction).
The combined organic layers were washed with brine (x1), dried over sodium sulfate and concentrated *in vacuo.* Finally the crude material was purified by column chromatography to afford the desired ether.

### 2'-((6-(4-(pyridin-3-yl)-1H-1,2,3-triazol-1-yl)hexyl)oxy)-[1,1'-biphenyl]-3-carboxylic acid

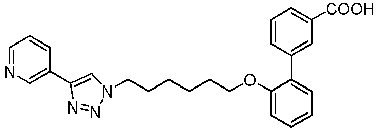

The compound was purified by column chromatography, using EtOAc and EtOAc/MeOH 95:5 as eluants to give a yellow solid; yield 57%; m.p. 130-131 °C; IR (KBr) 2934, 2867, 1707, 1499, 1455, 1251, 1056, 758 cm⁻¹; 1H-NMR (300 MHz, CDCl₃) *δ* 8.99 (d, J = 2.1 Hz, 1-H), 8.48 (dd, J = 4.9/1.5 Hz, 1-H), 8.40 (s, 1-H), 8.18 (d, J = 8.0 Hz, 1-H), 8.07 (d, J = 8.0 Hz, 1-H), 7.93 (s, 1-H), 7.68 (d, J = 7.6 Hz, 1-H), 7.49 (t, J = 7.6 Hz, 1-H), 7.35-7.29 (m, 3-H), 7.03 (t, J = 7.3 Hz, 1-H), 6.97 (d, J = 8.2 Hz, 1-H), 4.29 (t, J = 7.3 Hz, 2-H), 3.99 (t, J = 5.8 Hz, 2- H), 1.91 (quint, J = 7.6 Hz, 2-H), 1.71 (quint, J = 7.6 Hz, 2-H), 1.49 (quint, J = 7.9 Hz, 2-H), 1.33 (quint, J = 7.3 Hz, 2-H) ppm; ¹³C-NMR (75 MHz, CDCl₃) *δ* 170.1, 156.1, 147.4, 145.7, 144.0, 139.1, 134.2, 134.1, 131.8, 130.9, 130.4, 130.1, 129.2, 128.5, 128.4, 127.7, 124.4, 121.1, 120.7, 112.5, 68.2, 50.5, 30.3, 29.1, 26.3, 25.8 ppm; MS (ESI) *m*/*z* 443 (M+H)⁺.

### 2'-((6-(4-(pyridin-3-yl)-1H-1,2,3-triazol-1-yl)hexyl)oxy)-[1,1'-biphenyl]-4-carboxylic acid

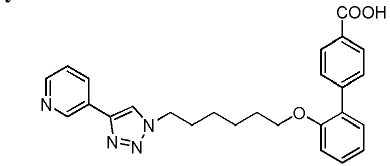

The compound was purified by column chromatography, using EtOAc and EtOAc/MeOH 95:5 as eluants to give a yellow solid; yield 55%; m.p. 174-175 °C; IR (KBr) 3040, 2937, 2860, 1701, 1405, 1280, 1172, 793 cm⁻¹; ¹H-NMR (300 MHz, CDCl₃) *δ* 9.11 (d, *J* = 1.8 Hz, 1-H), 8.52 (d, *J* = 4.9 Hz, 1-H), 8.37 (d, *J* = 8.0 Hz, 1-H), 8.15 (d, *J* = 8.0 Hz, 2-H), 8.02 (s, 1-H), 7.63 (d, *J* = 7.7 Hz, 2-H), 7.42 (dd, *J* = 7.9/4.9 Hz, 1-H), 7.37-7.31 (m, 2-H), 7.05 (t, *J* = 7.6 Hz, 1-H), 6.99 (d, *J* = 8.2 Hz, 1-H), 4.29, (t, *J* = 7.3 Hz, 2-H), 3.98 (t, *J* = 5.8 Hz, 2-H), 1.88 (quint, *J* = 7.3 Hz, 2-H), 1.70 (quint, *J* = 7.3 Hz, 2-H), 1.44-1.29 (m, 4-H) ppm; ¹³C-NMR (75 MHz, CDCl₃) *δ* 169.9, 156.1, 147.6, 146.0, 144.3, 143.8, 134.2, 130.8, 130.3, 130.0, 129.6 (2-C), 129.0, 127.8, 124.4, 121.3, 120.5, 68.5, 50.6, 30.4, 29.1, 26.3, 25.8 ppm; MS (ESI) *m*/*z* 443 (M+H)⁺.

### 3-(2-((6-(4-(pyridin-3-yl)-1H-1,2,3-triazol-1-yl)hexyl)oxy) phenyl)pyridine

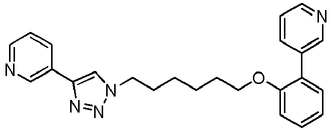

The compound was purified by column chromatography, using EtOAc and EtOAc/MeOH 95:5 as eluants, to give a yellow oil; yield 97%; IR (KBr) 2936, 2860, 1667, 1467, 1450, 1409, 1268, 1238, 756, 710 cm⁻¹; ¹H-NMR (300 MHz, CD₃OD) *δ* 8.99 (dd, *J* = 2.2/0.6 Hz, 1-H), 8.66 (dd, *J* = 2.4/0.9 Hz, 1-H), 8.50 (dd, *J* = 4.9/1.5 Hz, 1-H), 8.47 (s, 1-H), 8.43 (dd, *J* = 4.9/1.5 Hz, 1-H), 8.25 (dt, *J* = 7.9/2.1 Hz, 1-H), 7.95 (dt, *J* = 7.9/2.1 Hz, 1-H), 7.51 (ddd, *J* = 7.9/4.9/0.9 Hz, 1-H), 7.46 (ddd, *J* = 7.9/4.9/0.6 Hz, 1-H), 7.38-7.29 (m, 2-H), 7.08-7.00 (m, 2-H), 4.42 (t, *J* = 7.0 Hz, 2-H), 4.00 (t, *J* = 6.1 Hz, 2-H), 1.93 (quint, *J* = 6.1 Hz, 2-H), 1.72 (quint, *J* = 6.1 Hz, 2-H), 1.48-1.31 (m, 4-H) ppm; ¹³C-NMR (75 MHz, CD₃OD) *δ* 157.2, 150.4, 149.4, 147.9, 147.1, 145.2, 138.9, 136.4, 134.9, 131.4, 131.0, 128.6, 127.7, 125.5, 124.7, 123.2, 122.1, 113.7, 69.3, 51.4, 31.0, 29.9, 26.9, 26.5 ppm; MS (ESI) *m*/*z* 443 (M+H)⁺.

### 3-(1-(6-((2'-methyl-[1,1'-biphenyl]-2-yl)oxy)hexyl)-1H-1,2,3-triazol-4-yl)pyridine

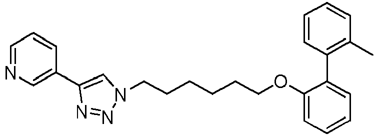

The compound was purified by column chromatography, using PE/EtOAc 5:5 and PE/EtOAc 3:7 as eluants, to give a light yellow oil; yield 83%; IR (KBr) 3057, 2927, 2860, 1480, 1439, 1262, 1228, 1119, 755, 542 cm⁻¹; ¹H-NMR (300 MHz, CDCl₃) *δ* 8.97 (br s, 1-H), 8.56 (d, *J* = 4.9 Hz, 1-H), 8.19 (dd, *J* = 7.9/1.5 Hz, 1-H), 7.72 (s, 1-H), 7.38-7.33 (m, 1-H), 7.30-7.12 (m, 6-H), 6.99 (t, *J* = 7.3 Hz, 1-H), 6.93 (d, *J* = 8.2 Hz, 1-H), 4.28 (t, *J* = 7.0 Hz, 2-H), 3.89 (t, *J* = 5.8 Hz, 2-H), 2.13 (s, 3-H), 1.81 (quint, *J* = 7.0 Hz,2-H), 1.60 (quint, *J* = 6.7 Hz, 2-H), 1.29-1.25 (m, 4-H) ppm; ¹³C-NMR (75 MHz, CDCl₃) *δ* 156.1, 149.2, 147.1, 144.6, 139.0, 136.9, 133.0, 131.4, 131.0, 130.1, 129.4, 128.6, 127.1, 126.9, 125.3, 123.8, 120.7, 120.0, 112.3, 68.1, 50.3, 30.1, 28.8, 25.9, 25.3, 20.1 ppm; MS (ESI) *m*/*z* 413 (M+H)⁺.

### Example 11. General Procedure for the Preparation of Carbamate (11)

Biphenyl-2-isocyanate (1 eq) was added dropwise to a stirred solution of the corresponding intermediate (**4**) (1 eq) in CH₃CN (0.15 M). The mixture was heated to 50 °C overnight. After completion of the reaction, the mixture was concentrated *in vacuo.* The crude residue was purified by column chromatography using PE/EtOAc 2:8 as eluant, to give the desired carbamate.

### 8-(4-(pyridin-3-yl)-1H-1,2,3-triazol-1-yl)octyl[1,1'-biphenyl]-2-ylcarbamate

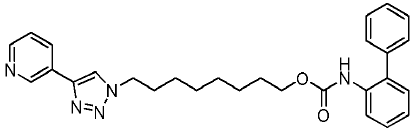

White solid; yield 76%; m.p. 81-85 °C; IR (KBr) 3140, 3055, 2930, 2859, 1713, 1586, 1533, 1450, 1223, 1067, 760 cm⁻¹; ¹H-NMR (300 MHz, CDCl₃) *δ* 8.98 (s, 1-H), 8.57 (br s, 1-H), 8.21 (d, *J* = 7.4 Hz, 1-H), 8.10 (d, *J* = 7.9 Hz, 1-H), 7.81 (s, 1-H), 7.49-7.32 (m, 7-H), 7.20 (d, *J* = 7.4 Hz, 1-H), 7.11 (t, *J* = 7.4 Hz, 1-H), 6.61 (br s, 1-H), 4.40 (t, *J* = 7.0 Hz, 2-H), 4.09 (t, *J* = 7.0 Hz, 2-H), 1.95 (quint, *J* = 6.7 Hz, 2-H), 1.60 (quint, *J* = 6.7 Hz, 2-H), 1.33-1.31 (m, 8-H) ppm; ¹³C-NMR (75 MHz, CDCl₃) *δ* 153.8, 149.3, 147.1, 144.8, 138.3, 135.0, 133.1, 131.6, 130.3, 129.4 (2-C), 129.2, 128.6, 128.0, 126.9, 123.9, 123.5, 119.8, 65.3, 50.6, 30.4, 29.0, 28.9, 28.9, 26.5, 25.7 ppm; MS (ESI) *m*/*z* 469 (M+H)⁺.

### 7-(4-(pyridin-3-yl)-1H-1,2,3-triazol-1-yl)heptyl[1,1'-byphenyl]-2-ylcarbamate

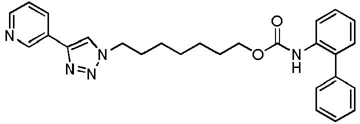

White solid; yield 63%; m.p. 105-106 °C; IR (KBr) 3131, 3040, 2933, 2854, 1715, 1587, 1537, 1451, 1221, 1072, 701 cm⁻¹; ¹H-NMR (300 MHz, CDCl₃) *δ* 8.98 (d, *J* = 2.2 Hz, 1-H), 8.56 (dd, *J* = 4.9/1.9 Hz, 1-H), 8.21 (dt, *J* = 8.0/2.1 Hz, 1-H), 8.10 (d, *J* = 7.7 Hz, 1-H), 7.81 (s, 1-H), 7.49-7.32 (m, 7-H), 7.20 (dd, *J* = 7.6/1.8 Hz, 1-H), 6.60 (br s, 1-H), 4.41 (t, *J* = 7.3 Hz, 2- H), 4.09 (t, *J* = 6.7 Hz, 2-H), 1.95 (quint, *J* = 6.7 Hz, 2-H), 1.61 (quint, *J* = 7.3 Hz, 2-H), 1.36 (br s, 6-H) ppm; ¹³C-NMR (75 MHz, CDCl₃) *δ* 153.8, 149.3, 147.2, 144.8, 138.3, 135.0, 133.1, 131.7, 130.3, 129.4 (2-C), 129.2, 128.6, 128.0, 127.0, 123.9, 123.5, 119.9, 65.2, 50.6, 30.3, 28.8, 28.7, 26.5, 25.7 ppm; MS (ESI) *m*/*z* 456 (M+H)⁺.

### 6-(4-(pyridin-3-yl)-1H-1,2,3-triazol-1-yl)hexyl[1,1'-byphenyl]-2-ylcarbamate

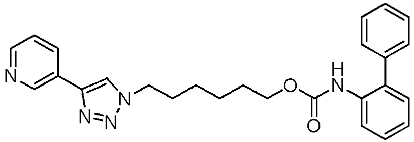

White solid; yield 60%; m.p. 143-146 °C; IR (KBr) 3128, 3055, 2931, 2860, 1719, 1587, 1537, 1224, 1067, 760 cm⁻¹; ¹H-NMR (300 MHz, CDCl₃) *δ* 8.98 (s, 1-H), 8.56 (d, *J* = 4.3 Hz, 1-H), 8.20 (d, *J* = 7.9 Hz, 1-H), 8.10 (d, *J* = 7.7 Hz, 1-H), 7.81 (s, 1-H), 7.49-7.32 (m, 7-H), 7.20 (d, *J* = 6.4 Hz, 1-H), 7.11 (t, *J* = 7.3 Hz, 1-H), 6.60 (br s, 1-H), 4.40 (t, *J* = 7.0 Hz, 2-H), 4.09 (t, *J* = 6.7 Hz, 2-H), 1.95 (t, *J* = 7.0 Hz, 2-H), 1.62 (t, *J* = 6.7 Hz, 2-H), 1.39-1.36 (m, 4-H) ppm; ¹³C-NMR (75 MHz, CDCl₃) *δ* 153.8, 149.3, 147.1, 144.8, 138.2, 134.9, 133.1, 131.7, 130.3, 129.3 (2-C), 129.2, 128.5, 128.0, 126.8, 123.8, 123.5, 119.9, 65.0, 50.5, 30.3, 28.7, 26.4, 26.2, 25.3 ppm; MS (ESI) *m*/*z* 442 (M+H)⁺.

### Synthesis of 1,4-disubstituted triazoles (12) - (Scheme c)

### Example 12. General Procedure for the Preparation of the 1,4-disubstituted Triazoles (12)

The corresponding azide (5) (1 eq) and the appropriate alkyine (1 eq) were suspended in a mixture of water/*tert*-butanol (1:1) Sodium ascorbate (0.1 eq), of a freshly prepared 1 M solution in water, was added, followed by the addition of copper (II) sulfate pentahydrate (0.01 eq). The resulting mixture was heated to 40 °C and vigorously stirred for 24 h. The reaction mixture was then diluted with water, cooled, and the precipitate was collected by filtration, washed with diethyl ether and dried *in vacuo.* When addition of water failed to precipitate the triazole, the reaction mixture was extracted with EtOAc (x3). The combined organic layers were washed with brine (x1) dried over sodium sulfate and concentrated *in vacuo.* Finally the crude was purified by column chromatography using PE/EtOAc 2:8 e EtOAc as eluants to give the desired triazole.

### 3-(1-(7-(4-phenyl-1H-1,2,3-triazol-1-yl)heptyl)-1H-1,2,3-triazol-4-yl)pyridine

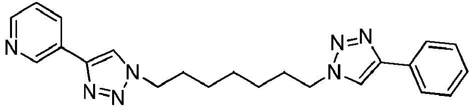

The compound precipitated as a white solid; yield 60%; m.p. 143-144 °C; IR (KBr) 3115, 2929, 2850, 1463, 1218, 807, 695 cm⁻¹; ¹H-NMR (300 MHz, (CD₃)₂SO) *δ* 9.08 (br s, 1-H), 8.68 (s, 1-H), 8.55 (s, 1-H), 8.18 (d, *J* = 8.0 Hz, 1-H), 7.80 (d, *J* = 7.0 Hz, 2-H), 7.48 (br s, 1-H), 7.41 (t, *J* = 7.3 Hz, 2-H), 7.29 (t, *J* = 7.3 Hz, 1-H), 4.36 (m, 4-H), 1.82 (br t, 4-H), 1.24 (br s, 6-H) ppm; ¹³C-NMR (75 MHz, (CD₃)₂SO) *δ* 150.9, 148.5, 148.4, 145.7, 135.1, 134.4 (2-C), 133.0, 131.0, 129.9, 127.2, 124.1, 123.3, 51.7, 51.6, 31.4, 31.6, 29.8, 27.9, 27.8 ppm; MS (ESI) *m*/*z* 388 (M+H)⁺.

### 3-(1-(6-(4-phenyl-1H-1,2,3-triazol-1-yl)hexyl)-1H-1,2,3-triazol-4-yl)pyridine

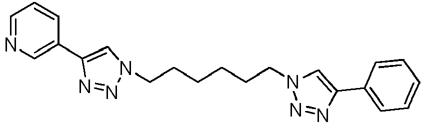

The compound precipitated as a yellowish solid; yield 88%; m.p. 172-173 °C; IR (KBr) 3117, 2929, 2854, 1464, 1215, 1079, 764, 695 cm⁻¹; ¹H-NMR (300 MHz, (CD₃)₂SO) *δ* 8.60 (br s, 1-H), 8.46 (br s, 1-H), 8.19 (d, *J* = 6.4 Hz, 1-H), 7.81 (d, *J* = 7.0 Hz, 2-H), 7.54 (m, 1-H), 7.41 (t, *J* = 7.0 Hz, 2-H), 7.31 (d, *J* = 7.0 Hz, 1-H), 4.39-4.32 (m, 4-H), 1.88 (br s, 4-H), 1.34 (br s, 4-H) ppm; ¹³C-NMR (75 MHz, (CD₃)₂SO) *δ* 150.8, 148.6, 148.3, 145.7, 135.1, 134.4 (2-C), 133.0, 131.0, 129.9, 127.2, 124.1, 123.3, 49.4, 49.3, 29.2 (2-C), 25.1 (2-C) ppm; MS (ESI) *m*/*z* 374 (M+H)⁺.

### 3-(1-(8-(4-([1,1'-byphenyl]-2-yl)-1H-1,2,3-triazol-1-yl)octyl)-1H-1,2,3-triazol-4-yl)pyridine

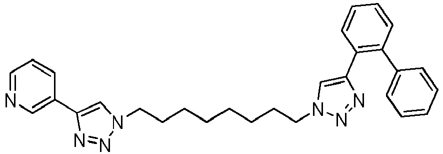

White solid; yield 49%; m.p. 136-140 °C; IR (KBr) 3122, 3033, 2933, 2854, 1466, 1452, 1217, 1073, 1053, 763, 702 cm⁻¹; ¹H-NMR (300 MHz, CDCl₃) *δ* 8.99 (s, 1-H), 8.56 (d, *J* = 4.6 Hz, 1-H), 8.20 (dt, *J* = 7.9/1.8 Hz, 1-H), 8.11 (dd, *J* = 7.6/1.2 Hz, 1-H), 7.84 (s, 1-H), 7.55-7.21 (m, 9-H), 6.37 (s, 1-H), 4.40 (t, *J* = 7.0 Hz, 2-H), 4.13 (t, *J* = 7.0 Hz, 2-H), 1.94 (quint, *J* = 7.0 Hz, 2-H), 1.72-1.62 (m, 4-H), 1.31-1.13 (m, 6-H) ppm; ¹³C-NMR (75 MHz, CDCl₃) *δ* 149.1, 147.0, 146.3, 144.7, 141.8, 140.2, 133.0, 130.2, 129.3, 129.2, 128.6, 128.4, 128.0, 127.9, 127.3, 126.9, 123.8, 122.2, 120.0, 50.5, 49.9, 30.2, 30.0, 28.7, 28.6, 26.3, 26.0 ppm; MS (ESI) *m*/*z* 478 (M+H)⁺.

### 3-(1-(7-(4-([1,1'-byphenyl]-2-yl)-1H-1,2,3-triazol-1-yl)heptyl)-1H-1,2,3-triazol-4-yl)pyridine

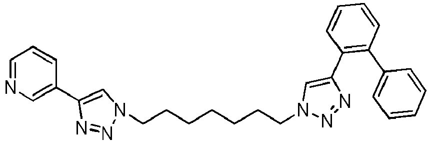

Off-white solid; yield 47%; m.p. 101-103 °C; IR (KBr) 3124, 3032, 2930, 2853, 1475, 1437, 1219, 1051, 769, 701 cm⁻¹; ¹H-NMR (300 MHz, CD₃OD) *δ* 8.99 (s, 1-H), 8.50-8.49 (m, 2-H), 8.25 (d, *J* = 8.0 Hz, 1-H), 7.80-7.78 (m, 1-H), 7.52-7.42 (m, 3-H), 7.34-7.28 (m, 4-H), 7.15-7.13 (m, 2-H), 6.89 (s, 1-H), 4.47 (t, *J* = 7.0 Hz, 2-H), 4.21 (t, *J* = 6.7 Hz, 2-H), 1.94 (quint, *J* = 7.0 Hz, 2-H), 1.69 (quint, *J* = 6.7 Hz, 2-H), 1.33-1.30 (m, 4-H), 1.16-1.11 (m, 2-H) ppm; ¹³C-NMR (75 MHz, CD₃OD) *δ* 149.5, 147.6, 147.2, 145.3, 142.7, 142.3, 134.8, 131.3, 130.4, 130.2, 130.0, 129.5, 129.4, 128.8, 128.7, 128.4, 125.6, 124.3, 123.0, 51.4, 50.9, 31.0, 30.9, 29.2, 27.2, 26.9 ppm; MS (ESI) *m*/*z* 464 (M+H)⁺.

### 3-(1-(6-(4-([1,1'-byphenyl]-2-yl)-1H-1,2,3-triazol-1-yl)hexyl)-1H-1,2,3-triazol-4-yl)pyridine

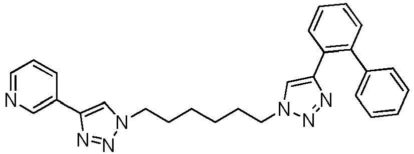

Off white solid; yield 68%; m.p. 139-140 °C; IR (KBr) 3129, 3030, 2935, 2865, 1471, 1451, 1219, 1053, 764, 702 cm⁻¹; ¹H-NMR (300 MHz, (CD₃)₂SO) *δ* 9.14 (br s, 1-H), 8.71 (s, 1-H), 8.60 (br s, 1-H), 8.21 (d, *J* = 8.0 Hz, 1-H), 7.82-7.79 (m, 1-H), 7.49-7.41 (m, 3-H), 7.34-7.29 (m, 4-H), 7.17-7.14 (m, 2-H), 7.02 (s, 1-H), 4.40 (t, *J* = 6.7 Hz, 2-H), 4.19 (t, *J* = 6.7 Hz, 2-H), 1.82 (quint, *J* = 7.3 Hz, 2-H), 1.61 (quint, *J* = 7.3 Hz, 2-H), 1.22 (quint, *J* = 7.3 Hz, 2-H), 1.09 (quint, *J* = 7.3 Hz, 2-H) ppm; ¹³C-NMR (75 MHz, (CD₃)₂SO) *δ* 148.8, 146.3, 145.3, 143.5, 141.0, 140.2, 132.3, 130.2, 129.3, 129.0, 128.8, 128.3, 128.1, 127.7, 127.2, 126.3, 124.8, 122.9, 121.9, 49.6, 49.0, 29.4 (2-C), 25.2, 25.0 ppm; MS (ESI) *m*/*z* 450 (M+H)⁺.

### Synthesis of amide derivatives (13), (15), (16), thioamides (14), sulfonamide derivatives (17), (18), secondary amines (19), ureas (20) - (Scheme d)

### Example 13. General Procedure for the Preparation of Amides (13)

Under nitrogen atmosphere, the corresponding amine (**6**) (1 eq) was dissolved in dry dichloromethane (0.4 M). While stirring at room temperature the corresponding carboxylic acid (1.1 eq) was added, followed by the addition of EDCl *N*-(3-dimethylaminopropyl)-*N*'-ethylcarbodimide hydrochloride (1 eq) and DMAP (dimethylaminopyridine) (1 eq). The reaction mixture was stirred overnight at 40 °C. After completion of the reaction, water was added and the aqueous layer was extracted with dichloromethane (x3). The combined organic layers were washed with 2 M NaOH (x1), brine (x1), dried over sodium sulfate and concentrated *in vacuo.* The crude material was purified by column chromatography using EtOAc as eluant to give the desired amide.

### N-(8-(4-(pyridin-3-yl)-1H-1,2,3-triazol-1-yl)octyl)-[1,1'-biphenyl]-2-carboxamide

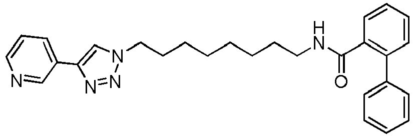

Colorless oil; yield 63%; IR (KBr) 3295, 3130, 2851, 1641, 1541, 1310, 802, 700 cm⁻¹; ¹H-NMR (300 MHz, CDCl₃) *δ* 8.93 (s, 1-H), 8.48 (d, *J* = 4.6 Hz, 1-H), 8.13 (d, *J* = 8.0 Hz, 1-H), 7.84 (s, 1-H), 7.62 (d, *J* = 7.4 Hz 1-H) 7.42-7.27 (m, 10-H), 5.38 (br t, 1-H), 4.35 (t, *J* = 7.4 Hz, 2-H), 3.07 (quart, *J* = 6.8 Hz, 2-H), 1.89 (quint, *J* = 7.0 Hz, 2-H), 1.25-1.09 (m, 8-H), 0.96 (br s, 2-H) ppm; ¹³C-NMR (75 MHz, CDCl₃) *δ* 171.0, 150.7, 148.6, 146.2, 141.9, 140.9, 137.6, 134.5, 131.7, 131.5, 130.3 (2-C), 130.1, 129.3, 129.1, 128.5, 125.3, 121.5, 52.1, 41.3, 31.8, 30.5, 30.4, 30.3, 28.1, 27.9 ppm; MS (ESI) *m*/*z* 454 (M+H)⁺.

### N-(7-(4-(pyridin-3-yl)-1H-1,2,3-triazol-1-yl)heptyl)-[1,1'-biphenyl]-2-carboxamide

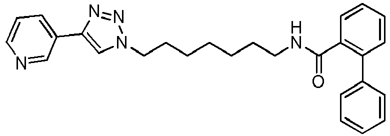

White solid; yield 53%; m.p. 72.9-73.5 °C; IR (KBr) 3291, 3130, 2930, 2853, 1633, 1539, 1449, 743, 700 cm⁻¹; ¹H-NMR (300 MHz, CDCl₃) *δ* 8.73 (br s, 1-H), 8.24 (d, *J* = 3.1 Hz, 1-H), 7.91 (d, *J* = 8.0 Hz, 1-H), 7.70 (s, 1-H), 7.38 (d, *J* = 7.3 Hz, 1-H), 7.22-7.06 (m, 9-H), 5.48 (br s, 1-H), 4.14 (t, *J* = 7.0 Hz, 2-H), 2.88 (quart, *J* = 6.7 Hz, 2-H), 1.66 (br s, 2-H), 1.00-0.76 (m, 8-H) ppm; ¹³C-NMR (75 MHz, CDCl₃) *δ* 168.4, 147.9, 145.8, 143.4, 139.2, 138.2, 135.0, 131.8, 129.0, 128.8, 127.6, 127.4, 126.5, 126.3, 125.8, 122.6, 119.1, 49.3, 38.5, 29.0, 27.7, 27.3, 25.3, 25.1 ppm; MS (ESI) *m*/*z* 440 (M+H)⁺.

### N-(6-(4-(pyridin-3-yl)-1H-1,2,3-triazol-1-yl)hexyl)-[1,1'-biphenyl]-2-carboxamide

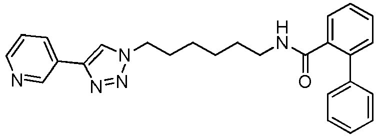

White solid; yield 49%; m.p. 91-92 °C; IR (KBr) 3305, 3067, 2922, 2847, 1647, 1541, 1309, 809, 701 cm⁻¹; ¹H-NMR (300 MHz, CDCl₃) *δ* 8.93 (br s, 1-H), 8.47 (br s, 1-H), 8.13 (dd, *J* = 4.0/1.8 Hz, 1-H), 7.85 (s, 1-H), 7.59 (d, *J* = 7.6 Hz, 1-H), 7.43-7.26 (m, 9-H), 5.48 (br s, 1-H), 4.31 (t, *J* = 7.4 Hz, 2-H), 3.07 (quart, *J* = 6.7 Hz, 2-H), 1.81 (quint, *J* = 7.0 Hz, 2-H), 1.23-1.09 (m, 4-H), 1.05-0.98 (m, 2-H) ppm; ¹³C-NMR (75 MHz, CDCl₃) *δ* 169.5, 149.0, 146.9, 144.6, 140.4, 139.3, 136.0, 133.0, 130.2, 130.0, 128.7,128.6, 128.5, 127.7, 126.6, 126.9, 123.8, 120.1, 50.4, 39.5, 30.1, 28.8, 26.0 (2-C) ppm; MS (ESI) *m*/*z* 426 (M+H)⁺.

### N-(7-(4-(pyridin-3-yl)-1H-1,2,3-triazol-1-yl)heptyl) benzamide

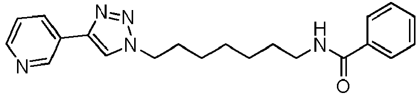

White solid; yield 58%; m.p. 123-124 °C; IR (KBr) 3330, 3119, 2929, 2851, 1631, 1532, 1288, 806, 709 cm⁻¹; ¹H-NMR (300 MHz, CDCl₃) *δ* 8.99 (br s, 1-H), 8.56 (br s, 1-H), 8.19 (d, *J* = 8.0 Hz, 1-H), 7.85 (s, 1-H), 7.76-7.73 (m, 2-H), 7.47-7.37 (m, 4-H), 6.24 (br s, 1-H), 4.41 (t, *J* = 7.0 Hz, 2-H), 3.43 (quart, *J* = 7.0 Hz, 2-H), 1.96 (quint, *J* = 7.0 Hz, 2-H), 1.60 (quint, *J* = 6.7 Hz, 2-H), 1.40-1.24 (m, 6-H) ppm; ¹³C-NMR (75 MHz, CDCl₃) *δ* 167.7, 149.2, 147.1, 144.8, 134.9, 133.1, 131.4, 128.6, 127.3, 126.9, 125.4, 120.0, 50.6, 39.9, 30.3, 29.6, 28.6, 26.7, 26.4 ppm: MS (ESI) *m*/*z* 364 (M+H)⁺.

### methyl 2'-((7-(4-(pyridin-3-yl)-1H-1,2,3-triazol-1-yl) heptyl) carbamoyl)-[1,1'-biphenyl]-2-carboxylate

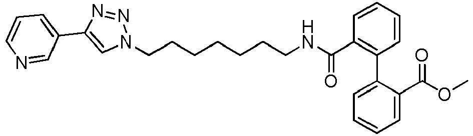

Colorless oil; yield 91%; IR (KBr) 3328, 3059, 2930, 1715, 1651, 1531, 1436, 1292, 755, 709 cm⁻¹; ¹H-NMR (300 MHz, CDCl₃) *δ* 8.97 (d, *J* = 2.2 Hz, 1-H), 8.52 (dd, *J* = 4.9/1.5 Hz, 1-H), 8.16 (dt, *J* = 8.0/1.8 Hz, 1-H), 7.87 (s, 1-H), 7.76 (dd, *J* = 7.6/1.5 Hz, 1-H), 7.63-7.60 (m, 1-H), 7.42-7.30 (m, 5-H), 7.21-7.18 (m, 1H), 7.00-6.98 (m, 1H), 6.39 (br t, 1-H), 4.36 (t, *J* = 7.0 Hz, 2-H), 3.68 (s, 3-H), 3.19-3.10 (m, 1-H), 2.96-2.85 (m, 1-H), 1.87 (quint, *J* =7.4 Hz, 2-H), 1.20-1.14 (m, 4-H), 0.99-0.87 (m, 4-H) ppm; ¹³C-NMR (75 MHz, CDCl₃) *δ* 169.3, 169.2, 149.2, 147.1, 144.7, 141.7, 138.7, 136.4, 133.0, 131.6, 131.0, 130.6, 129.3, 129.2, 129.0, 128.0, 127.8, 127.7, 126.9, 123.8, 120.0, 52.5, 50.5, 39.2, 30.2, 29.0, 28.5, 26.2 (2-C) ppm; MS (ESI) *m*/*z* 499 (M+H)⁺.

### 2'-((7-(4-(pyridin-3-yl)-1H-1,2,3-triazol-1-yl)heptyl)carbamoyl)-[1,1'-biphenyl]-2-carboxylic acid

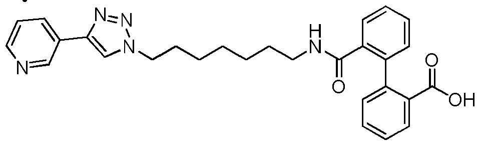

White solid; yield 84%; m.p. 81-82 °C; IR (KBr) 3281, 3060, 2929, 2854, 1715, 1651, 1548, 1266, 760 cm⁻¹; ¹H-NMR (300 MHz, CDCl₃) *δ* 11.93 (br s. 1-H), 8.92 (br s, 1-H), 8.42 (br s, 1-H), 8.20 (d, *J* = 8.0 Hz, 1-H), 7.94 (s, 1-H), 7.77-7.75 (m, 1-H), 7.53-7.50 (m, 1-H), 7.34-7.24 (m, 6-H), 7.04-7.00 (m, 2-H), 4.31 (t, *J* = 7.0 Hz, 2-H), 3.15-3.08 (m, 1-H), 2.88-2.83 (m, 1-H), 1.81 (br quint, 2-H), 1.19-1.12 (m, 4-H), 0.97-0.87 (m, 4-H) ppm; ¹³C-NMR (75 MHz, CDCl₃) *δ* 174.5, 170.3, 147.1, 145.2, 143.8, 140.6, 139.2, 135.6, 134.4, 132.5, 130.7, 130.3, 129.4, 129.3, 128.9, 127.6, 127.5 (2-C), 124.4, 120.7, 50.4, 39.3, 29.9, 28.7, 28.2, 26.1, 26.0 ppm; MS (ESI) *m*/*z* 484 (M+H)⁺.

### N²-(7-(4-(pyridin-3-yl)-1H-1,2,3-triazol-1-yl)heptyl)-[1,1'-biphenyl]-2,2'-dicarboxamide

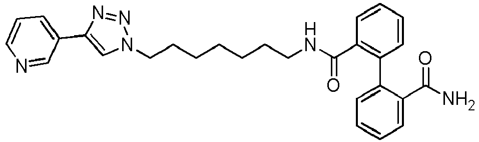

White solid; yield 44%; m.p. 100-101 °C; IR (KBr) 3505, 3175, 2929, 2854, 1652, 1558, 1436, 759, 708 cm⁻¹; ¹H-NMR (300 MHz, CDCl₃) *δ* 8.96 (br s, 1-H), 8.52 (d, *J* = 4.0 Hz, 1-H), 8.16 (dt, *J* = 8.0/2.2 Hz, 1-H), 7.85 (s, 1-H), 7.53-7.49 (m, 2-H), 7.35-7.30 (m, 6-H), 7.14-7.02 (m, 3-H), 5.74 (br s, 1-H), 4.37 (t, *J* = 7.0 Hz, 2-H), 3.16-3.12 (m, 1-H), 3.02-2.98 (m, 1-H), 1.89 (quint, *J* = 7.0 Hz, 2-H), 1.12-1.01 (m, 8-H) ppm; ¹³C-NMR (75 MHz, CDCl₃) *δ* 172.4, 170.1, 149.3, 147.2, 144.8, 139.8, 138.9, 136.7, 135.3, 133.2, 130.1 (2-C), 129.6, 129.4, 128.0, 127.9, 127.4, 127.1 (2-C), 124.0, 120.2, 50.6, 39.5, 30.3, 29.1, 28.6, 26.5, 26.4 ppm; MS (ESI) *m*/*z* 483 (M+H)⁺.

### Example 14. General Procedure for the Preparation of Thioamides (14)

To a solution of the corresponding amide **(13)** (0.094 g, 0.21 mmol, 1 eq) in dry toluene (3.0 mL), Lawesson's reagent (0.086 g, 0.21 mmol, 1 eq) was added. The reaction was stirred overnight at 80 °C, under nitrogen. After completion of the reaction, water was added and the desired product was extracted with EtOAc (x3). The combined organic layers were washed with brine (x1), dried over sodium sulfate and *concentrated in vacuo.* The residue was purified by column cromatography, using PE/EtOAc 2:8 and EtOAc as eluants.

### N-(7-(4-(pyridin-3-yl)-1H-1,2,3-triazol-1-yl)heptyl)-[1,1'-biphenyl]-2-carbothioamide

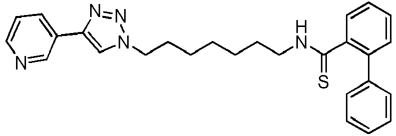

Yellowish oil; yield 72 %; IR (KBr) 3209, 3040, 2929, 1558, 1435, 1178, 943, 755, 705 cm⁻¹; ¹H-NMR (300 MHz, CDCl₃) *δ* 8.86 (d, *J* = 1.5 Hz, 1-H), 8.40-8.38 (m, 1-H), 8.11 (dt, *J* = 6.1/1.8 Hz, 1-H), 7.84 (s, 1-H), 7.69 (dd, *J* = 7.4/1.5 Hz, 1-H), 7.44-7.21 (m, 10-H), 4.34 (t, *J* = 7.0 Hz, 2-H), 3.40 (q, *J* = 6.8 Hz, 2-H), 1.86 (quint, *J* = 7.0 Hz, 2-H), 1.20-1.12 (m, 6-H), 0.90 (br quint, 2-H) ppm; ¹³C-NMR (75 MHz, CDCl₃) *δ* 200.1, 148.9, 146.8, 144.5, 142.4, 139.9, 137.1, 133.0, 130.2, 129.9, 129.7, 128.6, 128.5, 127.5, 127.4, 126.8, 123.8, 120.0, 50.4, 46.3, 30.1, 28.5, 27.0, 26.4, 26.1 ppm; MS (ESI) *m*/*z* 456 (M+H)⁺.

### Example 15. General Procedure for the Preparation of 2-Iodobenzamides (15)

Under nitrogen atmosphere, the corresponding amine **(6)** (1 eq) was dissolved in dry dichloromethane (0.4 M). While stirring at room temperature the 2-iodobenzoic acid (1.1 eq) was added, followed by the addition of EDCl *N-*(3-dimethylaminopropyl)-*N*'-ethylcarbodimide hydrochloride (1 eq) and DMAP (dimethylaminopyridine) (1 eq). The reaction mixture was stirred overnight at 40 °C. After completion of the reaction, water was added and the aqueous layer was extracted with dichloromethane (x3). The combined organic layers were washed with 2 M NaOH (x1), brine (x1), dried over sodium sulfate and concentrated *in vacuo.* The crude material was purified by column chromatography using CH₂Cl₂/MeOH 98:2 as eluant to give the desired amide. The compound was crystallized with EtOAc.

### 2-iodo-N-(7-(4-(pyridin-3-yl)-1H-1,2,3-triazol-1-yl)heptyl)benzamide

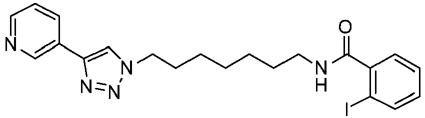

White solid; yield 64%; m.p. 106-108 °C; IR (KBr) 3331, 3120, 2929, 2852, 1631, 1533, 1289, 806, 709 cm⁻¹; ¹H-NMR (300 MHz, CDCl₃) *δ* 8.99 (s, 1-H), 8.56 (d, *J* = 4.9 Hz, 1-H), 8.20 (d, *J* = 8.0 Hz, 1-H), 7.86-7.83 (m, 2-H), 7.37-7.33 (m, 3-H), 7.11-7.05 (m, 1-H), 5.79 (br s, 1-H), 4.43 (t, *J* = 7.0 Hz, 2-H), 3.45 (quart, *J* = 6.7 Hz, 2-H), 1.98 (quint, *J* = 7.0 Hz, 2-H), 1.66-1.61 (m, 4-H), 1.42 (br s, 4-H) ppm; ¹³C-NMR (75 MHz, CDCl₃) *δ* 169.5, 148.9, 146.8, 144.4, 142.5, 139.5, 132.9, 130.7, 128.0 (2-C), 126.8, 123.7, 120.2, 92.5, 50.4, 39.7, 30.1, 29.1, 28.4, 26.6, 26.2 ppm

### Example 16. General Procedure for the Preparation of 2-Arylbenzamides (16)

Under a nitrogen atmosphere, the appropriate commercially boronic acid (1.3 eq), K₂CO₃ (3 eq) and Pd(PPh₃)₄ (0.1 eq) were added to a stirred solution of the corresponding 2-iodobenzamide derivative **(15)** (1 eq) in CH₃CN/H₂O 1:1 (0,15 M) at 25 °C. The mixture was heated to 90 °C for 24-48 h. After completion of the reaction the mixture was filtered.
The solvent of the filtrate was removed by rotatory evaporation and the resulting aqueous layer was extracted with EtOAc (x3).
(When 3-carboxyphenylboronic acid or 4-carboxyphenylboronic acid were used for the reaction, the resulting aqueous layer was acidified dropwise with HCl 3 M to pH 4 before extraction).
The combined organic layers were washed with brine (x1), dried over sodium sulfate and *concentrated in vacuo.* Finally the crude material was purified by column chromatography to afford the desired amide.

### 2'-((7-(4-(pyridin-3-yl)-1H-1,2,3-triazol-1-yl)heptyl)carbamoyl)-[1,1'-biphenyl]-3-carboxylic acid

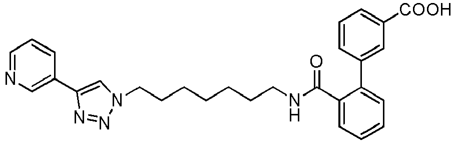

The compound was purified by column chromatography, using EtOAc/MeOH 95:5 and EtOAc/MeOH 8:2 as eluants, to give a off-white solid; yield 53%; m.p. 172-175 °C; IR (KBr) 3067, 2928, 2854, 1705, 1638, 1556, 1265, 1241, 757 cm⁻¹; ¹H-NMR (300 MHz, CD₃OD) *δ* 9.03 (br s, 1-H), 8.50 (br s, 2-H), 8.28 (d, *J* = 7.6 Hz, 1-H), 8.08 (s, 1-H), 7.97 (d, *J* = 7.6 Hz, 1-H), 7.57-7.40 (m, 7-H), 4.46 (t, *J* = 7.3 Hz, 2-H), 3.11 (t, *J* = 6.7 Hz, 2-H), 1.94-1.85 (m, 2-H), 1.28-1.21 (m, 6-H), 1.04-0.97 (m, 2-H) ppm; ¹³C-NMR (75 MHz, CD₃OD) *δ* 172.7, 170.7, 149.5, 147.2, 145.3, 142.0, 140.4, 138.0, 134.9, 133.7, 132.3, 131.2, 131.0 (2-C), 129.8, 129.4, 128.8, 128.7, 126.2, 123.2, 121.2, 51.6, 40.7, 31.2, 29.8, 29.7, 27.6, 27.3 ppm; MS (ESI) *m*/*z* 484 (M+H)⁺.

### 2'-((7-(4-(pyridin-3-yl)-1H-1,2,3-triazol-1-yl)heptyl)carbamoyl)-[1,1'-biphenyl]-4-carboxylic acid

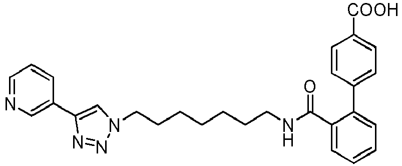

The compound was purified by column chromatography, using EtOAc/MeOH 9:1 and EtOAc/MeOH 8:2 as eluants, to give a white solid; yield 42%; m.p. 134-135 °C; IR (KBr) 3055, 2935, 2858, 1704, 1541, 1438, 1287, 756 cm⁻¹; ¹H-NMR (300 MHz, (CD₃)₂SO) *δ* 9.04 (s, 1-H), 8.78 (s, 1-H), 8.52 (d, *J* = 5.5 Hz, 1-H), 8.20 (d, *J* = 7.6 Hz, 1-H), 8.08 (br t, 1-H), 7.92 (d, *J* = 8.0 Hz, 2-H), 7.49-7.42 (m, 6-H), 4.40 (t, *J* = 6.4 Hz, 2-H), 3.01 (quart, *J* = 5.8 Hz, 2-H), 1.82 (br s, 2-H), 1.22-1.17 (m, 8-H) ppm; ¹³C-NMR (75 MHz, (CD₃)₂SO) *δ* 169.1, 168.1, 148.9, 146.4, 144.0, 143.6, 138.4, 137.6, 134.0, 132.6, 131.6, 129.8, 129.4, 129.2, 128.4, 127.8, 127.0, 124.2, 122.2, 49.8, 29.7, 28.6, 28.3, 26.3, 25.9 ppm; MS (ESI) *m*/*z* 484 (M+H)⁺.

### 2-(pyridin-3-yl)-N-(7-(4-(pyridin-3-yl)-1H-1,2,3-triazol-1-yl)heptyl)benzamide

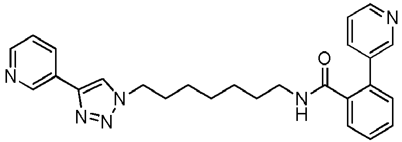

The compound was purified by column chromatography, using EtOAc/MeOH 98:2 and EtOAc/MeOH 9:1 as eluants, to give a pale yellow oil; yield 64%; IR (KBr) 3244, 3056, 2932, 2855, 1646, 1542, 1467, 811, 759, 711 cm⁻¹; ¹H-NMR (300 MHz, CD₃OD) *δ* 9.00 (d, *J* = 1.5 Hz, 1-H), 8.56-8.48 (m, 4-H), 8.28 (dt, *J* = 7.9/1.8 Hz, 1-H), 7.86 (dt, *J* = 7.9/1.8 Hz, 1-H), 7.54.7.42 (m, 6-H), 4.57 (br s, 1-H), 4.48 (t, *J* = 7.0 Hz, 2-H), 3.15 (t, *J* = 7.0 Hz, 2-H), 2.00-1.93 (m, 2-H), 1.34-1.30 (m, 6-H), 1.13-1.11 (m, 2-H) ppm; ¹³C-NMR (75 MHz, CD₃OD) *δ* 172.3, 149.7 (2-C), 149.0, 147.3, 145.4, 138.3, 138.2, 137.1, 135.0, 131.4, 131.3, 129.5, 129.1, 128.9 (2-C), 125.7, 125.1, 123.2, 51.7, 40.7, 31.2, 30.0, 29.7, 27.8, 27.4 ppm; MS (ESI) *m*/*z* 441 (M+H)⁺.

### 2'-methyl-N-(7-(4-(pyridin-3-yl)-1H-1,2,3-triazol-1-yl)heptyl)-[1,1'-biphenyl]-2-carboxamide

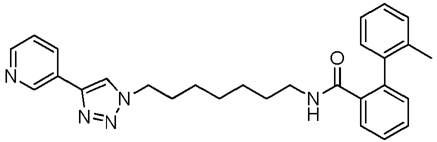

The compound was purified by column chromatography, using PE/EtOAc 3:7 and EtOAc as eluants, to give a pale yellow oil; yield 75%; IR (KBr) 3286, 3057, 2932, 2857, 1651, 1529, 1456, 1438, 1308, 757 cm⁻¹; ¹H-NMR (300 MHz, CDCl₃) *δ* 8.99 (s, 1-H), 8.57 (d, *J* = 4.8 Hz, 1-H), 8.21 (dt, *J* = 8.0/1.5 Hz, 1-H), 7.92 (dd, *J=* 7.9/1,5 Hz, 1-H), 7.84 (s, 1-H), 7.38-7.34 (m, 1-H), 7.27-7.16 (m, 7-H), 5.24 (br s, 1-H), 4.41 (t, *J* = 7,3 Hz, 2-H), 3.12-3.03 (m, 2-H), 2.08 (s, 3-H), 1,93 (quint, *J* = 7.0 Hz, 2-H), 1.59 (br s, 2-H), 1.30-1.25 (m, 4-H), 1.06-1.02 (m, 2-H) ppm; ¹³C-NMR (75 MHz, CDCl₃) *δ* 168.2. 155.3, 149.3, 147.2, 144.8, 140.5, 139.1, 136.4, 135.0, 133.1, 130.6, 130.5 (2-C), 129.5, 129.3, 128.3, 127.9, 126.3, 123.9, 120.0, 50.6, 39.6, 30.3, 29.0, 28.6 26.5, 26.4, 20.2 ppm; MS (ESI) *m*/*z* 454 (M+H)⁺.

### Example 17. General Procedure for the Preparation of 2-Bromobenzenesulfonamides(17)

Under a nitrogen atmosphere the corresponding amine (6) (1 eq) was dissolved in dry dichloromethane (0.2 M). To the cooled (0 °C) solution, TEA (2 eq) and the 2-bromobenzenesulfonyl chloride (1.1 eq) were added. The resulting mixture was stirred overnight at room temperature.
After completion of the reaction, water was added and the product was extracted with dichloromethane (x2). The combined organic layers were washed with brine, dried over sodium sulfate and *concentrated in vacuo.* The crude was purified by flash column chromatography using PE/EtOAc 3:7 and EtOAc as eluants, to give the desired 2-bromobenzenesulfonamide.

### 2-bromo-N-(8-(4-(pyridin-3-yl)-1H-1,2,3-triazol-1-yl)octyl)benzene sulfonamide

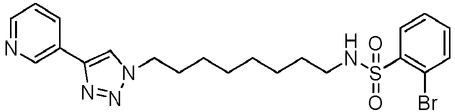

Orange oil; yield 59%; IR (KBr) 3131, 2930, 2857, 1574, 1446, 1330, 1161, 763, 708, 587 cm⁻¹; ¹H-NMR (300 MHz, CDCl₃) *δ* 8.99 (d, *J* = 2.1 Hz, 1-H), 8.56 (d, *J* = 4.6 Hz, 1-H), 8.21 (d, *J* = 8.0 Hz, 1-H), 8.14 (d, *J* = 7.6 Hz, 1-H), 7.83 (s, 1-H), 7.72 (d, *J* = 7.6 Hz, 1-H), 7.46-7.34 (m, 3-H), 5.09 (t, *J* = 5.5 Hz, 1-H), 4.40 (t, *J* = 7.0 Hz, 2-H), 2.88 (quart, *J* = 6.7 Hz, 2-H), 1.94 (quint, *J* = 7.0 Hz, 2-H), 1.47 (quint, *J* = 7.0 Hz, 2-H), 1.29-1.22 (m, 8-H) ppm; ¹³C-NMR (75 MHz, CDCl₃) *δ* 149.1, 147.0, 144.7, 138.9, 135.1, 133.7, 133.0, 131.6, 127.9, 126.9, 123.8, 120.1, 119.7, 50.5, 43.2, 30.2, 29.4, 28.7 (2-C), 26.3 (2-C) ppm.

### 2-bromo-N-(7-(4-(pyridin-3-yl)-1H-1,2,3-triazol-1-yl)heptyl)benzene sulfonamide

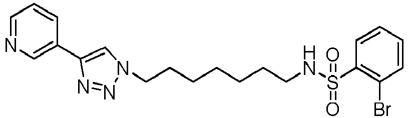

Orange oil; yield 82%; IR (KBr) 3128, 2934, 2858, 1574, 1446, 1329, 1161, 762, 708, 586 cm⁻¹; ¹H-NMR (300 MHz, CDCl₃) *δ* 8.99 (d, *J* = 1.2 Hz, 1-H), 8.57 (d, *J* = 4.9 Hz, 1-H), 8.21 (d, *J* = 8.0 Hz, 1-H), 8.13 (dd, *J* = 7.6 Hz, 1-H), 7.83 (s, 1-H), 7.72 (d, *J* = 7.6 Hz, 1-H), 7.46-7.34 (m, 3-H), 5.10 (br t, 1-H), 4.40 (t, *J* = 7.0 Hz, 2-H), 2.88 (quart, *J* = 6.7 Hz, 2-H), 1.93 (quint, *J* = 6.7 Hz, 2-H), 1.46 (quint, *J* = 7.0 Hz, 2-H), 1.30 (br s, 6-H) ppm; ¹³C-NMR (75 MHz, CDCl₃) δ 149.2, 147.1, 144.8, 138.9, 135.1, 133.8, 133.1, 131.7, 128.0, 127.0, 123.9, 120.1, 119.7, 50.5, 43.2, 30.2, 29.4, 28.4, 26.3, 26.2 ppm.

### 2-bromo-N-(6-(4-(pyridin-3-yl)-1H-1,2,3-triazol-1-yl)hexyl)benzene sulfonamide

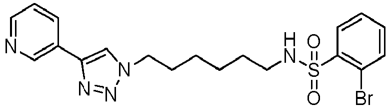

Pale yellow oil; yield 73%; IR (KBr) 3132, 2935, 2860, 1574, 1447, 1330, 1162, 1026, 764, 708, 589 cm⁻¹; ¹H-NMR (300 MHz, CDCl₃) *δ* 8.99 (d, *J* = 2.1 Hz, 1-H), 8.55 (dd, *J* = 4.9/1.5 Hz, 1-H), 8.18 (dt, *J* = 7.9/1.5 Hz, 1-H), 8.08 (dd, *J* = 7.35/1.8 Hz, 1-H), 7.85 (s, 1-H), 7.70 (dd, *J* = 7.3/1.5 Hz, 1-H), 7.63-7.35 (m, 3-H), 5.38 (t, *J* = 6.1 Hz, 1-H), 4.37 (t, *J* = 7.0 Hz, 2-H), 2.87 (quart, *J* = 6.7 Hz, 2-H), 1.89 (quint, *J* = 7.3 Hz, 2-H), 1.45-1.28 (m, 6-H) ppm; ¹³C-NMR (75 MHz, CDCl₃) *δ* 149.1, 147.0, 144.6, 138.8, 135.1, 133.8, 133.0, 131.6, 127.9, 126.9, 123.8, 120.2, 119.7, 50.3, 43.2, 43.0, 30.1, 29.2, 25.8 ppm.

### Example 18. General Procedure for the Preparation of Biphenylsulfonamides(18)

Under a nitrogen atmosphere, phenylboronic acid (1.5 eq), Na₂CO₃ (dissolved in minimum quantity of H₂O)(3 eq) and Pd(PPh₃)₄ (0.1 eq) were added to a stirred solution of the corresponding 2-bromobenzensulfonamide derivative **(17)** (1 eq) in dry DMF (0,15 M) at 25 °C.
The reaction was vigorously stirred at 90 °C for 48 h. After completion of the reaction, the mixture was filtered. The filtrate was diluted with water and extracted with EtOAc (x3).
The combined organic layers were washed with brine (x1), dried over sodium sulfate and *concentrated in vacuo.* Finally the crude material was purified by column chromatography using PE/EtOAc 3:7 as eluant, to afford the desired biphenylsulfonamide.

### N-(8-(4-(pyridin-3-yl)-1H-1,2,3-triazol-1-yl)octyl) biphenyl-2-sulfonamide

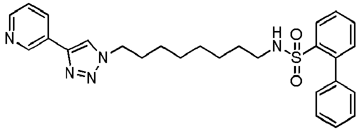

Yellow oil; yield 49%; IR (KBr) 3057, 2929, 2856, 1465, 1325, 1158, 761, 702, 588, 542 cm⁻¹; ¹H-NMR (300 MHz, CDCl₃) *δ* 8.99 (s, 1-H), 8.57 (d, *J* = 4.9 Hz, 1-H), 8.22 (dd, *J* = 8.0/1.5 Hz, 1-H), 8.13 (d, *J* = 7.6 Hz, 1-H), 7.83 (s, 1-H), 7.59-7.31 (m, 9-H), 4.40 (t, *J* = 7.0 Hz, 2-H), 3.31 (t, *J* = 5.8 Hz, 2-H), 2.57 (quart, *J* = 6.7 Hz, 2-H), 1.93 (quint, *J* = 7.6 Hz, 2-H), 1.55 (br s, 6-H), 1.28 (br s, 2-H), 1.21-1.12 (m, 2-H) ppm; ¹³C-NMR (75 MHz, CDCl₃) *δ* 149.1, 147.0, 144.6, 140.1, 138.9, 138.0, 133.0, 132.3, 132.2, 129.4, 129.2, 128.6, 128.2, 128.0, 126.9, 123.8, 120.1, 50.5, 42.9, 30.2, 29.3, 28.7 (2-C), 26.2 (2-C) ppm; MS (ESI) *m*/*z* 490 (M+H)⁺.

### N-(7-(4-(pyridin-3-yl)-1H-1,2,3-triazol-1-yl)heptyl) biphenyl-2-sulfonamide

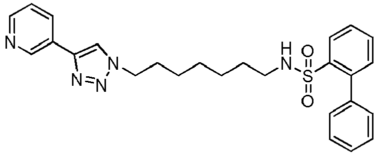

Yellow oil; yield 32%; IR (KBr) 3055, 2933, 2858, 1465, 1437, 1326, 1159, 1120, 722, 542 cm⁻¹; ¹H-NMR (300 MHz, CDCl₃) *δ* 8.99 (s, 1-H), 8.57 (d, *J* = 4.6 Hz, 1-H), 8.20 (d, *J* = 7.6 Hz, 1-H), 8.12 (d, *J* = 7.6 Hz, 1-H), 7.82 (s, 1-H), 7.59-7.31 (m, 9-H), 4.39 (t, *J* = 7.0 Hz, 2-H), 3.32 (t, *J* = 6.4 Hz, 1-H), 2.57 (quart, *J* = 6.4 Hz, 2-H), 1.91 (quint, *J* = 7.0 Hz, 2-H), 1.24-1.15 (m, 8-H) ppm; ¹³C-NMR (75 MHz, CDCl₃) *δ* 149.3, 147.1, 144.8, 140.1, 139.0, 138.0, 133.1, 132.4, 132.2, 129.5, 129.2, 128.7, 128.4, 128.1, 126.9, 123.9, 120.0, 50.5, 42.8, 30.2, 29.2, 28.3, 26.3, 26.1 ppm; MS (ESI) *m*/*z* 476 (M+H)⁺.

### N-(6-(4-(pyridin-3-yl)-1H-1,2,3-triazol-1-yl)hexyl) biphenyl-2-sulfonamide

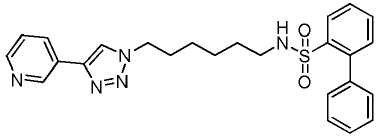

Colorless oil; yield 80%; IR (KBr) 3305, 3067, 2922, 2847, 1647, 1541, 1309, 809, 701 cm⁻¹; ¹H-NMR (300 MHz, CDCl₃) *δ* 8.98 (s, 1-H), 8.54 (d, *J* = 4.9 Hz, 1-H), 8.18 (d, *J* = 8.0 Hz, 1-H), 8.10 (d, *J* = *7*.*7 Hz*, 1-H), 7.83 (s, 1-H), 7.60-7.28 (m, 9-H), 4.35 (t, *J* = *6,4* Hz, 2-H), 3.45 (t, *J* = *6,4* Hz, 1-H), 2.55 (quart, *J* = *5.8* Hz, 2-H), 1.87 (quint, *J* = 6.7 Hz, 2-H), 1.38-1.10 (m, 6-H) ppm; ¹³C-NMR (75 MHz, CDCl₃) *δ* 149.3, 147.1, 144.8, 140.1, 138.9, 137.9, 133.1, 132.4, 132.2, 129.5, 129.2, 128.7, 128.4, 128.1, 126.9, 123.8, 120.0, 50.4, 42.7, 30.1, 29.2, 25.9 25.8 ppm; MS (ESI) *m*/*z* 462 (M+H)⁺.

### Example 19. General Procedure for the Preparation of Secondary Amines(19)

Under a nitrogen atmosphere, biphenyl-2-carboxaldehyde (1 eq) and Na₂SO₄ (10 eq) were added to a stirred solution of the corresponding intermediate **(6)** (1 eq) in dry CH₂Cl₂ (0.2 M). The mixture was stirred at room temperature for 2 h, then the solid was filtered off.
The filtrate was *concentrated in vacuo* and the residue was dissolved in MeOH (0.2 M). To the cooled (0 °C) mixture, NaBH₄ (1.1 eq) was added portionwise and the reaction was stirred at room temperature for 1 h. The solvent was evaporated *in vacuo* and the crude material was purified by column chromatography using EtOAc and EtOAc/MeOH 8:2 as eluants, to give the desired secondary amine.

### N-([1,1'-biphenyl]-2-ylmethyl)-8-(4-(pyridin-3-yl)-1H-1,2,3-triazol-1-yl)octan-1-amine

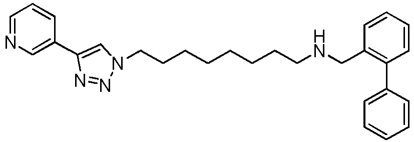

Amorphous solid; yield 44%; IR (KBr) 3310, 3058, 2926, 2854, 1476, 1461, 1218, 1049, 754, 706 cm⁻¹; ¹H-NMR (300 MHz, CDCl₃) *δ* 8.98 (d, *J* = 1.8 Hz, 1-H), 8.56 (dd, *J* = 4.9/1.5 Hz, 1-H), 8.21 (dt, *J* = 7.9/1.8 Hz, 1-H), 7.81 (s, 1-H), 7.48-7.21 (m, 10-H), 4.40 (t, *J* = 7.0 Hz, 2-H), 3.73 (s, 2-H), 2.43 (t, *J* = 7.0 Hz, 2-H), 1.94 (quint, *J* = 7.0 Hz, 2-H), 1.81 (br s, 1-H), 1.37-1.21 (m, 10-H) ppm; ¹³C-NMR (75 MHz, CDCl₃) *δ* 149.2, 147.0, 144.7, 141.8, 141.2, 137.2, 133.0, 130.1, 129.2, 129.1, 128.2, 127.6, 127.1, 127.0, 126.9, 123.8, 119.9, 51.3, 50.6, 49.1, 30.3, 29.6, 29.2, 28.9, 27.1, 26.4 ppm; MS (ESI) m/z 440 (M+H)⁺.

### N-([1,1'-biphenyl]-2-ylmethyl)-7-(4-(pyridin-3-yl)-1H-1,2,3-triazo1-1-yl)heptan-1-amine

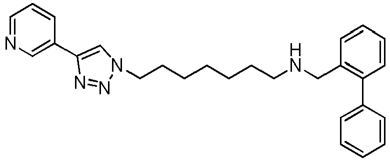

White solid; yield 68%; m.p. 86-91 °C; IR (KBr) 3295, 3123, 2926, 2855, 2812, 1476, 1434, 1123, 815, 763 cm⁻¹; ¹H-NMR (300 MHz, CDCl₃) *δ* 8.98 (d, *J* = 1.5 Hz, 1-H), 8.56 (dd, *J* = 4.9/1.5 Hz, 1-H), 8.21 (dt, *J* = 7.9/1.8 Hz,1-H), 7.81 (s, 1-H), 7.42-7.24 (m, 10-H), 4.39 (t, *J* = 7.3 Hz, 2-H), 3.72 (s, 2-H), 2.43 (t, *J* = 7.0 Hz, 2-H), 1.93 (quint, *J* = 7.3 Hz, 2-H), 1.71 (br s, 1-H), 1.36-1.23 (m, 8-H) ppm; ¹³C-NMR (75 MHz, CDCl₃) *δ* 149.2, 147.1, 144.7, 141.8, 141.3, 137.6, 133.1, 130.2, 129.2, 129.1, 128.3, 127.6, 127.1, 127.0, 126.9, 123.9, 119.9, 51.5, 50.6, 49.2, 30.3, 29.8, 28.9, 27.0, 26.5 ppm; MS (ESI) *m*/*z* 426 (M+H)⁺.

### N-([1,1'-biphenyl]-2-ylmethyl)-6-(4-(pyridin-3-yl)-1H-1,2,3-triazol-1-yl)hexan-1-amine

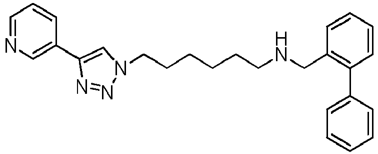

White solid; yield 61%; m.p. 55-58 °C; IR (KBr) 3325, 3058, 2925, 2857, 1474, 1452, 1223, 808, 760, 703 cm⁻¹; ¹H-NMR (300 MHz, CDCl₃) *δ* 8.98 (d, *J* = 1.5 Hz, 1-H), 8.56 (dd, *J* = 4.6/1.5 Hz, 1-H), 8.20 (dt, *J* = 7.9/1.8 Hz, 1- H), 7.80 (s, 1-H), 7.50-7.19 (m, 10-H), 4.38 (t, *J* = 7.0 Hz, 2-H), 3.72 (s, 2-H), 2.44 (t, *J* = 6.7, 2-H), 1.92 (quint, *J* = 6.7 Hz, 2- H), 1.66 (br s, 1-H), 1.38-1.25 (m, 6-H) ppm; ¹³C-NMR (75 MHz, CDCl₃) *δ* 149.2, 147.1, 144.7, 141.8, 141.3, 137.5, 133.1, 130.2, 129.2, 129.1, 128.3, 127.6, 127.2, 127.0, 126.9, 123.9, 119.9, 51.4, 50.5, 49.0, 30.3, 29.6, 26.6, 26.4 ppm; MS (ESI) *m*/*z* 412 (M+H)⁺.

### Example 20. General Procedure for the Preparation of Ureas (20)

Biphenyl-2-isocyanate (1 eq) was added dropwise to a stirred solution of the corresponding intermediate (6) (1 eq) in CH₃CN (0.15 M). The mixture was stirred at room temperature overnight. After completion of the reaction, the mixture was cooled at 0 °C for 15 minutes and the resulting precipitate was then filtered. The solid was washed with Et₂O several times and dried *in vacuo* to give the desired urea.

### 1-([1,1'-biphenyl]-2-il)-3-(8-(4-(pyridin-3-yl)-1H-1,2,3-triazol-1-yl)octyl)urea

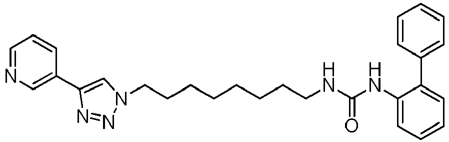

White solid; yield 85%; m.p. 90-95 °C; IR (KBr) 3361, 3290, 3056, 2934, 2856, 1686,1556, 1448, 1224, 757, 704 cm⁻¹; ¹H-NMR (300 MHz, CDCl₃) *δ* 8.95 (s, 1-H), 8.95 (s, 1-H), 8.52 (s, 1-H), 8.18 (dt, *J* = 8.0/1.8 Hz, 1-H), 7.86-7.83 (m, 2-H), 7.39-7.12 (m, 9-H), 6.25 (br s, 1-H), 4.39 (t, *J* = 7.3 Hz, 2-H), 3.09 (quart, *J* = 6.7 Hz, 2-H), 1.93 (br s, 2- H), 1.40-1.25 (m, 10-H) ppm; ¹³C-NMR (75 MHz, CDCl₃) *δ* 155.9, 148.9, 146.9, 144.6, 138.8, 135.9, 133.3, 133.1, 130.4, 129.3, 128.8, 128.4, 127.5, 127.0, 123.8, 123.7, 122.6, 120.1, 50.5, 40.3, 30.2, 30.0, 28.9, 28.7, 26.6, 26.3 ppm; MS (ESI) *m*/*z* 469 (M+H)⁺.

### 1-([1,1'-biphenyl]-2-yl)-3-(7-(4-(pyridin-3-yl)-1H-1,2,3-triazol-1-yl)heptyl)urea

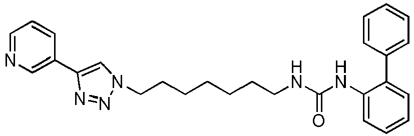

White solid; yield 78%; m.p. 148-150 °C; IR (KBr) 3376, 3295, 3122, 2934, 2854, 1678, 1537, 1449, 1217, 810, 764, 706 cm⁻¹; ¹H-NMR (300 MHz, CD₃OD) *δ* 8.99 (s, 1-H), 8.50 (d, *J* = 4.9 Hz, 1-H), 8.48 (s, 1-H), 8.25 (d, *J* = 7.6 Hz, 1-H), 7.66 (d, *J* = 8.0 Hz, 1-H), 7.51 (dd, *J* = 8.0/4.9, 1-H), 7.42-7.26 (m, 6-H), 7.20 (d, *J* = 7.0 Hz, 1-H), 7.13 (t, *J* = 7.0 Hz, 1-H), 4.47 (t, *J* = 7.0 Hz, 2-H), 3.09 (t, *J* = 6.7 Hz, 2-H), 1.96 (quint, *J* = 6.7 Hz, 2-H), 1.42-1.35 (m, 8-H) ppm; ¹³C-NMR (75 MHz, CD₃OD) *δ* 158.9, 149.5, 147.2, 145.4, 140.5, 137.0, 136.0, 134.9, 131.4, 130.3, 129.8, 129.0, 128.8, 128.5, 125.6, 125.3, 125.2, 123.0, 51.5, 40.7, 31.2, 31.0, 29.7, 27.6, 27.4 ppm; MS (ESI) *m*/*z* 455 (M+H)⁺.

### 1-([1,1'-biphenyl]-2-yl)-3-(6-(4-(pyridin-3-yl)-1H-1,2,3-triazol-1-yl)hexyl)urea

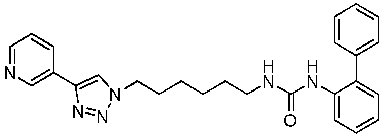

White solid; yield 54%; m.p. 156-159 °C; IR (KBr) 3370, 3280, 3116, 2934, 2853, 1694, 1541, 1435, 1222, 764, 700 cm⁻¹; ¹H-NMR (300 MHz, CD₃OD) *δ* 9.00 (d, *J* = 1.5 Hz, 1-H), 8.51 (dd, *J* = 4.49/1.5 Hz, 1-H), 8.49 (s, 1-H), 8.27 (dt, *J* = 8.2/1.5 Hz, 1-H), 7.67 (dd, *J* = 7.0/1.2 Hz, 1-H), 7.51 (ddd, *J* = 7.9/4.9/0.6 Hz, 1- H), 7.45-7.26 (m, 6-H), 7.21 (dd, *J* = 7.6/1.8 Hz, 1-H), 7.13 (td, *J* = 7.3/1.2 Hz, 1-H), 4.47 (t, *J* = 7.0 Hz, 2-H), 3.09 (t, *J* = 6.4 Hz, 2- H), 1.96 (quint, *J* = 7.3 Hz, 2- H), 1.45-1.36 (m, 6- H) ppm; ¹³C-NMR (75 MHz, CD₃OD) *δ* 158.9, 149.5, 147.2, 145.3, 140.6, 137.0, 136.1, 134.9, 131.4, 130.3, 129.8, 129.0, 128.8, 128.5, 125.6, 125.3, 125.2, 123.1, 51.5, 40.6, 31.2, 30.9, 27.1 (2-C) ppm; MS (ESI) *m*/*z* 441 (M+H)⁺.

### Synthesis of amide (27) and ether (28) - (Scheme e)

### Example 21. Preparation of methyl 2-(4-(bromomethyl)phenyl)acetate (21)

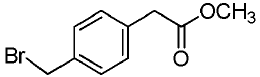

Under a nitrogen atmosphere, SOCl₂ (0.55 mL, 7.55 mmol, 0.2 eq). was added dropwise to a stirred suspension of 2-(4-(bromomethyl)phenyl)acetic acid (8.65 g, 37.74 mmol, 1 eq) in MeOH (85.0 mL) at 0 °C. The mixture was stirred at room temperature for 2 h, then was diluted with water. The solvent was partially removed *in vacuo* and the resulting aqueous phase was extracted with EtOAc (x3) The combined organic layers were washed with brine (x1), dried over sodium sulfate and *concentrated in vacuo,* to give 8,23 g of product as an amorphous off-white solid; yield 90%, which was used in the next step without further purification.
IR (KBr) 2998, 2951, 1736, 1435, 1160, 1012, 602; ¹H-NMR (300 MHz, CDCl₃) *δ* 7.35 (d, *J* = 8.0 Hz, 2-H), 7.25 (d, *J* = 8.0 Hz, 2-H), 4.48 (s, 2-H), 3.69 (s, 3-H), 3.62 (s, 2-H) ppm; ¹³C-NMR (75 MHz, CDCl₃) *δ* 171.7, 136.7, 134.3, 129.8, 129.3, 52.1, 40.9, 33.2 ppm.

### Example 22. Preparation of methyl 2-(4-(azidomethyl)phenyl)acetate (22)

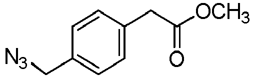

The title compound was prepared from ester **(21)** according to the general procedure of example **1**
Yellowish oil; yield 93%; IR (KBr) 3030, 2960, 2099, 1738, 1259, 1159 cm⁻¹; ¹H-NMR (300 MHz, CDCl₃) *δ* 7.29 (br, s, 4-H), 4.32 (s, 2-H), 3.69 (s, 3-H), 3.63 (s, 2-H) ppm; ¹³C-NMR (75 MHz, CDCl₃) *δ* 171.7, 134.3, 134.1, 129.7, 128.4, 54.4, 52.0, 40.8 ppm.

### Example 23. Preparation of methyl 2-(4-((4-pyridin-3-yl)-1H- 1,2,3-triazol-1-yl)methyl)phenyl)acetate (23)

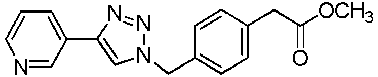

The title compound was prepared from azide **(22)** according to the general procedure of example **2.** The compound precipitated as a white solid; yield 87%; m.p. 176-178 °C dec.; IR (KBr) 3123, 2989, 1721, 1445, 1263, 1023, 810 cm ⁻¹; ¹H-NMR (300 MHz, CDCl₃) *δ* 8.95 (br s, 1-H), 8.55 (br s, 1-H), 8.15 (br d, 1-H), 7.75 (s, 1-H), 7.27 (br s, 5-H), 5.55 (s, 2-H), 3.66 (s, 3-H), 3.61 (s, 2-H) ppm; ¹³C-NMR (75 MHz, CDCl₃) *δ* 171.6, 149.3, 147.2, 145.3, 135.0, 133.5, 133.0, 130.2, 128.4, 126.7, 123.8, 120.0, 54.1,52.1, 40.8 ppm.

### Example 24. Preparation of 2-(4-((4-(pyridin-3-yl)-1H-1,2,3-triazol-1-yl)methyl)phenyl)ethanol (24)

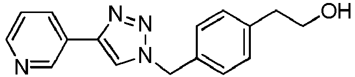

The title compound was prepared from **(23)** according to the general procedure of example **4.**
The crude material was purified by column chromatography using EtOAc and EtOAc/MeOH 98:2 as eluants to give a white solid; yield 72%; m.p. 135-137 °C; IR (KBr) 3392, 3118, 2950, 1449, 1217, 1049, 816, 767 cm⁻¹; ¹H-NMR (300 MHz, CDCl₃) *δ* 8.93 (s, 1-H), 8.54 (d, *J* = 4.3 Hz, 1-H), 8.17 (d, *J* = 7.9 Hz, 1-H), 7.74 (s, 1-H), 7.34 (dd, *J* = 7.9/4.9 Hz, 1-H), 7.27 (br s, 4-H), 5.57 (s, 2-H), 3.87(t, *J* = 6.4 Hz, 2-H), 2.88 (t, *J* = 6.4 Hz, 2-H) ppm; ¹³C-NMR (75 MHz, CDCl₃) *δ* 149.3, 147.0, 145.2, 139.9, 133.1, 132.6, 130.0, 128.5, 126.8, 123.9, 119.9, 63.4, 54.2, 38.9 ppm.

### Example 25. Preparation of 3-(1-(4-(2-azidoethyl)benzyl)-1H-1,2,3-triazol-4-yl)pyridine (25)

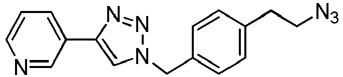

The title compound was prepared from **(24)** according to the general procedure of example **5.**
The crude material was purified by column chromatography using PE/EtOAc 2:8 as eluant to give an off-white solid; yield 93%; m.p. 106-108 °C; IR (KBr) 3120, 2942, 2127, 1449, 1217, 1049, 764 cm⁻¹; ¹H-NMR (300 MHz, CDCl₃) *δ* 8.94 (d, *J* = 2.1 Hz, 1-H), 8.55 (d, *J* = 3.7 Hz, 1-H), 8.18 (d, *J* = 7.9 Hz, 1-H), 7.72 (s, 1-H), 7.36-7.25 (m, 5-H), 5.58 (s, 2-H), 3.51 (t, *J* = 7.0 Hz, 2-H), 2.90 (t, *J* = 7.0 Hz, 2-H) ppm; ¹³C-NMR (75 MHz, CDCl₃) *δ* 149.3, 147.1, 145.2, 139.1, 133.0, 132.9, 129.8, 128.6, 126.8, 123.8, 119.9, 54.1, 52.3, 35.1 ppm.

### Example 26. Preparation of 2-(4-((4-(pyridin-3-yl)-1H-1,2,3-triazol-1-yl)methyl)phenyl) ethanamine (26)

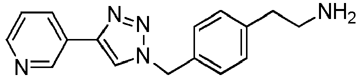

The title compound was prepared from **(25)** according to the general procedure of example **6.**
White solid; yield 91%; m.p. 152-153 °C; IR (KBr) 3304, 3119, 2864, 1552, 1449, 1328, 767 cm⁻¹; ¹H-NMR (300 MHz, CDCl₃) *δ* 8.93 (s, 1-H), 8.53 (d, *J* = 4.8 Hz, 1-H), 8.16 (dt, *J* = 7.9/1.9 Hz, 1-H), 7.73(s, 1-H), 7.35-7.20 (m, 5-H), 5.55 (s, 2-H), 2.95 (t, *J* = 6.7 Hz, 2-H), 2.74 (t, *J* = 6.7 Hz, 2-H) ppm; ¹³C-NMR (75 MHz, CDCl₃) *δ* 149.4, 147.2, 145.3, 141.1, 133.0, 132.4, 129.8, 128.4, 126.9, 123.7, 119.9, 54.3, 43.5, 39.9 ppm.

### Example 27. Preparation of N-(4-((4-(pyridin-3-yl)-1H-1,2,3-triazol-1-yl)methyl)phenethyl)-[1,1'-biphenyl]-2-carboxamide (27)

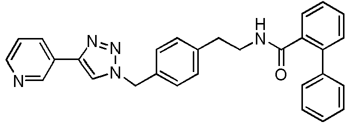

The title compound was prepared from **(26)** and biphenyl-2-carboxylic acid, according to the general procedure of example **13.**
The crude material was purified by column chromatography, using PE/EtOAc 5:5 as eluant, to give a white solid; yield 66%; m.p. 148-149 °C; IR (KBr) 3302, 3064, 2939, 1644, 1531, 1449, 1053, 744 cm⁻¹; ¹H-NMR (300 MHz, CDCl₃) *δ* 8.92 (d, *J* = 1.8 Hz, 1-H), 8.54 (dd, *J* = 4.9/1.5 Hz, 1-H), 8.16 (dt, *J* = 6.1/1.8 Hz, 1-H), 7.71 (s, 1-H), 7.62 (d, *J* = 7.6 Hz, 1-H), 7.47-7.31 (m, 9-H), 7.17 (t, *J* = 8.0 Hz, 2-H), 6.94 (t, *J* = 8.0 Hz, 2-H), 5.52 (s, 2-H), 5.25 (br s, 1-H), 3.41 (quart, *J* = 7.0 Hz, 2-H), 2.51 (t, *J* = 7.0 Hz, 2-H) ppm; ¹³C-NMR (75 MHz, CDCl₃) *δ* 169.7, 149.3, 147.1, 145.2, 140.2, 139.7, 139.3, 135.8, 133.0, 132.6, 130.3, 130.2, 129.5, 128.8, 128.7, 128.6, 128.4, 127.9, 127.7, 126.8, 123.8, 119.9, 54.1, 40.8, 34.9 ppm; MS (ESI) m/z 460 (M+H)⁺.

### Example 28. Preparation of 3-(1-(4-(2-([1,1'-biphenyl]-2-yloxy)ethyl)benzyl)-1H-1,2,3-triazol-4-yl)pyridine (28)

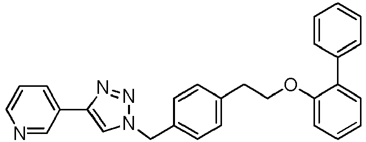

Under a nitrogen atmosphere, 2-phenylphenol (0.12 g, 0.71 mmol, 1 eq), triphenylphosphine (0.19 g, 0.71 mmol, 1 eq) and DIAD (0.14 mL, 0.71 mmol, 1 eq), were added to a stirred solution of alcohol **(24)** (0.20 g, 0.71 mmol, 1 eq) in dry THF (4.0 mL) at 0 °C. The reaction mixture was stirred at room temperature for 24 h. After completion of the reaction, the solvent was evaporated *in vacuo.* The crude material was purified by column chromatography, using PE/EtOAc 5:5 and PE/EtOAc 3:7 as eluants, to give 0.26 g of product as a white solid; yield 85%. The compound was crystallized with EtOAc; m.p. 140-141 °C; IR (KBr) 3060, 2916, 1484, 1433, 1236, 1123, 1029, 760 cm⁻¹; ¹H-NMR (300 MHz, CDCl₃) *δ* 8.93 (s, 1-H), 8.55 (d, *J* = 3.4 Hz, 1-H), 8.16 (d, *J* = 7.9 Hz, 1-H), 7.69 (s, 1-H), 7.41-7.25 (m, 8-H), 7.21-7.15 (m, 4-H), 7.00 (t, *J* = 7.3 Hz, 1-H), 6.92 (d, *J* = 8.2 Hz, 1-H), 5.55 (s, 2-H), 4.16 (t, *J* = 6.1 Hz, 2-H), 3.00 (t, *J* = 6.1 Hz, 2-H) ppm; ¹³C-NMR (75 MHz, CDCl₃) *δ* 155.7, 149.4, 147.2, 145.3, 139.8, 138.5, 133.1, 132.4, 131.0, 130.7, 130.2, 129.8, 128.7, 128.3, 127.9, 126.9, 126.8, 123.8, 121.2, 119.8, 112.3, 68.9, 54.3, 35.6 ppm; MS (ESI) m/z 433(M+H)⁺.

### Synthesis of compounds (29-34) - (Scheme f)

### Example 29. preparation of 4-((4-(pyridin-3-yl)-1H-1,2,3-triazol-1-yl)methyl)phenethyl[1,1'-biphenyl]-2-ylcarbamate (29)

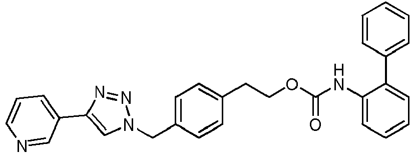

The title compound was prepared from **(24)** according to the general procedure of example **11.**
The crude material was purified by column chromatography, using PE/EtOAc 5:5 e PE/EtOAc 3:7 as eluants to give a white solid; yield 76%; m.p. 131-132 °C; IR (KBr) 3100, 3061, 1721, 1544, 1452, 1230, 1082, 749 cm⁻¹; ¹H-NMR (300 MHz, CDCl₃) *δ* 8.94 (s, 1-H), 8.55 (d, *J* = 3.4 Hz, 1-H), 8.16 (dt, *J* = 8.0/1.5 Hz, 1-H), 7.72 (s, 1-H), 7.44-7.10 (m, 14-H), 6.58 (br s, 1-H), 5.56 (s, 2-H), 4.31 (t, *J* = 7.0 Hz, 2-H), 2.94 (t, *J* = 7.0 Hz, 2-H) ppm; ¹³C-NMR (75 MHz, CDCl₃) *δ* 153.5, 149.4, 147.1, 145.3, 138.9, 138.2, 134.7, 133.1, 132.8, 131.8, 130.3, 129.9, 129.3, 129.2, 128.6, 128.4, 128.0, 126.8, 123.8, 123.7, 119.9, 65.4, 54.2, 35.2 ppm; MS (ESI) m/z 476 (M+H)⁺.

### Example 30. Preparation of 3-(1-(4-(2-(4-([1,1'-biphenyl]-2-yl)-1H-1,2,3-triazol-1-yl)ethyl)benzyl)-1H-1,2,3-triazol-4-yl)pyridine (30)

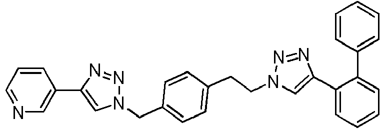

The title compound was prepared from **(25)** and 2-ethynylbiphenyle according to the general procedure of example **12.**
The crude material was purified by column chromatography, using PE/EtOAc 2:8 to give a white solid; yield 68%; m.p. 139-141 °C; IR (KBr) 3128, 3055, 2939, 1449, 1226, 1052, 812, 767, 701 cm⁻¹; ¹H-NMR (300 MHz, CD₃OD) *δ* 8.96 (d, *J* = 2.1 Hz, 1-H), 8.49 (d, *J* = 4.9/1.5 Hz, 1-H), 8.45 (s, 1-H), 8.22 (dt, *J* = 8.2/1.8 Hz, 1-H), 7.76-7.73 (m, 1-H), 7.49 (ddd, *J* = 8.0/4.9/0.9 Hz, 1-H), 7.41-7.37 (m, 2-H), 7.30-7.22 (m, 6-H), 7.07-7.02 (m, 4-H), 6.72 (s, 1-H), 5.52 (s, 2-H), 4.47 (t, *J* = 6.7 Hz, 2-H), 3.05 (t, *J* = 6.7 Hz, 2-H) ppm; ¹³C-NMR (75 MHz, CD₃OD) *δ* 149.6, 147.5, 147.4, 145.8, 142.8, 142.2, 139.3, 135.2, 134.8, 131.3, 130.4, 130.3, 130.1, 130.0, 129.5, 129.3, 128.7, 128.6, 128.3, 125.4, 124.4, 123.0, 118.9, 54.8, 52.1, 36.8 ppm; MS (ESI) m/z 484 (M+H)⁺.

### Example 31. Preparation of 1-([1,1'-biphenyl]-2-yl)-3-(4-((4-(pyridin-3-yl)-1H-1,2,3-triazol-1-yl)methyl)phenethyl)urea (31)

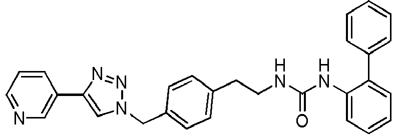

The title compound was prepared from **(26)** according to the general procedure of example **20.**
White solid; yield 84%; m.p. 208-210 °C; IR (KBr) 3329, 1625, 1570, 1435, 1279, 1049, 744 cm⁻¹; ¹H-NMR (300 MHz, CDCl₃) *δ* 8.92 (s, 1-H), 8.54 (d, *J* = 4.9 Hz, 1-H), 8.16 (d, *J* = 7.6 Hz, 1-H), 7.71 (s, 1-H), 7.70 (d, *J* = 6.1 Hz, 1-H), 7.40-7.10 (m, 13-H), 5.99 (br s, 1-H), 5.55 (s, 2-H), 4.55 (br s, 1-H), 3.42 (quart, *J* = 6.1 Hz, 2-H), 2.79 (t, *J* = 6.1 Hz, 2-H) ppm; ¹³C-NMR (75 MHz, CDCl₃) *δ* 155.5, 149.4, 147.3, 145.2, 140.3, 138.7, 135.6, 134.0, 133.1, 132.8, 130.6, 129.8, 129.4, 129.1, 128.6, 128.5, 127.9, 127.0, 124.4, 123.8, 123.1, 119.9, 54.3, 41.6, 36.1 ppm; MS (ESI) m/z 475 (M+H)⁺.

### Example 32. Preparation of N-([1,1'-biphenyl]-2-ylmethyl)-2-(4-((4-(pyridin-3-yl)-1H-1,2,3-triazol-1-yl)methy)phenyl)ethanamine (32)

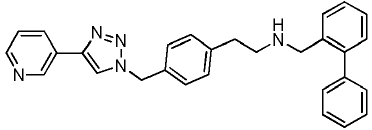

The title compound was prepared from **(26)** according to the general procedure of example **19.**
The crude material was purified by column chromatography, using PE/EtOAc 2:8 and EtOAc/MeOH 9:1 as eluants, to give a pale yellowish oil; yield 44%; IR (KBr) 3304, 3055, 2925, 1477, 1453, 1049, 753, 705 cm⁻¹; ¹H-NMR (300 MHz, CDCl₃) *δ* 8.92 (s, 1-H), 8.54 (d, *J* = 4.9 Hz, 1-H), 8.15 (dt, *J* = 8.0/1.5 Hz, 1-H), 7.70 (s, 1-H), 7.45-7.13 (m, 14-H), 5.55 (s, 2-H), 3.72 (s, 2-H), 2.71-2.68 (m, 4-H) ppm; ¹³C-NMR (75 MHz, CDCl₃) *δ* 149.3, 147.1, 145.2, 141.9, 141.3 (2-C), 137.5, 133.1, 132.2, 130.2, 129.7, 129.2, 129.1, 128.4, 128.3, 127.6, 127.2, 127.1, 126.8, 123.8, 120.0, 54.3, 51.4, 50.3, 36.1 ppm; MS (ESI) m/z 446 (M+H)⁺.

### Example 33. Preparation of 2-bromo-N-(4-((4-(pyridin-3-yl)-1H-1,2,3-triazol-1-yl)methyl)phenethyl) benzenesulfonamide (33)

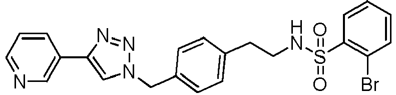

The title compound was prepared from **(26)** according to the general procedure of example **17.**
The crude material was purified by column chromatography, using PE/EtOAc 2:8 as eluant to give a yellow solid; yield 79%; m.p. 104-106 °C; IR (KBr) 3058, 2867, 1574, 1435, 1329, 1161, 760, 583 cm⁻¹; ¹H-NMR (300 MHz, CDCl₃) *δ* 8.94 (s, 1-H), 8.53 (d, *J* = 3.7 Hz, 1-H), 8.16 (d, *J* = 7.6 Hz, 1-H), 8.10 (d, *J* = 7.6 Hz, 1-H), 7.7 (s, 1-H), 7.6 (d, *J* = 7.3 Hz, 1-H), 7.46-7.31 (m, 3-H), 7.26-7.22 (m, 2-H), 7.14-7.11 (m, 2-H), 5.54 (s, 2-H), 5.22 (br s, 1-H), 3.16 (m, 2-H), 2.79 (t, *J* = 6.7 Hz, 2-H) ppm; ¹³C-NMR (75 MHz, CDCl₃) *δ* 149.2, 147.0, 145.2, 138.7, 138.6, 135.1, 133.8, 133.2, 133.1, 131.6, 129.7, 128.6, 127.9, 126.8, 123.8, 120.1, 119.7, 54.1, 44.2, 35.5 ppm.

### Example 34. Preparation N-(4-((4-(pyridin-3 yl)-1H-1,2,3-triazol-1-yl)methyl)phenethyl)-[1,1'-biphenyl]-2-sulfonamide (34)

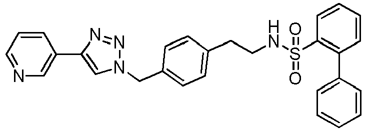

Under a nitrogen atmosphere, phenylboronic acid (0.05 g, 0.41 mmol, 1.5 eq), K₂CO₃ (0.11 g, 0.81 mmol, 3 eq) and Pd(PPh₃)₄ (0.03 g, 0.03 mmol, 0.1 eq) were added to a stirred solution of 2-bromobenzensulfonamide **(33)** (0.13 g, 0.27 mmol, 1 eq) in CH₃CN/H₂O 1:1 (2.0 mL) at 25 °C.
The mixture was heated to 90 °C for 48 h. After completion of the reaction, the solvent was evaporated by rotatory evaporation and the resulting aqueous layer was extracted with EtOAc (x3).
The combined organic layers were washed with NaOH 2 M (x2), brine (x1), dried over sodium sulfate and *concentrated in vacuo.* The crude material was purified by column chromatography, using PE/EtOAc 5:5 and PE/EtOAc 3:7 as eluants, to give 0.08 g of product as a colorless oil; yield 60%; IR (KBr) 3334, 2945, 1516, 1406, 1325, 1158, 1024, 704 cm⁻¹; ¹H-NMR (300 MHz, CDCl₃) *δ* 8.94 (s, 1-H), 8.55 (d, *J* = 3. 1 Hz, 1-H), 8.17 (dd, *J* = 8.0/2.1 Hz, 1-H), 8.12 (d, *J* = 8.0 Hz, 1-H ), 7.73 (s, 1-H), 7.58 (t, *J* = 7.6 Hz, 1-H), 7.49 (t, *J* = 7.6 Hz, 1-H), 7.37-7.25 (m, 7-H), 7.21 (d, *J* = 7.3 Hz, 2-H), 7.03 (d, *J* = 7.0 Hz, 2-H), 5.55 (s, 2-H), 3.43 (t, *J* = 6.1 Hz, 1-H), 2.85 (quart, *J* = 7.0 Hz, 2-H), 2.57 (t, *J* = 7.0 Hz, 2-H) ppm; ¹³C-NMR (75 MHz, CDCl₃) *δ* 149.3, 147.0, 145.2, 140.2, 138.9, 138.8, 137.9, 133.0, 132.9, 132.4, 132.2, 129.6, 129.4, 129.0, 128.5, 128.2, 128.0 (2-C), 126.7, 123.8, 120.0, 54.0, 43.9, 35.5 ppm; MS (ESI) m/z 496 (M+H)⁺.

### Synthesis of amide (38) - (Scheme g)

### Example 35. Preparation of 2-(4-(aminomethyl)phenyl)ethanol (35)

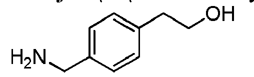

The title compound was prepared from **(22)** according to the general procedure of example **4.**
The crude material was purified by column chromatography, using MeOH e MeOH/NH₄OH conc. 98:2 as eluants, to give a pale yellowish oil; yield 38%; IR (KBr) 3283, 3024, 2937, 2867, 1570, 1513, 1048, 814 cm⁻¹; ¹H-NNM (300 MHz, CD₃OD) *δ* 7.24 (d, *J* = 8.0 Hz, 2-H), 7.18 (d, *J* = 8.0 Hz, 2-H), 3.73 (s, 2-H), 3.72 (t, *J* = 7.0 Hz, 2-H), 2.79 (t, *J* = 7.0 Hz, 2-H) ppm; ¹³C-NMR (75 MHz, CD₃OD) *δ* 141.1, 139.0, 130.2, 128.5, 64.2, 46.3, 39.9 ppm.

### Example 36. Preparation of N-(4-(2-hydroxyethyl) benzyl)-[1-1'-biphenyl]-2-carboxamide (36)

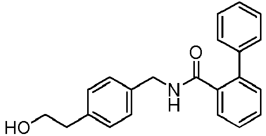

To a solution of **(35)** (0.27 g, 1.80 mmol, 1 eq) in dry CH₂Cl₂ (6.0 mL), biphenyl-2-carboxylic acid (0.39 g, 1.98 mmol, 1.1 eq), TEA (1.0 mL, 7.19 mmol, 4 eq), and propane phosphonic acid anhydride (PPAA) solution 50% in EtOAc (1.28 mL, 2.16 mmol, 1.2 eq) were subsequently added at 0 °C.
The reaction was stirred at 50°C for 24 h under a nitrogen atmosphere. The reaction was worked up by dilution with water and extracted with CH₂Cl₂ (x3). The combined organic layers were washed with NaOH 2 M (x2) and dried over sodium sulfate and concentrated in vacuo.
The crude material was purified by column chromatography, using PE/EtOAc 3:7 as eluant, to give 0.29 g of product as a pale yellowish oil; yield 49%; IR (KBr) 3302, 3054, 2928, 1644, 1529, 1308, 1044, 743 cm⁻¹; ¹H-NMR (300 MHz, CDCl₃) *δ* 7.69 (dd, *J* = 7.3/1.5 Hz, 1-H), 7.46-7.33 (m, 8-H), 7.04 (d, *J* = 8.0 Hz, 2-H), 6.80 (d, *J* = 8.0 Hz, 2-H), 5.51 (br s, 1-H), 4.28 (d, *J* = 5.5 Hz, 2-H), 3.79 (t, *J* = 6.7 Hz, 2-H), 2.79 (t, *J* = 6.7 Hz, 2-H) ppm; ¹³C-NMR (75 MHz, CDCl₃) *δ* 169.6, 140.5, 139.6, 137.9, 137.5 (2-C), 130.4, 130.3, 129.3, 128.9, 128.8 (2-C), 128.1, 127.8, 127.7, 63.7, 44.0, 38.9 ppm.

### Example 37. Preparation of N-(4-(2-azidoethyl)benzyl)-[1,1'-biphenyl]-2-carboxamide (37)

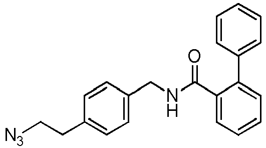

The title compound was prepared from **(36)** according to the general procedure of example **5.**
The crude material was purified by column chromatography, using PE/EtOAc 9:1 and PE/EtOAc 8:2 as eluants, to give a white solid; yield 66%; m.p. 88-89 °C; IR (KBr) 3267, 3057, 2928, 2097, 1636, 1541, 1292, 1251, 746 cm⁻¹; ¹H-NMR (300 MHz, CDCl₃) *δ* 7.72 (d, *J* = 7.3 Hz, 1-H), 7.47-7.34 (m, 8-H), 7.04 (d, *J* = 8.0 Hz, 2-H), 6.82 (d, *J* = 7.0 Hz, 2-H), 5.30 (br s, 1-H), 4.30 (d, *J* = 5.5 Hz, 2-H), 3.46 (t, *J* = 7.0 Hz, 2-H), 2.83 (t, *J* = 7.0 Hz, 2-H) ppm; ¹³C-NMR (75 MHz, CDCl₃) *δ* 169.5, 140.3, 139.6, 137.3, 136.1, 135.7, 130.3, 130.2, 129.0, 128.8 (2-C), 128.2 (2-C), 127.8, 127.7, 52.5, 43.9, 35.0 ppm.

### Example 38. Preparation of N-(4(2-(4-(pyridin-3yl)-1H-1,2,3-triazol-1-yl)ethyl)benzyl)-[1,1'-biphenyl]-2-carboxamide (38)

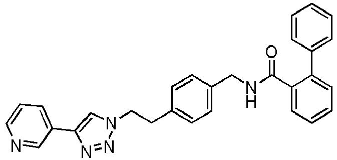

The title compound was prepared from **(37)** according to the general procedure of example **2.**
The crude material was purified by column chromatography, using PE/EtOAc 2:8 as eluant, to give a white solid; yield 48%; m.p. 204-205 °C; IR (KBr) 3272, 3060, 2930, 1636, 1547, 1451, 802, 703 cm⁻¹; ¹H-NMR (300 MHz, CDCl₃) *δ* 8.91 (d, *J* = 2.2 Hz, 1-H), 8.55 (d, *J* = 4.9 Hz, 1-H), 8.16 (d, *J* = 8.0 Hz, 1-H), 7.72 (d, *J* = 7.3 Hz, 1-H), 7.55 (s, 1-H), 7.48-7.33 (m, 9-H), 6.96 (d, *J* = 7.6 Hz, 2-H), 6.80 (d, *J* = 8.0 Hz, 2-H), 5.55 (br s, 1-H), 4.62 (t, *J* = 7.3 Hz, 2-H), 4.31 (d, *J* = 5.5 Hz, 2-H), 3.22 (t, *J* = 7.3 Hz, 2-H) ppm; ¹³C-NMR (75 MHz, CDCl₃) *δ* 169.5, 149.4, 147.3, 144.9, 140.6, 139.7, 137.0, 136.1, 135.8, 133.1, 130.4, 130.3, 128.9 (2-C), 128.8, 128.3 (2-C), 127.8, 127.7, 126.9, 123.8, 120.3, 51.8, 43.9, 36.4 ppm; MS (ESI) m/z 460 (M+H)⁺.

### Synthesis of ether (43) - (Scheme h)

### Example 39. Preparation of methyl 2-(4-(hydroxymethyl)phenyl)acetate (39)

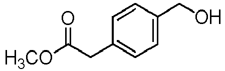

The title compound was prepared from commercially available 4-(hydroxymethyl)phenylacetic acid according to the procedure of example **21.**
Colorless oil; yield 82%; IR (KBr) 3374, 3011, 2952, 1736, 1436, 1260, 1160, 1013 cm⁻¹; ¹H-NMR (300 MHz, CDCl₃) *δ* 7.31 (d, *J* = 8.3 Hz, 2-H), 7.26 (d, *J* = 8.0 Hz, 2-H), 4.65 (s, 2-H), 3.68 (s, 3-H), 3.61 (s, 2-H) ppm; ¹³C-NMR (75 MHz, CDCl₃) *δ* 172.2, 139.9, 133.4, 129.6, 127.4, 65.1, 52.2, 40.9 ppm.

### Example 40. Preparation of methyl 2-(4-(([1,1'-biphenyl]-2-yloxy)methyl)phenyl)acetate (40)

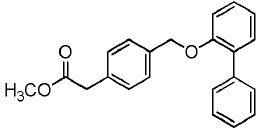

The title compound was prepared from **(39)** according to the procedure of example **28.**
The crude material was purified by column chromatography on basic aluminum oxide (Brockman grade I), using PE/EtOAc 98:2 and PE/EtOAc 95:5 as eluants, to give a white solid; yield 65%; m.p. 70-72 °C; IR (KBr) 3064, 2922, 1731, 1434, 1221, 1164, 1004, 698 cm⁻¹; ¹H-NMR (300 MHz, CDCl₃) *δ* 7.59-7.56 (m, 2-H), 7.43-7.22 (m, 9-H), 7.08-7.01 (m, 2-H), 5.06 (s, 2-H), 3.69 (s, 3-H), 3.61 (s, 2-H) ppm; ¹³C-NMR (75 MHz, CDCl₃) *δ* 171.8, 155.5, 138.5, 136.1, 133.3, 131.3, 131.0, 129.6, 129.3, 128.6, 127.9, 127.0, 126.9, 121.3, 113.3, 70.0, 51.9, 40.7 ppm.

### Example 41. Preparation of 2-(4-(([1,1'-biphenyl]-2-yloxy)methyl)phenyl)ethanol (41)

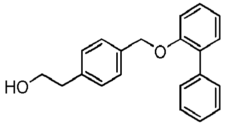

The title compound was prepared from **(40)** according to the general procedure of example **4.**
The crude material was purified by column chromatography using PE/EtOAc 8:2 as eluant, to give a white solid; yield 78%; m.p. 50-52 °C; IR (KBr) 3313, 3057, 2924, 1433, 1265, 1231, 1039, 752 cm⁻¹; ¹H-NMR (300 MHz, CDCl₃) *δ* 7.59-7.57 (m, 2-H), 7.43-7.26 (m, 7-H), 7.19-7.17 (m, 2-H), 7.08-7.01 (m, 2-H), 5.05 (s, 2-H), 3.84 (br s, 2-H), 2.85 (t, *J* = 6,4 Hz, 2-H) ppm; ¹³C-NMR (75 MHz, CDCl₃) *δ* 155.6, 138.6, 138.0, 135.4, 131.5, 131.1, 129.7, 129.2, 128.7, 128.0, 127.2, 127.0, 121.4, 113.5, 70.3, 63.6, 38.9 ppm.

### Example 42. Preparation of 2-((4-(2-azidoethyl)benzyl)oxy)-1,1'-biphenyle (42)

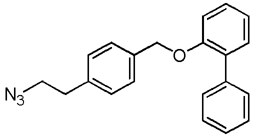

The title compound was prepared from **(41)** according to the general procedure of example **5.**
The crude material was purified by column chromatography using PE/EtOAc 98:2 as eluant, to give a colorless oil; yield 68%; IR (KBr) 3025, 2925, 2098, 1481, 1434, 1263, 1226, 753 cm⁻¹; ¹H-NMR (300 MHz, CDCl₃) *δ* 7.59 (d, *J* = 8.0 Hz, 2-H), 7.44-7.26 (m, 7-H), 7.18 (d, *J* = 8.0 Hz, 2-H), 7.09-7.01 (m, 2-H), 5.06 (s, 2-H), 3.49 (t, *J* = 7.3 Hz, 2-H), 2.88 (t, *J* = 7.3 Hz, 2-H) ppm; ¹³C-NMR (75 MHz, CDCl₃) *δ* 155.7, 138.6, 137.5, 135.9, 131.6, 131.2, 129.8, 128.9, 128.7, 128.0, 127.3, 127.0, 121.5, 113.6, 70.4, 52.5, 35.2 ppm.

### Example 43. Preparation of 3-(1-(4-(([1,1'-biphenyl]-2-yloxy)methyl)phenethyl)-1H-1,2,3-triazol-4-yl)pyridine (43)

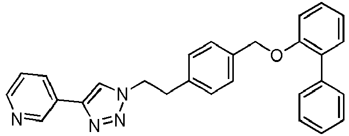

The title compound was prepared from **(42)** according to the general procedure of example **2.**
The crude material was purified by column chromatography using PE/EtOAc 5:5 and EtOAc as eluants, to give a white-grey solid; yield 57%; m.p. 136-137 °C; IR (KBr) 3061, 2858, 1481, 1435, 1264, 1230, 1051, 700 cm⁻¹; ¹H-NMR (300 MHz, CDCl₃) *δ* 8.91 (d, *J* = 1.9 Hz, 1-H), 8.55 (d, *J* = 4.9 Hz, 1-H), 8.13 (dt, *J* = 7.6/1.5 Hz, 1-H), 7.57-7.53 (m, 3-H), 7.40-7.23 (m, 8-H), 7.09-6.99 (m, 3-H), 5.04 (s, 2-H), 4.63 (t, *J* = 7.0 Hz, 2-H), 3.24 (t, *J* = 7.0 Hz, 2-H) ppm; ¹³C-NMR (75 MHz, CDCl₃) *δ* 155.6, 149.4, 147.2, 144.6, 138.6, 136.5, 136.3, 133.1, 131.6, 131.2, 129.8, 128.9, 128.7, 128.0, 127.6, 127.1, 126.9, 123.9, 121.6, 120.4, 113.6, 70.3, 51.9, 36.5 ppm; MS (ESI) m/z 433 (M+H)⁺.

### Synthesis of amide (49) and ether (50) - (Scheme i)

### Example 44. Preparation of 3-(4-aminophenyl)propan-1-ol (44)

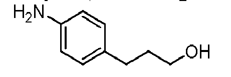

To a solution of (*E*)-3-(4-nitrophenyl)prop-2-en-1-ol (5.00 g, 0.03 mmol, 1 eq) in MeOH (50.0 mL) under a nitrogen atmosphere was added Nickel Raney (4 mL slurry in water). The reaction mixture was stirred under hydrogen atmosphere at room temperature overnight. After completion of the reaction, the suspension was filtered through a pad of Celite and the filter cake was washed with EtOAc and MeOH. The filtrate was washed with brine (x1), dried over sodium sulfate and concentrated in vacuo to give 4.09 g of product as an orange oil, which was used in the next step without further purification; yield 97%; IR (KBr) 3332, 2936, 1623, 1517, 1267, 1056, 828 cm⁻¹; ¹H-NMR (300 MHz, CDCl₃) *δ* 6.98 (d, *J* = 8.3 Hz, 2-H), 6.62 (d, *J* = 8.3 Hz, 2-H), 3.65 (t, *J* = 6.4 Hz, 2-H), 3.56 (br s, 2-H), 2.59 (t, *J* = 7.3 Hz, 2-H), 1.83 (quint, *J* = 6.4 Hz, 2-H), 1.43 (br s, 1-H) ppm; ¹³C-NMR (75 MHz, CDCl₃) *δ* 144.1, 132.0, 129.1, 115.4, 61.9, 34.4, 31.1 ppm.

### Example 45. Preparation of 3-(4-azidophenyl)propan-1-ol (45)

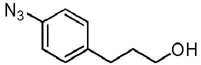

3-(4-aminophenyl)propan-1-ol **(44)** (2.68 g, 17.73 mmol, 1 eq) was dissolved in water (80.0 mL). To the stirred and cooled (0° C) solution, HCl conc (7.22 mL, 86.7 mmol, 5 eq) and a solution of NaNO₂. (1.22 g, 17.73 mmol, 1 eq) in water (25.0 mL) were added dropwise.
After stirring at 0 °C for 10 minutes, NaN₃ (1.38 g, 20.81 mmol, 1.2 eq) was added. The resulting mixture was stirred for 1.5 h. at room temperature. At the completion of the reaction, the product was extracted with EtOAc (x3). The combined organic layers were washed with brine (x1), dried over sodium sulfate and concentrated *in vacuo* to give 2.90 g of product as a yellowish oil, which was used in the next step without further purification; yield 94%; IR (KBr) 3334, 2941, 2108, 1506, 1289, 1057, 825 cm⁻¹; ¹H-NMR (300 MHz, CDCl₃) *δ* 7.18 (d, *J* = 7.3 Hz, 2-H), *6.93* (d, *J* = 7.0 Hz, 2-H), 3.64 (t, *J* = 7.0 Hz, 2-H), 2.67 (t, *J* = 7.6 Hz, 2-H), 1.85 (quint, *J* = 7.6 Hz, 2-H), 1.59 (br s, 1-H) ppm; ¹³C-NMR (75 MHz, CDCl₃) *δ* 138.8, 137.7, 129.9, 119.1, 62.1, 34.3, 31.5 ppm.

### Example 46. Preparation of 3-(4-(4-(pyridin-3-yl)-1H-1,2,3-triazol-1-yl)phenyl)propan-1-ol (46)

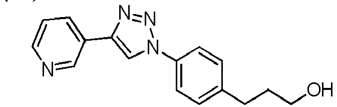

The title compound was prepared from **(45)** according to the general procedure of example **2.**
The compound precipitated as a white solid and it was crystallized with EtOAc/MeOH; yield 50%; m.p. 178-179 °C; IR (KBr) 3359, 3118, 2951, 1519, 1402, 1231, 1078, 812, 709 cm⁻¹; ¹H-NMR (300 MHz, CD₃OD) *δ* 9.02 (br s, 1-H), 8.60 (br s, 1-H), 8.37 (d, *J* = 7.6 Hz, 1-H), 7.81 (d, *J* = 8.5 Hz, 2-H), 7.57 (br s, 1-H), 7.45 (d, *J* = 8.5 Hz, 2-H), 4.58 (br s, 1-H), 3.60 (t, *J* = 6.4 Hz, 2-H), 2.79 (t, *J* = 7.6 Hz, 2-H), 1.93-1.84 (m, 2-H) ppm; ¹³C-NMR (75 MHz, CD₃OD) *δ* 149.7, 147.6, 146.2, 145.1, 136.3, 134.9, 130.9, 128.5, 127.6, 121.7, 121.1, 62.1, 35.2, 32.7 ppm.

### Example 47. Preparation of 3-(1-(4-(3-azidoprophyl)phenyl)-1H-1,2,3-triazol-4-yl)pyridine (47)

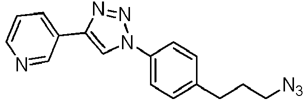

The title compound was prepared from **(46)** according to the general procedure of example **5.**

The crude material was purified by column chromatography using PE/EtOAc 5:5 as eluant, to give an off-white solid; yield 81%; m.p. 97-98 °C; IR (KBr) 3117, 2949, 2096, 1521, 1402, 1308, 1232, 1045, 808 cm⁻¹; ¹H-NMR (300 MHz, CDCl₃) *δ* 9.07 (d, *J* = 1.5 Hz, 1-H), 8.61 (dd, *J* = 4.9/1.5 Hz, 1-H), 8.28 (dt, *J* = 7.9/2.1 Hz, 1-H), 8.25 (s, 1-H), 7.74-7.70 (m, 2-H), 7.42-7.36 (m, 3-H), 3.33 (t, *J* = 6.7 Hz, 2-H), 2.80 (t, *J* = 7.3 Hz, 2-H), 1.95 (quint, *J* = 6.7 Hz, 2-H) ppm; ¹³C-NMR (75 MHz, CDCl₃) *δ* 149.3, 147.0, 145.2, 142.0, 135.0, 132.9, 129.7, 126.4, 123.7, 120.6, 118.2, 50.4, 32.2, 30.2 ppm.

### Example 48. Preparation of 3-(4-(4-(pyridin-3-yl)-1H-1,2,3-triazol-1-yl)phenyl)propan-1-amine (48)

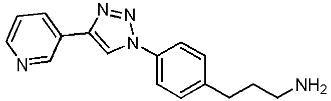

The title compound was prepared from **(47)** according to the general procedure of example **6.**
Amorphous off-white solid; yield 82%; IR (KBr) 3356, 1665, 1401, 1231, 1129, 810, 565 cm⁻¹; ¹H-NMR (300 MHz, CD₃OD) *δ* 9.10 (dd, *J* = 2.1/0.9 Hz, 1-H), 9.04 (s, 1-H), 8.55 (dd, *J* = 4.9/1.5 Hz, 1-H), 8.36 (dt, *J* = 8.3/2.1 Hz, 1-H), 7.85 (d, *J* = 8.6 Hz, 2-H), 7.56 (ddd, *J* = 7.9/4.9/0.6 Hz, 1-H), 7.48 (d, *J* = 8.6 Hz, 2-H), 2.86-2.77 (m, 4-H), 1.98-1.90 (m, 2-H) ppm; ¹³C-NMR (75 MHz, CD₃OD) *δ* 149.8, 147.4, 146.1, 144.2, 136.5, 135.0, 130.9, 128.5, 125.6, 121.6, 121.1, 41.2, 33.4, 32.9 ppm.

### Example 49. Preparation of N-(3-(4-(4-(pyridin-3yl)-1H-1,2,3-triazol-1-yl)phenyl)propyl)-[1,1'-biphenyl]-2-carboxamide (49)

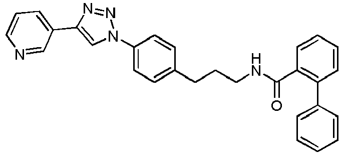

The title compound was prepared from **(48)** and biphenyl-2-carboxylic acid according to the general procedure of example **13.**

The crude material was purified by column chromatography using EtOAc as eluant, to give a white solid which was crystallized with EtOAc/MeOH; yield 50%; m.p. 202-203 °C; IR (KBr) 3237, 3057, 2949, 1634, 1550, 1522, 1235, 1046, 800 cm⁻¹; ¹H-NMR (300 MHz, CDCl₃) *δ* 9.07 (s, 1-H), 8.61-8.59 (m, 1-H), 8.29-8.22 (m, 2-H), 7.72-7.65 (m, 3-H), 7.50-7.35 (m, 9-H), 7.25-7.22 (m, 2-H), 5.25 (br s, 1-H), 3.21 (quart, *J* = 5.8 Hz, 2-H), 2.43 (t, *J* = 6.4 Hz, 2-H), 1.56 (t, *J* = 6.7 Hz, 2-H) ppm; ¹³C-NMR (75 MHz, CDCl₃) *δ* 168.0, 149.6, 147.4, 142.8, 140.7, 139.6, 136.0, 135.3, 133.2, 130.3, 130.2, 129.8, 128.9 (2-C), 128.8, 127.9, 127.8, 126.7, 125.4, 123.9, 120.9, 118.1, 39.4, 32.7, 30.5 ppm; MS (ESI) m/z 460 (M+H)⁺.

### Example 50. Preparation of 3-(1-(4-(3-([1,1'-biphenyl]-2-yloxy)propyl)phenyl)-1H-1,2,3-triazol-4-yl)pyridine (50)

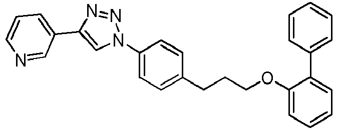

The title compound was prepared from **(46)** according to the procedure of example **28.**

The crude material was purified by column chromatography using PE/EtOAc 6:4 and PE/EtOAc 2:8 as eluants, to give a white solid which was crystallized with EtOAc; yield 43%; m.p. 164-165 °C; IR (KBr) 3034, 2929, 1521, 1260, 1235, 1044, 804, 705 cm⁻¹; ¹H-NMR (300 MHz, CDCl₃) *δ* 9.07 (s, 1-H), 8.61 (s, 1-H), 8.28 (d, J = 7.9 Hz, 1-H), 8.21 (s, 1-H), 7.65-7.64 (m, 2-H), 7.59 -7.57 (m, 2-H), 7.45-7.23 (m, 8-H), 6.99 (t, J = 7.6 Hz, 1-H), 6.92 (d, J = 8.2 Hz, 1-H), 3.93 (t, J = 5.8 Hz, 2-H), 2.71 (t, J = 7.3 Hz, 2-H), 1.99 (quint, J = 5.2 Hz, 2-H) ppm; ¹³C-NMR (75 MHz, CDCl₃) *δ* 155.8, 149.6, 147.3, 145.4, 143.0, 138.7, 135.0, 133.2, 131.2, 131.0, 129.9, 129.8, 128.7, 127.9, 127.0, 126.6, 123.9,121.2, 120.8, 118.1, 112.8, 67.1, 31.8, 30.9 ppm; MS (ESI) m/z 433 (M+H)⁺.

### Synthesis of amide (53) - (Scheme i)

### Example 51. Preparation of 4-(3-azidopropyl)aniline (51)

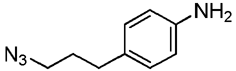

The title compound was prepared from **(44)** according to the general procedure of example **5.**

The crude material was purified by column chromatography using PE/EtOAc 95:5 and PE/EtOAc 9:1 as eluants, to give an orange oil; yield 91%; IR (KBr) 3359, 2938, 2097, 1622, 1517, 1278, 1180, 825 cm⁻¹; ¹H-NMR (300 MHz, CDCl₃) *δ* 6.97 (d, J = 8.0 Hz, 2-H), 6.36 (d, J = 8.2 Hz, 2-H), 3.58 (br s, 2-H), 3.26 (t, J = 6.7 Hz, 2-H), 2.60 (t, J = 7.3 Hz, 2-H), 1.86 (quint, J = 6.7 Hz, 2-H) ppm; ¹³C-NMR (75 MHz, CDCl₃) *δ* 144.6, 130.7, 129.2, 115.3, 50.6, 31.8, 30.6 ppm.

### Example 52. Preparation of N-(4-(3-azidopropyl)phenyl)-[1,1'-biphenyl]-2-carboxamide (52)

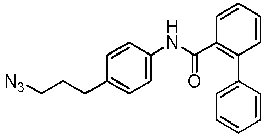

The title compound was prepared from **(51)** and biphenyl-2-carboxylic acid according to the general procedure of example **13.**

The crude material was purified by column chromatography using PE/EtOAc 9:1 as eluant, to give an off-white solid; yield 90%; m.p. 107-108 °C; IR (KBr) 3219, 3056, 2946, 2094, 1647, 1597, 1543, 1329, 746 cm⁻¹; ¹H-NMR (300 MHz, CDCl₃) *δ* 7.88 (t, J = 7.3 Hz, 1-H), 7.54-7.42 (m, 8-H), 7.03-7.00 (m, 4-H), 3.23 (t, J = 6.7 Hz, 2-H), 2.61 (t, J = 6.7 Hz, 2-H), 1.85 (quint, J = 7.0 Hz, 2-H) ppm; ¹³C-NMR (75 MHz, CDCl₃) *δ* 167.3, 139.6, 136.9, 135.7, 135.4, 130.6, 130.3, 129.4, 128.9, 128.8, 128.7, 128.0, 127.8, 120.3, 50.5, 32.1, 30.3 ppm.

### Example 53. Preparation of N-(4-(3-(4-(pyridin-3-yl)-1H-1,2,3-triazol-1-yl)propyl)phenyl)-[1,1'-biphenyl]-2-carboxamide (53)

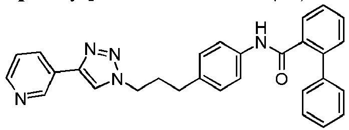

The title compound was prepared from **(52)** according to the general procedure of example **2.**
The crude material was purified by column chromatography using PE/EtOAc 8:2, PE/EtOAc 5:5 and PE/EtOAc 2:8 as eluants, to give a white solid; yield 48%; m.p.65-67 °C; IR (KBr) 3028, 2928, 1664, 1598, 1515, 1410, 1322, 745, 701 cm-1; 1H-NMR (300 MHz, CDCl₃) *δ* 8.98 (s, 1-H), 8.56 (d, *J* = 4.6 Hz, 1-H), 8.19 (d, *J* = 7.6 Hz, 1-H), 7.89 (d, *J* = 7.6 Hz, 1-H), 7.77 (s, 1-H), 7.54-7.34 (m, 9-H), 7.07-7.04 (m, 4-H), 6.84 (br s, 1-H), 4.38 (t, *J* = 7.0 Hz, 2-H), 2.62 (t, *J* = 7.0 Hz, 2-H), 2.25 (quint, *J* = 7.0 Hz, 2-H) ppm; ¹³C-NMR(75 MHz, CDCl₃) *δ* 167.4, 149.0, 146.9, 144.6, 139.9, 139.6, 136.1 (2-C), 135.4, 132.9, 130.5, 130.3, 129.3, 128.9, 128.8, 128.7, 128.0, 127.7, 126.8, 123.8, 120.4, 120.1, 49.6, 31.8, 31.6 ppm; MS (ESI) m/z 460 (M+H)⁺.

### Synthesis of ether (57) - (Schema k)

### Example 54. Preparation of 3-(4-bromophenyl)propan-1-ol (54)

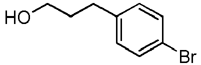

Under a nitrogen atmosphere LiAlH₄ (0.27 g, 7.01 mmol, 1.5 eq) was added portionwise to a stirred solution of 3-(4-bromophenyl)propionic acid (1.08 g, 4.70 mmol, 1 eq) in dry THF (22.0 mL) at 0 °C.
The reaction mixture was heated to 70 °C for 2 h. After completion of the reaction, the mixture was quenched by addition of HCl 1 M dropwise at 0 °C. The resulting aqueous phase was extracted with EtOAc (x3) The combined organic layers were washed with HCl 1 M (x1), sat. aq. NaHCO₃ (x2), brine (x1), dried over sodium sulfate and concentrated *in vacuo,* to give 0.97 g product as a colorless oil, which was used in the next step without further purification; yield 96%; IR (KBr) 3318, 3024, 2939, 1488, 1404, 1071, 1011, 833, 795 cm⁻¹; ¹H-NMR (300 MHz, CDCl₃) *δ* 7.39 (d, *J* = 8.2 Hz, 2-H), 7.06 (d, *J* = 8.2 Hz, 2-H), 3.65 (t, *J* = 6.4 Hz, 2-H), 2.66 (t, *J* = 7.3 Hz, 2-H), 1.90-1.83 (m, 2-H), 1.65 (br s, 1-H) ppm; ¹³C-NMR (75 MHz, CDCl₃) *δ* 140.8, 131.4, 130.2, 119.5, 61.7, 33.9, 31.4 ppm.

### Example 55. Preparation of 1-(3-azidopropyl)-4-bromobenzene (55)

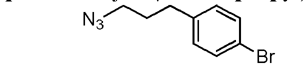

The title compound was prepared from **(54)** according to the general procedure of example **5,** but the reaction mixture was heated to 50 °C for 24 h.
The crude material was purified by column chromatography using PE/EtOAc 98:2 as eluant, to give a colorless oil; yield 79%; IR (KBr) 2941, 2097, 1488, 1254, 1072, 1011, 831, 795 cm⁻¹; ¹H-NMR (300 MHz, CDCl₃) *δ* 7.41 (dd, *J* = 6.4/1.8 Hz, 2-H), 7.06 (dd, *J* = 6.4/1.8 Hz, 2-H), 3.28 (t, *J* = 6.7 Hz, 2-H), 2.66 (t, *J* = 7.3 Hz, 2-H), 1.88(quint, *J* = 6.7 Hz, 2-H) ppm; ¹³C-NMR (75 MHz, CDCl₃) *δ* 139.9, 131.7, 130.3, 120.0, 50.6, 32.2, 30.4 ppm.

### Example 56. Preparation of 3-(1-(3-(4-bromophenyl)propyl)-1H-1,2,3-triazol-4-yl)pyridine (56)

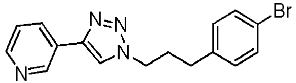

The title compound was prepared from **(55)** according to the general procedure of example **2**.
The crude material was purified by column chromatography using PE/EtOAc 5:5 and PE/EtOAc 2:8 as eluants, to give a white solid; yield 85%; m.p. 111-113 °C; IR (KBr) 3115, 2943, 2866, 1488, 1459, 1229, 1174, 1010, 808, 706 cm⁻¹; ¹H-NMR (300 MHz, CDCl₃) *δ* 8.96 (d, *J* = 2.1 Hz, 1-H), 8.54 (dd, *J* = 4.9/1.5 Hz, 1-H), 8.17 (dt, *J* = 7.9/1.5 Hz, 1-H), 7.80 (s, 1-H), 7.39 (d, *J* = 8.5 Hz, 2-H), 7.35 (dd, *J* = 7.9/4.9 Hz, 1-H), 7.04 (d, *J* = 8.2 Hz, 2-H), 4.39 (t, *J* = 7.0 Hz, 2-H), 2.63 (t, *J* = 7.3 Hz, 2-H), 2.26 (quint, *J* = 7.3 Hz, 2-H) ppm; ¹³C-NMR (75 MHz, CDCl₃) *δ* 149.2, 146.9, 144.7, 138.9, 132.9, 131.6, 130.2, 126.7, 123.8, 120.1, 120.0, 49.5, 31.9, 31.4 ppm.

### Example 57. Preparation of 3-(1-(3-(4-([1,1'-biphenyl]-2-yloxy)phenyl)propyl)-1H-1,2,3-triazol-4-yl)pyridine (57)

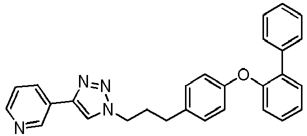

Aryl Bromide **(56)** (0.08 g, 0.24 mmol, 1 eq) was dissolved in dry toluene (2.0 mL) in a Carius tube. While stirring at room temperature 2-phenylphenol (0.06 g, 0.33 mmol, 1.4 eq), K₂CO₃ (0.06 g, 0.047 mmol, 2 eq), CuI (0.02 g, 0.09 mmol, 0.4 eq) and 1-butylimidazole (0.03 mL, 0.24 mmol, 1 eq) were added. The reaction mixture was heated at 120 °C for 48 h under nitrogen atmosphere. The mixture was filtered and the filtrate was diluted with sat. aq. Na₂CO₃ and was extracted with EtOAc (x3). The combined organic layers were washed with brine (x1), dried over sodium sulfate and concentrated *in vacuo.* The crude material was purified by column chromatography using PE/EtOAc 5:5 and PE/EtOAc 3:7 as eluants to give 0.02 g of product as a off-white amorphous solid; yield 20%; IR (KBr) 3065, 2855, 1473, 1431, 1261, 1239, 1077, 703 cm⁻¹; ¹H-NMR (300 MHz, CDCl₃) *δ* 8.97 (s, 1-H), 8.57 (dd, *J* = 4.6/1.5 Hz, 1-H), 8.21 (dt, *J* = 7.9/1.8 Hz, 1-H), 7.78 (s, 1-H), 7.53 (d, *J* = 7.0 Hz, 1-H), 7.39-7.18 (m, 11-H), 7.07 (d, *J* = 8.6 Hz, 1-H), 6.86 (d, *J* = 8.6 Hz, 1-H), 4.43 (t, *J* = 7.0 Hz, 2-H), 2.71 (t, *J* = 7.0 Hz, 2-H), 2.32 (quint, *J* = 7.3 Hz, 2-H) ppm; ¹³C-NMR(75 MHz, CDCl₃) *δ* 157.1, 153.2, 149.2, 147.0, 144.7, 139.0, 135.4, 134.1, 133.7, 132.9, 131.3, 130.5, 129.2, 128.8, 128.1, 127.2, 126.7, 123.8, 120.0, 118.3, 49.5, 31.9, 31.4 ppm; MS (ESI) m/z 433 (M+H)⁺.

### Activity evaluation of compounds of formula (I) on Nicotinamide phosphoribosyltransferase

Determination of the activity of some compounds on NAMPT was performed by a number or direct and indirect detections methods.

### Analysis of the effects on cell viability

Cell based assays were used to evaluate the possible cytotoxic effects of some compounds. To measure cytotoxic effects two different methods were used, a MTT cell proliferation assay and trypan blue count.

The Trypan blue assay is used to determine the number of viable cells present in a cell suspension. It is based on the principle that live cells possess intact cell membranes that exclude certain dyes, such as trypan blue, eosin, or propidium, whereas dead cells do not. Briefly, 8 X 10⁵ cells (SH-SY5Y, HeLa; obtained from ATCC, LGC standards, Sesto San Giovanni) were seeded in 24-well plates and treated for 48 and 72 hours. After the treatments, cells were stained with trypan blue and counted.

To analyze cell viability was used the colorimetric 3-(4,5-dimethylthiazol-2-yl)-2,5-diphenyltetrazolium bromide (MTT) method. The assay is based on the cleavage of the yellow tetrazolium salt MTT to purple formazan crystals by metabolic active cells. Since reduction of MTT can only occur in metabolically active cells, the level of activity is a measure of the viability of cells and conversely the cytotoxic effects of various treatments. Briefly, 8 X 10⁵ cells (SH-SY5Y, code number CRL-2266; and HeLa, code number CCL-2) were seeded in 24-well plates and treated for 48 and 72 hours. Compounds were dissolved in DMSO and were added to the cells to give a final DMSO concentration no greater than 0.5%. Cells were washed once in Locke buffer and 300 µl of MTT (250 µg/ml in Locke buffer) were added before returning the cells to the incubator for 1 h to allow the formation of the purple formazan crystals. After 1 h, 600 µl of isopropanol/0.1 M HCl were added to each well, and the absorbance was read at 570 nm in a plate reader (Victor3V, PerkinElmer Life Sciences). For some compounds a dose response curve and IC₅₀ for the effects on cell viability and proliferation were calculated. Cell lines used were originally purchased from ATCC (LGC standards. Sesto San Giovanni).

Synergism of some compounds was tested treating cells with increasing concentrations of the compound and of cisplatin or etoposide.

### NAD levels determination

Total cellular NAD(P) was measured using an adaptation of the method described by Gasser et al. (Gasser A, Bruhn S, Guse AH. J. Biol. Chem. 2006, 281, 16906-13) Briefly, 8 X 10⁵ cells (SH-SY5Y and HeLa) were grown and treated in 24-well plates. After the treatment, cells were scraped with a piston of a 1 mL syringe in 100 mL ddH₂0 on ice, and were extracted with 1 volume of HClO₄ (2 M) for 45 minutes on ice. The samples were then centrifuged for 1 minute at 13,000 g and the supernatant was diluted with an equal volume of K₂CO₃ (1 M). After a further 45 minutes incubation on ice, the insoluble potassium perchlorate was removed by centrifugation for 1 minute at 13000 g. The final pH of the supernatant was 8-8.5. In a black 96-well plate (OptiPlateTM-96 F: PerkinElmer) 60 mL of cycling mix was added to 100 µl of the extracted NAD(P). The cycling mix (60 µl) consisted of: 20 µL 500 mM NaH₂PO₄, pH 8.0, 2 µL 5 mg/mL BSA, 0.1 µL 10 mM resazurin (prepared fresh), 5 µL 100 mM glucose-6-phosphate (G8404, Sigma), 15 µL 1 mg/mL of glucose-6-phosphate dehydrogenase, 15 µL of purified diaphorase (see below). Fluorescence (excitation 544 nm, emission 590 nm) was measured for each well using a plate reader (Victor3V, PerkinElmer). Standard curves were generated with authentic NAD subjected to mock extractions used to generate calibration curves. The diaphorase was purified as follows: 100 µL of enzyme solution (diaphorase 12 mg/mL in 50 mM NaH₂PO₄, pH 8.0 adjusted with NaOH) was mixed with 200 µL BSA (5 mg/ml in water) and 1.2 mL of a suspension (2% w/v) of activated charcoal in 50 mM NaCl, 20 mM NaH₂PO₄, pH 8.0. After incubation at 37 °C for 30 min, the charcoal was removed by centrifugation (11,000 g, 10 min, 4-8 °C), and the supernatant used directly for the cycling assay.

### Direct Evaluation of inhibition of NAMPT activity

Determination of the direct activity of compounds on NAMPT was performed using a commercial kit, Cyclex NAMPT colorimetric Assay Kit.

### Induction of Experimental Colitis by DNBS.

Animal work was authorized by the local ethical committee for animal studies (Università del Piemonte Orientale). BALB/c mice (Harlan Nossan, Milan, Italy) were anesthetized by enflurane. Dinitrobenzene sulfonic acid (DNBS; 4 mg in 100 *µ*l of 50% ethanol) was injected into the rectum through a catheter inserted 4.5 cm proximally to the anus. Carrier alone (100 *µ*l of 50% ethanol) was administered in control experiments. Thereafter, the animals were kept for 15 minutes in a Trendelenburg position to avoid reflux. Treatment with either solvent alone or drugs was done immediately after induction and thereafter twice a day. After colitis induction, the animals were observed for 3 days. On Day 4, the animals were weighed and anaesthetized with chloral hydrate, and the abdomen was opened by a midline incision. The colon was removed, freed from surrounding tissues, opened along the antimesenteric border, rinsed, weighed, and processed for histology, biochemical measurements and immunohistochemistry. Colon damage (macroscopic damage score) was evaluated and scored by two independent observers according to the following criteria: 0, no damage; 1, localized hyperemia without ulcers; 2, linear ulcers with no significant inflammation; 3, linear ulcers with inflammation at one site; 4, two or more major sites of inflammation and ulceration extending > 1 cm along the length of the colon; and 5-8, one point is added for each centimeter of ulceration beyond an initial 2 cm. Biochemical and histological analysis also included TNFalpha measurements and myeloperoxidase (MPO) measurements by ELISA assay, and VEGF, NAMPT, COX-2 mesurements by immunoblotting. (Esposito E, Mazzon E, Paterniti I, Dal Toso R, Pressi G, Caminiti R, Cuzzocrea S. PPAR-alpha Contributes to the Anti-Inflammatory Activity of Verbascoside in a Model of Inflammatory Bowel Disease in Mice. PPAR Res. 2010; 2010:917312).

### Induction of Experimental Colitis by dextran sulfate sodium (DSS)

Animal work was authorized by the local ethical committee for animal studies (Università del Piemonte Orientale). Colitis was induced in C57/BJ6 mice (Harlan Nossan, Milan, Italy) by addition of DSS (2%) to their daily drinking water for 7 days followed by a 3-day recovery period without DSS. The severity of colitis was assessed using these set of criteria: (1) clinical index (daily changes in body weight; diarrhea and rectal bleeding); (2) macroscopic evidence of colitis (shortening of colon length). On day 10 of the experiment, animals were sacrificed. Treatments were performed with either solvent alone or drugs once a day for the entire duration of the experiments from day 1 to day 7. The colon lengths were measured and colon tissues were harvested for biochemical measurements, histopathological assessment and scoring. (Tang Y, Preuss F, Turek FW, Jakate S, Keshavarzian A. Sleep deprivation worsens inflammation and delays recovery in a mouse model of colitis. Sleep Med. 2009 Jun; 10(6):597-603).

### Model of spinal cord injury

Male adult CD1 mice (25-30 g, Harlan Nossan, Milan, Italy) were used for all studies. Mice were housed in individual cages (5 for each group)and maintained under 12:12 light-dark cycle at 21 ± 1 °C and 50 ± 5% humidity.

The animals were acclimated to their environment for 1 wk and had ad libitum access to tap water and standard rodent standard diet. All animal experiments complied with regulations in Italy (D.M. 116192), Europe (O.J. of E.C. L 358/1 12/18/1986) and USA (Animal Welfare Assurance No A5594-01, Department of Health and Human Services, USA). All behavioral testing was conducted in compliance with the NHI laboratory animal care guidelines and with protocols approved by the Institutional Animal Care and Use Committee (Council directive # 87-848, October 19, 1987, Ministere de l'Agriculture et de la Forêt, Service Vétérinaire de la Santé et de la Protection Animale, permission # 92-256 to SC).

Spinal cord injury (SCI) was induced in male adult mice by the clip compression technique (Genovese et al. J. Pharmacol. Exp. Ther. 2008a, 325, 100-14). SHAM-operated animals were taken as control. Drugs were given i.p. one and 6 hours post injury. Animal care was in compliance with Italian regulations on protection of animals used for experimental and other scientific purpose (D.M. 116192) and with the EEC regulations (O.J. of E.C. L 358/1 12/18/1986). Animals were sacrificed at post-injury (at least five animals/experimental group were analysed).

The motor function of mice subjected to SCI was assessed once a day for 7 days after injury.

### Grading of motor disturbance.

The locomotor performance of animals was analyzed using the Basso mouse scale (BMS) open-field score for 20 day after injury, since the BMS has been shown to be a valid locomotor rating scale for mice. The evaluations were made by two blind observers for all analyzed groups.

Briefly, the BMS is a nine-point scale that pro vides a gross indication of locomotor ability and determines the phases of locomotor recovery and features of locomotion. The BMS score was determined for ten mice in each group. Spinal cord tissues were taken at 24 hours after trauma. Tissue was used for histological grading of the damage or for immunocytochemistry with inflammation, cell death or autophagy markers.

### Example a: analysis of the cytotoxic effects of compounds of formula (I) on cell viability and their synergistic activity with cisnlatin and etoposide.

To evaluate the ability of each compound to inhibit cellular proliferation, cells were seeded in 24-well plates and treated for 48 and 72 hours. After the treatments the MTT assay and trypan blue assay were performed.

Several compounds, listed below, showed an IC₅₀ values below 300 nM:
2-bromo-N-(6-(4-(pyridin-3-yl)-1H-1,2,3-triazol-1-yl)hexyl)benzenesulfonamide
N-(6-(4-(pyridin-3-yl)-1H-1,2,3-triazol-1-yl)hexyl)-[1,1'-biphenyl]-2-sulfonamide
2'-((6-(4-(pyridin-3-yl)-1H-1,2,3-triazol-1-yl)hexyl)oxy)-[1,1'-biphenyl]-4-carboxylic acid
2'-((6-(4-(pyridin-3-yl)-1H-1,2,3-triazol-1-yl)hexyl)oxy)-[1,1'-biphenyl]-3-carboxylic acid
2-bromo-N-(7-(4-(pyridin-3-yl)-1H-1,2,3-triazol-1-yl)heptyl)benzenesulfonamide
2-iodo-N-(7-(4-(pyridin-3-yl)-1H-1,2,3-triazol-1-yl)heptyl)benzamide
N-(7-(4-(pyridin-3-yl)-1H-1,2,3-triazol-1-yl)heptyl)-[1,1'-biphenyl]-2-sulfonamide
2'-((7-(4-(pyridin-3-yl)-1H-1,2,3-triazol-1-yl)heptyl)carbamoyl)-[1,1'-biphenyl]-4-carboxylic acid
3-(2-((6-(4-(pyridin-3-yl)-1H-1,2,3-triazol-1-yl)hexyl)oxy)phenyl)pyridine
2-(pyridin-3-yl)-N-(7-(4-(pyridin-3-yl)-1H-1,2,3-triazol-1-yl)heptyl)benzamide
3-(1-(8-([1,1'-biphenyl]-2-yloxy)octyl)-1H-1,2,3-triazol-4-yl)pyridine
3-(1-(7-([1,1'-biphenyl]-2-yloxy)heptyl)-1H-1,2,3-triazol-4-yl)pyridine
N-(7-(4-(pyridin-3-yl)-1H-1,2,3-triazol-1-yl)heptyl)-[1,1'-biphenyl]-2-carboxamide
3-(1-(6-([1,1'-biphenyl]-2-yloxy)hexyl)-1H-1,2,3 -triazol-4-yl)pyridine
N-(6-(4-(pyridin-3-yl)-1H-1,2,3-triazol-1-yl)hexyl)-[1,1'-biphenyl]-2-carboxamide
2'-((7-(4-(pyridin-3-yl)-1H-1,2,3-triazol-1-yl)heptyl)carbamoyl)-[1,1'-biphenyl]-2-carboxylic acid
3-(1-(6-((2'-methyl-[1,1'-biphenyl]-2-yl)oxy)hexyl)-1H-1,2,3-triazol-4-yl)pyridine
2'-methyl-N-(7-(4-(pyridin-3-yl)-1H-1,2,3-triazol-1-yl)heptyl)-[1,1'-biphenyl]-2-carboxamide
N-([1,1'-biphenyl]-2-ylmethyl)-7-(4-(pyridin-3-yl)-1H-1,2,3-triazol-1-yl)heptan-1-amine
N-([1,1'-biphenyl]-2-ylmethyl)-6-(4-(pyridin-3-yl)-1H-1,2,3-triazol-1-yl)hexan-1-amine
1-([1,1'-biphenyl]-2-yl)-3-(6-(4-(pyridin-3-yl)-1H-1,2,3-triazol-1-yl)hexyl)urea
1-([1,1'-biphenyl]-2-yl)-3-(7-(4-(pyridin-3-yl)-1H-1,2,3-triazol-1-yl)heptyl)urea
6-(4-(pyridin-3-yl)-1H-1,2,3-triazol-1-yl)hexyl [1,1'-biphenyl]-2-ylcarbamate
8-(4-(pyridin-3-yl)-1H-1,2,3-triazol-1-yl)octyl [1,1'-biphenyl]-2-ylcarbamate
7-(4-(pyridin-3-yl)-1H-1,2,3-triazol-1-yl)heptyl [1,1'-biphenyl]-2-ylcarbamate
1-([1,1'-biphenyl]-2-yl)-3-(8-(4-(pyridin-3-yl)-1H-1,2,3-triazol-1-yl)octyl)urea
N-([1,1'-biphenyl]-2-ylmethyl)-8-(4-(pyridin-3-yl)-1H-1,2,3-triazol-1-yl)octan-1-amine
2-bromo-N-(8-(4-(pyridin-3-yl)-1H-1,2,3-triazol-1-yl)octyl)benzenesulfonamide
N-(8-(4-(pyridin-3-yl)-1H-1,2,3-triazol-1-yl)octyl)-[1,1'-biphenyl]-2-sulfonamide
3-(1-(6-(4-([1,1'-biphenyl]-2-yl)-1H-1,2,3-triazol-1-yl)hexyl)-1H-1,2,3-triazol-4-yl)pyridine
3-(1-(7-(4-([1,1'-biphenyl]-2-yl)-1H-1,2,3-triazol-1-yl)heptyl)-1H-1,2,3-triazol-4-yl)pyridine
3-(1-(8-(4-([1,1'-biphenyl]-2-yl)-1H-1,2,3-triazol-1-yl)octyl)-1H-1,2,3-triazol-4-yl)pyridine
N-(4-(2-(4-(pyridin-3-yl)-1H-1,2,3-triazol-1-yl)ethyl)benzyl)-[1,1'-biphenyl]-2-carboxamide
N-(4-((4-(pyridin-3-yl)-1H-1,2,3-triazol-1-yl)methyl)phenethyl)-[1,1'-biphenyl]-2-carboxamide
3-(1-(4-(2-([1,1'-biphenyl]-2-yloxy)ethyl)benzyl)-1H-1,2,3-triazol-4-yl)pyridine
3-(1-(4-(([1,1'-biphenyl]-2-yloxy)methyl)phenethyl)-1H-1,2,3-triazol-4-yl)pyridine
N-(4-(3-(4-(pyridin-3-yl)-1H-1,2,3-triazol-1-yl)propyl)phenyl)-[1,1'-biphenyl]-2-carboxamide
3-(1-(4-(3-([1,1'-biphenyl]-2-yloxy)propyl)phenyl)-1H-1,2,3-triazol-4-yl)pyridine
N-(3-(4-(4-(pyridin-3-yl)-1H-1,2,3-triazol-1-yl)phenyl)propyl)-[1,1'-biphenyl]-2-carboxamide
1-([1,1'-biphenyl]-2-yl)-3-(4-((4-(pyridin-3-yl)-1H-1,2,3-triazol-1-yl)methyl)phenethyl)urea
3-(1-(4-(2-(4-([1,1'-biphenyl]-2-yl)-1H-1,2,3-triazol-1-yl)ethyl)benzyl)-1H-1,2,3-triazol-4-yl)pyridine
4-((4-(pyridin-3-yl)-1H-1,2,3-triazol-1-yl)methyl)phenethyl [1,1'-biphenyl]-2-ylcarbamate
N-([1,1'-biphenyl]-2-ylmethyl)-2-(4-((4-(pyridin-3-yl)-1H-1,2,3-triazol-1-yl)methyl)phenyl)ethan-1-amine
2-bromo-N-(4-((4-(pyridin-3-yl)-1H-1,2,3-triazol-1-yl)methyl)phenethyl)benzenesulfonamide
N-(4-((4-(pyridin-3-yl)-1H-1,2,3-triazol-1-yl)methyl)phenethyl)-[1,1'-biphenyl]-2-sulfonamide

To evaluate the ability of each compound to potentiate the cytotoxic effect of cisplatin and etoposide, cells were seeded in 24-well plates and treated for 48 and 72 hours with concentrations that did not have a relevant effect on cytotoxicity and a dose-response curve of cisplatin and etoposide was performed. After the treatments the MTT assay and trypan blue assay were performed.

Several compounds, listed below, shifted at least 10 fold the IC₅₀ of both cisplatin and etoposide:
3-(2-((6-(4-(pyridin-3-yl)-1H-1,2,3-triazol-1-yl)hexyl)oxy)phenyl)pyridine
2-(pyridin-3-yl)-N-(7-(4-(pyridin-3-yl)-1H-1,2,3-triazol-1-yl)heptyl)benzamide
3-(1-(8-([1,1'-biphenyl]-2-yloxy)octyl)-1H-1,2,3-triazol-4-yl)pyridine
3-(1-(7-([1,1'-biphenyl]-2-yloxy)heptyl)-1H-1,2,3-triazol-4-yl)pyridine
N-(7-(4-(pyridin-3-yl)-1H-1,2,3-triazol-1-yl)heptyl)-[1,1'-biphenyl]-2-carboxamide
3-(1-(6-([1,1'-biphenyl]-2-yloxy)hexyl)-1H-1,2,3-triazol-4-yl)pyridine
N-(6-(4-(pyridin-3-yl)-1H-1,2,3-triazol-1-yl)hexyl)-[1,1'-biphenyl]-2-carboxamide
2'-((7-(4-(pyridin-3-yl)-1H-1,2,3-triazol-1-yl)heptyl)carbamoyl)-[1,1'-biphenyl]-2-carboxylic acid
2'-methyl-N-(7-(4-(pyridin-3-yl)-1H-1,2,3-triazol-1-yl)heptyl)-[1,1'-biphenyl]-2-carboxamide
1-([1,1'-biphenyl]-2-yl)-3-(6-(4-(pyridin-3-yl)-1H-1,2,3-triazol-1-yl)hexyl)urea
1-([1,1'-biphenyl]-2-yl)-3-(7-(4-(pyridin-3-yl)-1H-1,2,3-triazol-1-yl)heptyl)urea
6-(4-(pyridin-3-yl)-1H-1,2,3-triazol-1-yl)hexyl[1,1'-biphenyl]-2-ylcarbamate
7-(4-(pyridin-3-yl)-1H-1,2,3-triazol-1-yl)heptyl[1,1'-biphenyl]-2-ylcarbamate
N-(8-(4-(pyridin-3-yl)-1H-1,2,3-triazol-1-yl)octyl)-[1,1'-biphenyl]-2-sulfonamide
3-(1-(7-(4-([1,1'-biphenyl]-2-yl)-1H-1,2,3-triazol-1-yl)heptyl)-1H-1,2,3-triazol-4-yl)pyridine
N-(4-((4-(pyridin-3-yl)-1H-1,2,3-triazol-1-yl)methyl)phenethyl)-[1,1'-biphenyl]-2-carboxamide
3-(1-(4-(2-([1,1'-biphenyl]-2-yloxy)ethyl)benzyl)-1H-1,2,3-triazol-4-yl)pyridine
N-(4-(3-(4-(pyridin-3-yl)-1H-1,2,3-triazol-1-yl)propyl)phenyl)-[1,1'-biphenyl]-2-carboxamide
1-([1,1'-biphenyl]-2-yl)-3-(4-((4-(pyridin-3-yl)-1H-1,2,3-triazol-1-yl)methyl)phenethyl)urea
N-([1,1'-biphenyl]-2-ylmethyl)-2-(4-((4-(pyridin-3-yl)-1H-1,2,3-triazol-1-yl)methyl)phenyl)ethan-1-amine
2-bromo-N-(4-((4-(pyridin-3-yl)-1H-1,2,3-triazol-1-yl)methyl)phenethyl)benzenesulfonamide
N-(4-((4-(pyridin-3-yl)-1H-1,2,3-triazol-1-yl)methyl)phenethyl)-[1,1'-biphenyl]-2-sulfonamide

To evaluate the ability of autophagy inhibitors to potentiate the cytotoxic effect of the compounds described, cells were seeded in 24-well plates and treated for 24, 48 and 72 hours with cloroquine (1 µM) and a dose-response curve was performed. After the treatments the MTT assay and trypan blue assay were performed.

Several compounds, listed below, displayed an anticipation of cell death (the IC₅₀ was obtained in the first 24 hours of treatment) and a shift of at least 10-fold for the IC₅₀ at 48 and 72 hours:
3-(2-((6-(4-(pyridin-3-yl)-1H-1,2,3-triazol-1-yl)hexyl)oxy)phenyl)pyridine
2-(pyridin-3-yl)-N-(7-(4-(pyridin-3-yl)-1H-1,2,3-triazol-1-yl)heptyl)benzamide
3-(1-(8-([1,1'-biphenyl]-2-yloxy)octyl)-1H-1,2,3-triazol-4-yl)pyridine
3-(1-(7-([1,1'-biphenyl]-2-yloxy)heptyl)-1H-1,2,3-triazol-4-yl)pyridine
N-(7-(4-(pyridin-3-yl)-1H-1,2,3-triazol-1-yl)heptyl)-[1,1'-biphenyl]-2-carboxamide
3-(1-(6-([1,1'-biphenyl]-2-yloxy)hexyl)-1H-1,2,3-triazol-4-yl)pyridine
N-(6-(4-(pyridin-3-yl)-1H-1,2,3-triazol-1-yl)hexyl)-[1,1'-biphenyl]-2-carboxamide
2'-((7-(4-(pyridin-3-yl)-1H-1,2,3-triazol-1-yl)heptyl)carbamoyl)-[1,1'-biphenyl]-2-carboxylic acid
2'-methyl-N-(7-(4-(pyridin-3-yl)-1H-1,2,3-triazol-1-yl)heptyl)-[1,1'-biphenyl]-2-carboxamide
1-([1,1'-biphenyl]-2-yl)-3-(6-(4-(pyridin-3-yl)-1H-1,2,3-triazol-1-yl)hexyl)urea
1-([1,1'-biphenyl]-2-yl)-3-(7-(4-(pyridin-3-yl)-1H-1,2,3-triazol-1-yl)heptyl)urea
6-(4-(pyridin-3-yl)-1H-1,2,3-triazol-1-yl)hexyl[1,1'-biphenyl]-2-ylcarbamate
7-(4-(pyridin-3-yl)-1H-1,2,3-triazol-1-yl)heptyl[1,1'-biphenyl]-2-ylcarbamate
N-(8-(4-(pyridin-3-yl)-1H-1,2,3-triazol-1-yl)octyl)-[1,1'-biphenyl]-2-sulfonamide
3-(1-(7-(4-([1,1'-biphenyl]-2-yl)-1H-1,2,3-triazol-1-yl)heptyl)-1H-1,2,3-triazol-4-yl)pyridine
N-(4-((4-(pyridin-3-yl)-1H-1,2,3-triazol-1-yl)methyl)phenethyl)-[1,1'-biphenyl]-2-carboxamide
3-(1-(4-(2-([1,1'-biphenyl]-2-yloxy)ethyl)benzyl)-1H-1,2,3-triazol-4-yl)pyridine
N-(4-(3-(4-(pyridin-3-yl)-1H-1,2,3-triazol-1-yl)propyl)phenyl)-[1,1'-biphenyl]-2-carboxamide
1-([1,1'-biphenyl]-2-yl)-3-(4-((4-(pyridin-3-yl)-1H-1,2,3-triazol-1-yl)methyl)phenethyl)urea
N-([1,1'-biphenyl]-2-ylmethyl)-2-(4-((4-(pyridin-3-yl)-1H-1,2,3-triazol-1-yl)methyl)phenyl)ethan-1-amine
2-bromo-N-(4-((4-(pyridin-3-yl)-1H-1,2,3-triazol-1-yl)methyl)phenethyl)benzenesulfonamide
N-(4-((4-(pyridin-3-yl)-1H-1,2,3-triazol-1-yl)methyl)phenethyl)-[1,1'-biphenyl]-2-sulfonamide

### Example b: indirect analysis of the inhibition properties of compounds of formula (I) on NAMPT activity

To evaluate the ability of some compounds to inhibit NAMPT activity, cells were seeded in 24-well plates and after 24 h of treatment total cellular NAD(P) was measured using a cycling assay.

Several compounds, listed below, were able to decrease NAD levels at concentrations below 300 nM:
3-(2-((6-(4-(pyridin-3-yl)-1H-1,2,3-triazol-1-yl)hexyl)oxy)phenyl)pyridine
2-(pyridin-3-yl)-N-(7-(4-(pyridin-3-yl)-1H-1,2,3-triazol-1-yl)heptyl)benzamide
3-(1-(8-([1,1'-biphenyl]-2-yloxy)octyl)-1H-1,2,3-triazol-4-yl)pyridine
3-(1-(7-([1,1'-biphenyl]-2-yloxy)heptyl)-1H-1,2,3 -triazol-4-yl)pyridine
N-(7-(4-(pyridin-3-yl)-1H-1,2,3-triazol-1-yl)heptyl)-[1,1'-biphenyl]-2-carboxamide
3-(1-(6-([1,1'-biphenyl]-2-yloxy)hexyl)-1H-1,2,3-triazol-4-yl)pyridine
N-(6-(4-(pyridin-3-yl)-1H-1,2,3-triazol-1-yl)hexyl)-[1,1'-biphenyl]-2-carboxamide
2'-((7-(4-(pyridin-3-yl)-1H-1,2,3-triazol-1-yl)heptyl)carbamoyl)-[1,1'-biphenyl]-2-carboxylic acid
2'-methyl-N-(7-(4-(pyridin-3-yl)-1H-1,2,3-triazol-1-yl)heptyl)-[1,1'-biphenyl]-2-carboxamide
1-([1,1'-biphenyl]-2-yl)-3-(6-(4-(pyridin-3-yl)-1H-1,2,3-triazol-1-yl)hexyl)urea
1-([1,1'-biphenyl]-2-yl)-3-(7-(4-(pyridin-3-yl)-1H-1,2,3-triazol-1-yl)heptyl)urea
6-(4-(pyridin-3-yl)-1H-1,2,3-triazol-1-yl)hexyl[1,1'-biphenyl]-2-ylcarbamate
7-(4-(pyridin-3-yl)-1H-1,2,3-triazol-1-yl)heptyl[1,1'-biphenyl]-2-ylcarbamate
N-(8-(4-(pyridin-3-yl)-1H-1,2,3-triazol-1-yl)octyl)-[1,1'-biphenyl]-2-sulfonamide
3-(1-(7-(4-([1,1'-biphenyl]-2-yl)-1H-1,2,3-triazol-1-yl)heptyl)-1H-1,2,3-triazol-4-yl)pyridine
N-(4-((4-(pyridin-3-yl)-1H-1,2,3-triazol-1-yl)methyl)phenethyl)-[1,1'-biphenyl]-2-carboxamide
3-(1-(4-(2-([1,1'-biphenyl]-2-yloxy)ethyl)benzyl)-1H-1,2,3-triazol-4-yl)pyridine
N-(4-(3-(4-(pyridin-3-yl)-1H-1,2,3-triazol-1-yl)propyl)phenyl)-[1,1'-biphenyl]-2-carboxamide
1-([1,1'-biphenyl]-2-yl)-3-(4-((4-(pyridin-3-yl)-1H-1,2,3-triazol-1-yl)methyl)phenethyl)urea
N-([1,1'-biphenyl]-2-ylmethyl)-2-(4-((4-(pyridin-3-yl)-1H-1,2,3-triazol-1-yl)methyl)phenyl)ethan-1-amine
2-bromo-N-(4-((4-(pyridin-3-yl)-1H-1,2,3-triazol-1-yl)methyl)phenethyl)benzenesulfonamide
N-(4-((4-(pyridin-3-yl)-1H-1,2,3-triazol-1-yl)methyl)phenethyl)-[1,1'-biphenyl]-2-sulfonamide

### Example c: evaluation of the direct inhibition of NAMPT

Several compounds, listed below, were able to inhibit the enzymatic activity of NAMPT, as assessed with a cycling assay:
3-(2-((6-(4-(pyridin-3-yl)-1H-1,2,3-triazol-1-yl)hexyl)oxy)phenyl)pyridine
2-(pyridin-3-yl)-N-(7-(4-(pyridin-3-yl)-1H-1,2,3-triazol-1-yl)heptyl)benzamide
3-(1-(8-([1,1'-biphenyl]-2-yloxy)octyl)-1H-1,2,3-triazol-4-yl)pyridine
3-(1-(7-([1,1'-biphenyl]-2-yloxy)heptyl)-1H-1,2,3-triazol-4-yl)pyridine
N-(7-(4-(pyridin-3-yl)-1H-1,2,3-triazol-1-yl)heptyl)-[1,1'-biphenyl]-2-carboxamide
3-(1-(6-([1,1'-biphenyl]-2-yloxy)hexyl)-1H-1,2,3-triazol-4-yl)pyridine
N-(6-(4-(pyridin-3-yl)-1H-1,2,3-triazol-1-yl)hexyl)-[1,1'-biphenyl]-2-carboxamide
2'-((7-(4-(pyridin-3-yl)-1H-1,2,3-triazol-1-yl)heptyl)carbamoyl)-[1,1'-biphenyl]-2-carboxylic acid
2'-methyl-N-(7-(4-(pyridin-3-yl)-1H-1,2,3-triazol-1-yl)heptyl)-[1,1'-biphenyl]-2-carboxamide
1-([1,1'-biphenyl]-2-yl)-3-(6-(4-(pyridin-3-yl)-1H-1,2,3-triazol-1-yl)hexyl)urea
1-([1,1'-biphenyl]-2-yl)-3-(7-(4-(pyridin-3-yl)-1H-1,2,3-triazol-1-yl)heptyl)urea
6-(4-(pyridin-3-yl)-1H-1,2,3-triazol-1-yl)hexyl[1,1'-biphenyl]-2-ylcarbamate
7-(4-(pyridin-3-yl)-1H-1,2,3-triazol-1-yl)heptyl[1,1'-biphenyl]-2-ylcarbamate
N-(8-(4-(pyridin-3-yl)-1H-1,2,3-triazol-1-yl)octyl)-[1,1'-biphenyl]-2-sulfonamide
3-(1-(7-(4-([1,1'-biphenyl]-2-yl)-1H-1,2,3-triazol-1-yl)heptyl)-1H-1,2,3-triazol-4-yl)pyridine
N-(4-((4-(pyridin-3-yl)-1H-1,2,3-triazol-1-yl)methyl)phenethyl)-[1,1'-biphenyl]-2-carboxamide
3-(1-(4-(2-([1,1'-biphenyl]-2-yloxy)ethyl)benzyl)-1H-1,2,3-triazol-4-yl)pyridine
N-(4-(3-(4-(pyridin-3-yl)-1H-1,2,3-triazol-1-yl)propyl)phenyl)-[1,1'-biphenyl]-2-carboxamide
1-([1,1'-biphenyl]-2-yl)-3-(4-((4-(pyridin-3-yl)-1H-1,2,3-triazol-1-yl)methyl)phenethyl)urea
N-([1,1'-biphenyl]-2-ylmethyl)-2-(4-((4-(pyridin-3-yl)-1H-1,2,3-triazol-1-yl)methyl)phenyl)ethan-1-amine
2-bromo-N-(4-((4-(pyridin-3-yl)-1H-1,2,3-triazol-1-yl)methyl)phenethyl)benzenesulfonamide
N-(4-((4-(pyridin-3-yl)-1H-1,2,3-triazol-1-yl)methyl)phenethyl)-[1,1'-biphenyl]-2-sulfonamide

### Example d: evaluation of neuronrotective activity of compounds of formula (I)

As previously described, the spinal cord of male adult CD1 mice was injured by the clip compression technique, compound of formula (I) was administered and the motor function was assessed.

The single compound tested, listed below, was able to partially protect from injury and to induce partial motor recovery:
N-(7-(4-(pyridin-3-yl)-1H-1,2,3-triazol-1-yl)heptyl)-[1,1'-biphenyl]-2-carboxamide
3-(1-(6-([1,1'-biphenyl]-2-yloxy)hexyl)-1H-1,2,3-triazol-4-yl)pyridine.

### Example e: evaluation of the anti-inflammatory activity of compounds of formula (I)

As previously described, the colon of DNBS- or dextran treated animals was removed after the induction of colitis. Compounds of formula (I) were administered intraperitoneally (i.p.).

The compounds, listed below, were able to protect from both DNBS- and Dextran-induced colitis at doses of between 20mg/Kg and 500 mg/Kg as assessed by the macroscopic damage score:
3-(1-(6-([1,1'-biphenyl]-2-yloxy)hexyl)-1H-1,2,3-triazol-4-yl)pyridine;
N-(7-(4-(pyridin-3-yl)-1H-1,2,3-triazol-1-yl)heptyl)-[1,1'-biphenyl]-2-carboxamide;
2'-((7-(4-(pyridin-3-yl)-1H-1,2,3-triazol-1-yl)heptyl)carbamoyl)-[1,1'-biphenyl]-2-carboxylic acid.

Furthermore, the compounds listed below given i.p. at doses of between 20mg/Kg and 500 mg/Kg were able to reduce TNFalpha, VEGF, MPO, and NAMPT, all indexes of inflammation, in the colon of DNBS-treated mice compared to mice in which colitis was induced but that were not treated:
3-(1-(6-([1,1'-biphenyl]-2-yloxy)hexyl)-1H-1,2,3-triazol-4-yl)pyridine;
N-(7-(4-(pyridin-3-yl)-1H-1,2,3-triazol-1-yl)heptyl)-[1,1'-biphenyl]-2-carboxamide;
2'-((7-(4-(pyridin-3-yl)-1H-1,2,3-triazol-1-yl)heptyl)carbamoyl)-[1,1'-biphenyl]-2-carboxylic acid.

***Microsomal stability with rat and human liver microsomes of selected compounds of formula (I) compared with compound 8 of*** J. Med Chem. 2010, 53, 616-623***.***

Male Wistar rat liver microsomes (RLM), (protein concentration: 25 mg/ml determined using the Omura and Sato method, total P450: 0.64 nmol/mg protein) were used throughout this study and prepared using an internal protocol. The cryopreserved pooled human liver microsomes (HLM), (pooled mixed sex, ten individual donors, protein concentration: 20 mg/ml, total P450: 0.36 nmol/mg) were purchased from BD Gentest™ (Woburn, MA) and used throughout this study.

The standard incubation mixture (1 ml final volume), was carried out in a 50 mM TRIS (Tris[hydroxymethyl]aminomethane) buffer (pH 7.4) containing 10 µl of acetonitrile (1% of total volume), and 50 µM substrate compounds. After pre-equilibration of the mixture, an appropriate volume of RLM or HLM suspension was added to give a final protein concentration of 1.0 mg/mL. The mixture was shaken for 60 minutes at 37°C. Control incubations were carried out without microsomes. Each incubation was stopped by addition of 1 ml ice-cold acetonitrile, vortexed and centrifuged at 11,000 r.p.m. for 5 min. The supernatants were analysed by LC-DAD-UV and LC-ESI-MS systems to determine the metabolic stability (% substrate residue) and the structure of metabolites respectively (Table 1).

### LC-DAD-UV and LC ESI MS analyses.

A Shimadzu HPLC system (Shimadzu, Kyoto, Japan), consisting of two LC-10AD Vp module pumps, an SLC-10A Vp system controller, an SIL-1OAD Vp autosampler, and a DGU-14-A on-line degasser were used for the analysis. Chromatographic separation was performed on a *Phenomenex Luna C18(2) 150 x 4.6 mm (5 µm)* (Phenomenex srl, Castel Bolognese, Italy) as a stationary phase protected by a C18 SecurityGuard (Phenomenex srl, Castel Bolognese, Italy). The SPD-M10Avp photodiode array detector was used to detect the analytes. LC-Solution 1.24 software was used to process the chromatograms. Aliquots (20 µL) of supernatants obtained from incubations were injected onto the HPLC system and eluted with a mobile phase (flow rate 1.0 mL/min) consisting of a A: 0.5% formic acid solution, B: acetonitrile/methanol 8:2. A Thermo Finningan LCQ Deca XP *plus* system equipped with a quaternary pump, a Surveyor AS autosampler, a Surveyor photodiode array detector and a vacuum degasser was used for LC/MS analyses (Thermo Electron Corporation, Waltham, MA). All the chromatographic separations were performed on a *Phenomenex Luna C18*(*2*)*150 x 2 mm (4 µm)* (kept at a 35 °C) as a stationary phase protected by a C18 SecurityGuard (Phenomenex srl, Castel Bolognese, Italy). Aliquots (2.0 µl) of supernatants obtained from incubations were injected onto the system and eluted with a mobile phase (flow rate 0.2 ml/min) consisting of a A: 0.2% formic acid solution, B: acetonitrile/methanol 8:2. The eluate was injected into the electrospray ion source (ESI) and MS and MSⁿ spectra were acquired and processed using Xcalibur® software. The operating conditions on the ion trap mass spectrometer were as follows: Positive mode, spray voltage, 5.30 kV; source current, 80 µA; capillary temperature, 350 °C; capillary voltage, 21.00 V; tube lens offset, 15.00 V; multipole 1 offset, -5.75 V; multipole 2 offset, -9.00 V; sheath gas flow (N₂), 55 Auxiliary Units.

**Table 2. Hydrolytic stability in the presence of rat liver microsomes (RLM) or human liver microsomes (HLM)**

| **Compound** | **RLM stability % substrate residue (area peak)** | **HLM stability % substrate residue (area peak)** | **Metabolites generated from hydrolyses** |
|---|---|---|---|
| Compound 8 on J. Med. Chem. 2010, 53, 616-623 | <10 | <10 | yes |
| 1-([1,1'-biphenyl]-2-yl)-3-(7-(4-(pyridin-3-yl)-1*H*-1,2,3-triazol-1-yl)heptyl)urea | >95 | >98 | yes (traces) |
| 3 -(2-((6-(4-(pyridin-3 -yl)-1*H*-1,2,3-triazol-1-yl)hexyl)oxy)phenyl)pyridine | >95 | >99 | no |
| *N*-(4-(3-(4-(pyridin-3-yl)-1*H-*1,2,3-triazol-1-yl)propyl)phenyl)-[1,1'-biphenyl]-2-carboxamide | >99 | >98 | no |
| 3-(1-(6-([1,1'-biphenyl]-2-yloxy)hexyl)-1*H*-1,2,3-triazol-4-yl)pyridine | >98 | >96 | no |
| *N*-(7-(4-(pyridin-3-yl)-1*H-*1,2,3-triazol-1-yl)heptyl)-[1,1'-biphenyl]-2-carboxamide | >99 | >99 | no |
| 2-(pyridin-3-yl)-*N*-(7-(4-(pyridin-3-yl)-1*H*-1,2,3-triazol-1-yl)heptyl)benzamide | >99 | >96 | no |
| 3-(l-(4-(3-([1,1-biphenyl]-2-yloxy)propyl)phenyl)-1*H-*1,2,3-triazol-4-yl)pyridine | >92 | >98 | no |
| 1-([ 1,1'-biphenyl]-2-yl)-3-(4-((4-(pyridin-3-yl)-1*H*-1,2,3-triazol-1-yl)methyl)phenethyl)urea | >99 | >99 | no |
| *N*-(8-(4-(pyridin-3-yl)-1*H-*1,2,3-triazol-1-yl)octyl)-[1,1'-biphenyl]-2-sulfonamide | >99 | >99 | no |
| 3-(1-(7-(4-([1,1'-biphenyl]-2-yl)-1*H*-1,2,3-triazol-1-yl)heptyl)-1*H*-1,2,3 -triazol-4-yl)pyridine | >99 | >99 | no |
| *N*-(4-((4-(pyridin-3-yl)-1*H-*1,2,3-triazol-1-yl)methyl)phenethyl)-[1,1'-biphenyl]-2-carboxamide | >99 | >99 | no |
| 3-(1-(4-(2-([1,1'-biphenyl]-2-yloxy)ethyl)benzyl)-1*H-*1,2,3-triazol-4-yl)pyridine | >97 | >99 | no |

Naturally, while the principle of the invention remains the same, the details of construction and the embodiments may widely vary with respect to what has been described and illustrated purely by way of example, without departing from the scope of the present invention.

## Claims

1. Compound of formula (I): 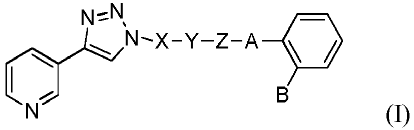 wherein
X is (CH₂)n;
Y is selected from (CH₂)ₘ, *para* substituted phenyl;
Z is (CH₂)ₚ;
n is an integer 0 to 4;
m is an integer 1 to 8;
p is an integer 0 to 4;
A is selected from the following groups: 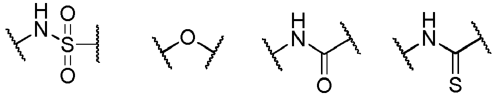 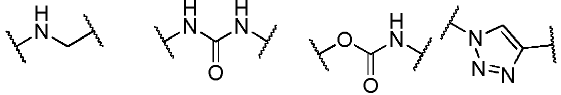
B is selected from Br, Cl, I, F, Methyl, Isopropyl, tert-butyl, substituted or unsubstituted aryl or heteroaryl (Ar) group,
wherein Ar is selected from benzene, furan, thiophene, pyrrolidine, pyrrole, 1,2,3-triazole, pyrazole, imidazole, oxazole, isooxazole, thiazole, isothiazole, 1,2,3-oxadiazole, 1,2,4-oxadiazole, 1,2,5-oxadiazole, pyridine, pyridazine, pyrimidine, pyrazine, naphthalene, indole, 1*H*-indazole, 1*H*-benzo[d]imidazole, benzofuran, benzothiophene, quinoline, isoquinoline, quinoxaline, or carbazole, and
wherein when B is selected from a substituted aryl or heteroaryl (Ar) group, the one or more substituents are independently selected from -COOH, -NH₂, - COOR¹, -CONH₂, -CONHR¹, -CONR¹R², -R³;
wherein
R¹ and R² are identical or different from each other and independently selected from -H, straight or branched, unsubstituted C₁₋₈ alkyl, unsubstituted C₃₋₆ cycloalkyl,
R³ is selected from straight or branched, unsubstituted C₁₋₈ alkyl, unsubstituted C₃₋₆ cycloalkyl;
pharmaceutically acceptable, hydrate, solvate or salt thereof.

2. Compound according to claim 1, wherein any of R¹, R² and R³ groups, if present, are independently selected from methyl, ethyl, n-propyl, isopropyl, n-butyl, isobutyl, tert-butyl, cyclopropyl, cyclobutyl, cyclopentyl, cyclohexyl, n-pentyl, n-hexyl; n-heptyl, n-octyl.

3. Compound according to any one of the preceding claims, selected from:
2-bromo-N-(6-(4-(pyridin-3-yl)-1H-1,2,3-triazol-1-yl)hexyl)benzenesulfonamide
N-(6-(4-(pyridin-3-yl)-1H-1,2,3-triazol-1-yl)hexyl)-[1,1'-biphenyl]-2-sulfonamide
2'-((6-(4-(pyridin-3-yl)-1H-1,2,3-triazol-1-yl)hexyl)oxy)-[1,1'-biphenyl]-4-carboxylic acid
2'-((6-(4-(pyridin-3-yl)-1H-1,2,3-triazol-1-yl)hexyl)oxy)-[1,1'-biphenyl]-3-carboxylic acid
2-bromo-N-(7-(4-(pyridin-3-yl)-1H-1,2,3-triazol-1-yl)heptyl)benzenesulfonamide
2-iodo-N-(7-(4-(pyridin-3-yl)-1H-1,2,3-triazol-1-yl)heptyl)benzamide
N-(7-(4-(pyridin-3-yl)-1H-1,2,3-triazol-1-yl)heptyl)-[1,1'-biphenyl]-2-sulfonamide
2'-((7-(4-(pyridin-3-yl)-1H-1,2,3-triazol-1-yl)heptyl)carbamoyl)-[1,1'-biphenyl]-4-carboxylic acid
3-(2-((6-(4-(pyridin-3-yl)-1H-1,2,3-triazol-1-yl)hexyl)oxy)phenyl)pyridine
2-(pyridin-3-yl)-N-(7-(4-(pyridin-3-yl)-1H-1,2,3-triazol-1-yl)heptyl)benzamide
3-(1-(8-([1,1'-biphenyl]-2-yloxy)octyl)-1H-1,2,3-triazol-4-yl)pyridine
3-(1-(7-([1,1'-biphenyl]-2-yloxy)heptyl)-1H-1,2,3-triazol-4-yl)pyridine
N-(7-(4-(pyridin-3-yl)-1H-1,2,3-triazol-1-yl)heptyl)-[1,1'-biphenyl]-2-carboxamide
3-(1-(6-([1,1'-biphenyl]-2-yloxy)hexyl)-1H-1,2,3-triazol-4-yl)pyridine
N-(6-(4-(pyridin-3-yl)-1H-1,2,3-triazol-1-yl)hexyl)-[1,1'-biphenyl]-2-carboxamide
2'-((7-(4-(pyridin-3-yl)-1H-1,2,3-triazol-1-yl)heptyl)carbamoyl)-[1,1'-biphenyl]-2-carboxylic acid
3-(1-(6-((2'-methyl-[1,1'-biphenyl]-2-yl)oxy)hexyl)-1H-1,2,3-triazol-4-yl)pyridine
2'-methyl-N-(7-(4-(pyridin-3-yl)-1H-1,2,3-triazol-1-yl)heptyl)-[1,1'-biphenyl]-2-carboxamide
N-([1,1'-biphenyl]-2-ylmethyl)-7-(4-(pyridin-3-yl)-1H-1,2,3-triazol-1-yl)heptan-1-amine
N-([1,1'-biphenyl]-2-ylmethyl)-6-(4-(pyridin-3-yl)-1H-1,2,3-triazol-1-yl)hexan-1-amine
1-([1,1'-biphenyl]-2-yl)-3-(6-(4-(pyridin-3-yl)-1H-1,2,3-triazol-1-yl)hexyl)urea
1-([1,1'-biphenyl]-2-yl)-3-(7-(4-(pyridin-3-yl)-1H-1,2,3-triazol-1-yl)heptyl)urea
6-(4-(pyridin-3-yl)-1H-1,2,3-triazol-1-yl)hexyl [1,1'-biphenyl]-2-ylcarbamate
8-(4-(pyridin-3-yl)-1H-1,2,3-triazol-1-yl)octyl[1,1'-biphenyl]-2-ylcarbamate
7-(4-(pyridin-3-yl)-1H-1,2,3-triazol-1-yl)heptyl [1,1'-biphenyl]-2-ylcarbamate
1-([1,1'-biphenyl]-2-yl)-3-(8-(4-(pyridin-3-yl)-1H-1,2,3-triazol-1-yl)octyl)urea
N-([1,1'-biphenyl]-2-ylmethyl)-8-(4-(pyridin-3-yl)-1H-1,2,3-triazol-1-yl)octan-1-amine
2-bromo-N-(8-(4-(pyridin-3-yl)-1H-1,2,3-triazol-1-yl)octyl)benzenesulfonamide
N-(8-(4-(pyridin-3-yl)-1H-1,2,3-triazol-1-yl)octyl)-[1,1'-biphenyl]-2-sulfonamide
3-(1-(6-(4-([1,1'-biphenyl]-2-yl)-1H-1,2,3-triazo1-1-yl)hexyl)-1H-1,2,3-triazo 1-4-yl)pyridine
3-(1-(7-(4-([1,1'-biphenyl]-2-yl)-1H-1,2,3-triazol-1-yl)heptyl)-1H-1,2,3-triazo 1-4-yl)pyridine
3-(1-(8-(4-([1,1'-biphenyl]-2-yl)-1H-1,2,3-triazol-1-yl)octyl)-1H-1,2,3-triazo 1-4-yl)pyridine
N-(4-(2-(4-(pyridin-3-yl)-1H-1,2,3-triazol-1-yl)ethyl)benzyl)-[1,1'-biphenyl]-2-carboxamide
N-(4-((4-(pyridin-3-yl)-1H-1,2,3-triazol-1-yl)methyl)phenethyl)-[1,1'-biphenyl]-2-carboxamide
3-(1-(4-(2-([1,1'-biphenyl]-2-yloxy)ethyl)benzyl)-1H-1,2,3-triazol-4-yl)pyridine
3-(1-(4-(([1,1'-biphenyl]-2-yloxy)methyl)phenethyl)-1H-1,2,3-triazol-4-yl)pyridine
N-(4-(3-(4-(pyridin-3-yl)-1H-1,2,3-triazol-1-yl)propyl)phenyl)-[1,1'-biphenyl]-2-carboxamide
3-(1-(4-(3-([1,1'-biphenyl]-2-yloxy)propyl)phenyl)-1H-1,2,3-triazol-4-yl)pyridine
N-(3-(4-(4-(pyridin-3-yl)-1H-1,2,3-triazol-1-yl)phenyl)propyl)-[1,1'-biphenyl]-2-carboxamide
1-([1,1'-biphenyl]-2-yl)-3-(4-((4-(pyridin-3-yl)-1H-1,2,3-triazol-1-yl)methyl)phenethyl)urea
3-(1-(4-(2-(4-([1,1'-biphenyl]-2-yl)-1H-1,2,3-triazol-1-yl)ethyl)benzyl)-1H-1,2,3-triazo1-4-yl)pyridine
4-((4-(pyridin-3-yl)-1H-1,2,3-triazol-1-yl)methyl)phenethyl [1,1'-biphenyl]-2-ylcarbamate
N-([1,1'-biphenyl]-2-ylmethyl)-2-(4-((4-(pyridin-3-yl)-1H-1,2,3-triazol-1-yl)methyl)phenyl)ethan-1-amine
2-bromo-N-(4-((4-(pyridin-3-yl)-1 H-1,2,3-triazol-1-yl)methyl)phenethyl)benzenesulfonamide
N-(4-((4-(pyridin-3-yl)-1H-1,2,3-triazol-1-yl)methyl)phenethyl)-[1,1'-biphenyl]-2-sulfonamide

4. Compound according to any one of claims 1 to 3, for use as therapeutic agent.

5. Compound according to any one of claims 1 to 3, for use in the treatment of an autoimmune disease, preferably selected from lupus erythematosus, psoriasis, rheumatoid arthritis, Crohn's disease, autoimmune encephalitis, graft *vs* host disease.

6. Compound according to any one of claims 1 to 3, for use in the treatment of acute and/or chronic inflammation disorder, preferably selected from ulcerative colitis, asthma, chronic obstructive pulmonary disease (COPD), acute respiratory distress syndrome (ARDS), allergic rhinitis, anaphylaxis, cystic fibrosis, myocardial infarction, heart failure, ischemic diseases and atherosclerosis, acute lung injury, spinal cord injury and sepsis.

7. Compound according to any one of claims 1 to 3, for use in the treatment of a metabolic-associated disorder, preferably selected from obesity and type II diabetes.

8. Compound according to any one of claims 1 to 3, for use in the prevention of neuronal degeneration determined by an injury, preferably selected from traumatic spinal cord injury.

9. Compound according to any one of claims 1 to 3, for use in the treatment of a solid tumor, wherein said solid tumor is preferably selected from Prostate cancer, ovarian cancer, lung cancer, breast cancer, colon cancer, pancreatic adenocarcinoma, melanoma, and neuroblastoma.

10. Compound according to any one of claims 1 to 3, for use in the treatment of a liquid tumor, wherein said liquid tumor is preferably selected from leukaemia and lymphoma.

11. Pharmaceutical composition comprising at least one compound according to any one of claims 1 to 3 and a pharmaceutically acceptable carrier and/or vehicle.

12. Product comprising a compound according to any one of claims 1 to 3 and an anticancer agent as a combined preparation for simultaneous, separate or sequential use in the treatment of a patient suffering from a cancer.

13. Product for use according to claim 12, wherein the anticancer agent is selected from DNA-alkylating agents, DNA intercalator agents and topoisomerase inhibitors.

14. Product comprising a compound according to any one of claims 1 to 3 and an autophagy inhibitor as a combined preparation for simultaneous, separate or sequential use in the treatment of a patient suffering from a cancer.

15. Product comprising a compound according to any one of claims 1 to 3 and an immunomodulating drug or anti-inflammatory drug as a combined preparation for simultaneous, separate or sequential use in the treatment of a patient suffering from a cancer or from an acute or chronic inflammatory disease.

16. Product for use according to claim 14, wherein the autophagy inhibitor is selected from cloroquine, hydroxychloroquine and PI3K modulators, such as GDC-0941 and PI103.

## Patentansprüche

1. Verbindung der Formel (I): 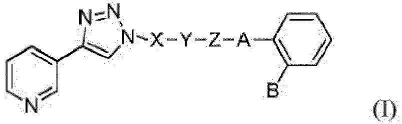 wobei
X (CH₂)ₙ ist;
Y aus (CH₂)ₘ, para-substituiertem Phenyl ausgewählt ist;
Z(CH₂)ₚ ist;
n eine ganze Zahl 0 bis 4 ist;
m eine ganze Zahl 1 bis 8 ist;
p eine ganze Zahl 0 bis 4 ist;
A aus den folgenden Gruppen ausgewählt ist: 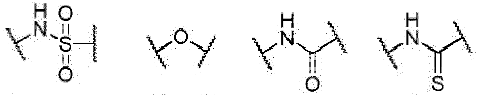 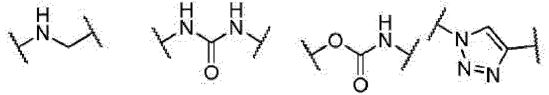
B aus Br, Cl, I, F, Methyl, Isopropyl, tert-Butyl, einer substituierten oder unsubstituierten Aryl- oder Heteroaryl- (Ar-) Gruppe ausgewählt ist,
wobei Ar aus Benzol, Furan, Thiophen, Pyrrolidin, Pyrrol, 1,2,3-Triazol, Pyrazol, Imidazol, Oxazol, Isooxazol, Thiazol, Isothiazol, 1,2,3-Oxadiazol, 1,2,4-Oxadiazol, 1,2,5-Oxadiazol, Pyridin, Pyridazin, Pyrimidin, Pyrazin, Naphthalin, Indol, 1H-Indazol, 1*H*-Benzo[d]imidazol, Benzofuran, Benzothiophen, Chinolin, Isochinolin, Chinoxalin oder Carbazol ausgewählt ist, und
wobei, wenn B aus einer substituierten Aryl- oder Heteroaryl- (Ar-) Gruppe ausgewählt ist, der eine oder die mehreren Substituenten unabhängig aus -COOH, -NH₂, -COOR¹, -CONH₂, -CONHR¹, -CONR¹R², -R³ ausgewählt sind;
wobei
R¹ und R² identisch oder voneinander verschieden sind und unabhängig aus -H, geradkettigem oder verzweigtem, unsubstituiertem C₁₋₈-Alkyl, unsubstituiertem C₃₋₆-Cycloalkyl ausgewählt sind,
R³ aus geradkettigem oder verzweigtem, unsubstituiertem C₁₋₈-Alkyl, unsubstituiertem C₃₋₆-Cycloalkyl ausgewählt ist;
ein pharmazeutisch verträgliches Hydrat, Solvat oder Salz davon.

2. Verbindung gemäß Anspruch 1, wobei beliebige der R¹-, R²- und R³- Gruppen, falls vorhanden, unabhängig aus Methyl, Ethyl, n-Propyl, Isopropyl, n-Butyl, Isobutyl, tert-Butyl, Cyclopropyl, Cyclobutyl, Cyclopentyl, Cyclohexyl, n-Pentyl, n-Hexyl, n-Heptyl, n-Octyl ausgewählt sind.

3. Verbindung gemäß einem der vorangehenden Ansprüche, ausgewählt aus:
2-Brom-N-(6-(4-(pyridin-3-yl)-1 H-1,2,3-triazol-1-yl)hexyl)benzolsulfonamid
N-(6-(4-(Pyridin-3-yl)-1H-1,2,3-triazol-1-yl)hexyl)-[1,1'-biphenyl]-2-sulfonamid
2'-((6-(4-(Pyridin-3-yl)-1H-1,2,3-triazol-1-yl)hexyl)oxy)-[1,1'-biphenyl]-4-carbonsäure
2'-((6-(4-(Pyridin-3-yl)-1H-1,2,3-triazol-1-yl)hexyl)oxy)-[1,1'-biphenyl]-3-carbonsäure
2-Brom-N-(7-(4-(pyridin-3-yl)-1H-1,2,3-triazol-1-yl)heptyl)benzolsulfonamid
2-Iod-N-(7-(4-(pyridin-3-yl)-1H-1,2,3-triazol-1-yl)heptyl)benzamid
N-(7-(4-(Pyridin-3-yl)-1H-1,2,3-triazol-1-yl)heptyl)-[1,1'-biphenyl] -2-sulfonamid
2'-((7-(4-(Pyridin-3-yl)-1H-1,2,3-triazol-1-yl)heptyl)carbamoyl)-[1,1'-diphenyl]-4-carbonsäure
3-(2-((6-(4-(Pyridin-3-yl)-1H-1,2,3-triazol-1-yl)hexyl)oxy)phenyl)pyridin
2-(Pyridin-3-yl)-N-(7-(4-(pyridin-3-yl)-1H-1,2,3-triazol-1 -yl)heptyl)benzamid
3-(1-(8-([1,1'-Biphenyl]-2-yloxy)octyl)-1H-1,2,3-triazol-4-yl)pyridin
3-(1-(7-([1,1'-Biphenyl]-2-yloxy)heptyl)-1H-1,2,3-triazol-4-yl)pyridin
N-(7-(4-(Pyridin-3-yl)-1H-1,2,3-triazol-1-yl)heptyl)-[1,1'-biphenyl]-2-carboxamid
3-(1-(6-([1,1'-Biphenyl]-2-yloxy)hexyl)-1H-1,2,3-triazol-4-yl)pyridin
N-(6-(4-(Pyridin-3-yl)-1H-1,2,3-triazol-1-yl)hexyl)-[1,1-biphenyl]-2-carboxamid
2'-((7-(4-(Pyridin-3-yl)-1H-1,2,3-triazol-1-yl)heptyl)carbamoyl)-[1,1'-diphenyl]-2-carbonsäure
3-(1-(6-((2'-Methyl-[1,1'-biphenyl]-2-yl)oxy)hexyl)-1H-1,2,3-triazol-4-yl)pyridin
2'-Methyl-N-(7-(4-(pyridin-3-yl)-1H-1,2,3-triazol-1-yl)heptyl)-[1,1'-biphenyl]-2-carboxamid
N-([1,1'-Biphenyl]-2-ylmethyl)-7-(4-(pyridin-3-yl)-1H-1,2,3-triazol-1-yl)heptan-1-amin
N-([1,1'-Biphenyl]-2-ylmethyl)-6-(4-(pyridin-3-yl)-1H-1,2,3-triazol-1-yl)hexan-1-amin
1-([1,1'-Biphenyl]-2-yl)-3-(6-(4-(pyridin-3-yl)-1H-1,2,3-triazol-1-yl)hexyl)harnstoff
1-([1,1'-Biphenyl]-2-yl)-3-(7-(4-(pyridin-3-yl)-1H-1,2,3-triazol-1-yl)heptyl)harnstoff
6-(4-(Pyridin-3-yl)-1H-1,2,3-triazol-1-yl)hexyl[1,1'-biphenyl]-2-ylcarbamat
8-(4-(Pyridin-3-yl)-1H-1,2,3-triazol-1-yl)octyl[1,1'-biphenyl]-2-ylcarbamat
7-(4-(Pyridin-3-yl)-1H-1,2,3-triazol-1-yl)heptyl[1,1'-biphenyl]-2-ylcarbamat
1-([1,1'-Biphenyl]-2-yl)-3-(8-(4-(pyridin-3-yl)-1H-1,2,3-triazol-1-yl)octyl)harnstoff
N-([1,1'-Biphenyl]-2-ylmethyl)-8-(4-(pyridin-3-yl)-1H-1,2,3-triazol-1-yl)octan-1-amin
2-Brom-N-(8-(4-(pyridin-3-yl)-1H-1,2,3-triazol-1-yl)octyl)benzolsulfonamid
N-(8-(4-(Pyridin-3-yl)-1H-1,2,3-triazol-1-yl)octyl)-[1,1'-biphenyl-2-sulfonamid
3-(1-(6-(4-([1,1'-Biphenyl]-2-yl)-1H-1,2,3-triazol-1-yl)hexyl)-1H-1,2,3-triazol-4-yl)pyridin
3-(1-(7-(4-([1,1'-Biphenyl]-2-yl)-1H-1,2,3-triazol-1-yl)heptyl)-1H-1,2,3-triazol-4-yl)pyridin
3-(1-(8-(4-([1,1'-Biphenyl]-2-yl)-1H-1,2,3-triazol-1-yl)octyl)-1H-1,2,3-triazol-4-yl)pyridin
N-(4-(2-(4-(Pyridin-3-yl)-1H-1,2,3-triazol-1-yl)ethyl)benzyl)-[1,1'-biphenyl]-2-carboxamid
N-(4-((4-(Pyridin-3-yl)-1H-1,2,3-triazol-1-yl)methyl)phenethyl)-[1,1'-diphenyl]-2-carboxamid
3-(1-(4-(2-([1,1'-Biphenyl]-2-yloxy)ethyl)benzyl)-1H-1,2,3-triazol-4-yl)pyridin
3-(1-(4-(([1,1'-Biphenyl]-2-yloxy)methyl)phenethyl)-1H-1,2,3-triazol-4-yl)pyridin
N-(4-(3-(4-(Pyridin-3-yl)-1H-1,2,3-triazol-1-yl)propyl)phenyl)-[1,1'-biphenyl]-2-carboxamid
3-(1-(4-(3-([1,1'-Biphenyl]-2-yloxy)propyl)phenyl)-1 H-1,2,3-triazol-4-yl)pyridin
N-(3-(4-(4-(Pyridin-3-yl)-1H-1,2,3-triazol-1-yl)phenyl)propyl)-[1,1'-biphenyl]-2-carboxamid
1-([1,1'-Biphenyl]-2-yl)-3-(4-((4-(pyridin-3-yl)-1H-1,2,3-triazol-1-yl)methyl)phenethyl)harnstoff
3-(1-(4-(2-(4-([1,1'-Biphenyl]-2-yl)-1H-1,2,3-triazol-1-yl)ethyl)benzyl)-1H-1,2,3-triazol-4-yl)pyridin
4-((4-(Pyridin-3-yl)-1H-1,2,3-triazol-1-yl)methyl)phenethyl[1,1'-biphenyl]-2-ylcarbamat
N-([1,1'-Biphenyl]-2-ylmethyl)-2-(4-((4-(pyridin-3-yl)-1H-1,2,3-triazol-1-yl)methyl)phenyl)ethan-1-amin
2-Brom-N-(4-((4-(pyridin-3-yl)-1H-1,2,3-triazol-1-yl)methyl)phenethyl)benzolsulfonamid
N-(4-((4-(Pyridin-3-yl)-1H-1,2,3-triazol-1-yl)methyl)phenethyl)-[1,1'-diphenyl]-2-sulfonamid

4. Verbindung gemäß einem der Ansprüche 1 bis 3, zur Verwendung als therapeutisches Mittel.

5. Verbindung gemäß einem der Ansprüche 1 bis 3, zur Verwendung bei der Behandlung einer Autoimmunerkrankung, vorzugsweise ausgewählt aus Lupus erythematodes, Psoriasis, rheumatoider Arthritis, Morbus Crohn, Autoimmun-Enzephalitis, Transplantat-Wirt-Reaktion.

6. Verbindung gemäß einem der Ansprüche 1 bis 3, zur Verwendung bei der Behandlung einer akuten und/oder chronischen Entzündungskrankheit, vorzugsweise ausgewählt aus Colitis ulcerosa, Asthma, chronisch-obstruktiver Lungenerkrankung (chronic obstructive pulmonary disease, COPD), akutem Atemnotsyndrom (acute respiratory distress syndrome, ARDS), allergischer Rhinitis, Anaphylaxie, zystischer Fibrose, Myokardinfarkt, Herzversagen, ischämischen Erkrankungen und Atherosklerose, einer akuten Lungenverletzung, Rückenmarksverletzung und Sepsis.

7. Verbindung gemäß einem der Ansprüche 1 bis 3, zur Verwendung bei der Behandlung einer mit dem Stoffwechsel zusammenhängenden Erkrankung, vorzugsweise ausgewählt aus Fettleibigkeit und Typ II-Diabetes.

8. Verbindung gemäß einem der Ansprüche 1 bis 3, zur Verwendung bei der Verhütung einer neuronalen Degeneration, die durch eine Verletzung bestimmt wird, vorzugsweise ausgewählt aus einer traumatischen Rückenmarksverletzung.

9. Verbindung gemäß einem der Ansprüche 1 bis 3, zur Verwendung bei der Behandlung eines soliden Tumors, wobei der solide Tumor vorzugsweise aus Prostatakrebs, Eierstockkrebs, Lungenkrebs, Brustkrebs, Dickdarmkrebs, Pankreasadenokarzinom, Melanom und Neuroblastom ausgewählt ist.

10. Verbindung gemäß einem der Ansprüche 1 bis 3, zur Verwendung bei der Behandlung eines liquiden Tumors, wobei der liquide Tumor vorzugsweise aus Leukämie und Lymphom ausgewählt ist.

11. Pharmazeutische Zusammensetzung, umfassend wenigstens eine Verbindung gemäß einem der Ansprüche 1 bis 3 und einen pharmazeutisch verträglichen Träger und/oder ein pharmazeutisch verträgliches Vehikel.

12. Produkt, umfassend eine Verbindung gemäß einem der Ansprüche 1 bis 3 und ein Krebsmittel als Kombinationspräparat zur gleichzeitigen, getrennten oder aufeinanderfolgenden Verwendung bei der Behandlung eines Patienten, der an einem Krebs leidet.

13. Produkt zur Verwendung gemäß Anspruch 12, wobei das Krebsmittel aus DNAalkylierenden Mitteln, DNA-interkalierenden Mitteln und Topoisomerase-Inhibitoren ausgewählt ist.

14. Produkt, umfassend eine Verbindung gemäß einem der Ansprüche 1 bis 3 und einen Autophagie-Inhibitor als Kombinationspräparat hat zur gleichzeitigen, getrennten oder aufeinanderfolgenden Verwendung bei der Behandlung eines Patienten, der an einem Krebs leidet.

15. Produkt, umfassend eine Verbindung gemäß einem der Ansprüche 1 bis 3 und ein immunmodulierendes Arzneimittel oder entzündungshemmendes Arzneimittel als Kombinationspräparat zur gleichzeitigen, getrennten oder aufeinanderfolgenden Verwendung bei der Behandlung eines Patienten, der an einem Krebs oder an einer akuten oder chronischen Entzündungskrankheit leidet.

16. Produkt zur Verwendung gemäß Anspruch 14, wobei der Autophagie-Inhibitor aus Chloroquin, Hydroxychloroquin und PI3K-Modulatoren, wie GDC-0941 und PI103 ausgewählt ist.

## Revendications

1. Composé de formule (I) : 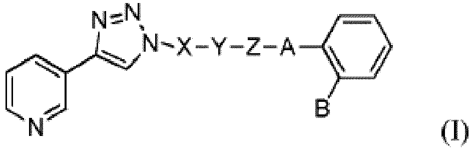 où
X représente (CH₂)ₙ ;
Y est choisi parmi (CH₂)ₘ, un phényle substitué en position *para ;*
Z représente (CH₂)ₚ ;
n est un nombre entier allant de 0 à 4 ;
m est un nombre entier allant de 1 à 8 ;
p est un nombre entier allant de 0 à 4 ;
A est choisi parmi les groupes suivants : 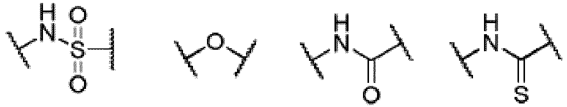 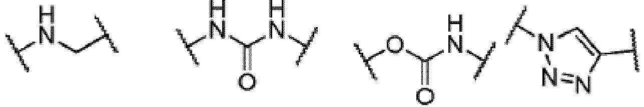
B est choisi parmi Br, Cl, I, F, un groupe méthyle, isopropyle, tert-butyle, aryle ou hétéroaryle (Ar) substitué ou non substitué,
où Ar est choisi parmi le benzène, le furanne, le thiophène, la pyrrolidine, le pyrrole, le 1,2,3-triazole, le pyrazole, l'imidazole, l'oxazole, l'isooxazole, le thiazole, l'isothiazole, le 1,2,3-oxadiazole, le 1,2,4-oxadiazole, le 1,2,5-oxadiazole, la pyridine, la pyridazine, la pyrimidine, la pyrazine, le naphtalène, l'indole, le 1*H*-indazole, le 1*H*-benzo[d]imidazole, le benzofuranne, le benzothiophène, la quinoléine, l'isoquinoléine, la quinoxaline et le carbazole, et
où, lorsque B est choisi parmi un groupe aryle ou hétéroaryle (Ar) substitué, le ou les plusieurs substituant(s) est/sont indépendamment choisi(s) parmi -COOH, -NH₂, -COOR¹, -CONH₂, -CONHR¹, -CONR¹R², -R³ ;
où
R¹ et R² sont identiques ou différents l'un de l'autre et choisis indépendamment parmi -H, un alkyle en C₁ à C₈ non substitué, linéaire ou ramifié, un cycloalkyle en C₃ à C₆ non substitué ;
R³ est choisi parmi un alkyle en C₁ à C₈ non substitué, linéaire ou ramifié, un cycloalkyle en C₃ à C₆ non substitué ;
l'un de ses hydrates, solvates ou sels pharmaceutiquement acceptables.

2. Composé selon la revendication 1, dans lequel l'un quelconque des groupes R¹, R² et R³, s'il est présent, est indépendamment choisi parmi le méthyle, l'éthyle, le n-propyle, l'isopropyle, le n-butyle, l'isobutyle, le tert-butyle, le cyclopropyle, le cyclobutyle, le cyclopentyle, le cyclohexyle, le n-pentyle, le n-hexyle ; le n-heptyle, le n-octyle.

3. Composé selon l'une quelconque des revendications précédentes, choisi parmi :
le 2-bromo-N-(6-(4-(pyridin-3-yl)-1H-1,2,3-triazol-1-yl)hexyl)benzènesulfonamide
le N-(6-(4-(pyridin-3-yl)-1H-1,2,3-triazol-1-yl)hexyl)-[1,1'-biphényl]-2-sulfonamide
l'acide 2'-((6-(4-(pyridin-3-yl)-1H-1,2,3-triazol-1-yl)hexyl)oxy)-[1,1'-biphényl]-4-carboxylique
l'acide 2'-((6-(4-(pyridin-3-yl)-1H-1,2,3-triazol-1-yl)hexyl)oxy)-[1,1'-biphényl]-3-carboxylique
le 2-bromo-N-(7-(4-(pyridin-3-yl)-1H-1,2,3-triazol-1-yl)heptyl)benzènesulfonamide
le 2-iodo-N-(7-(4-(pyridin-3-yl)-1H-1,2,3-triazol-1-yl)heptyl)benzamide
le N-(7-(4-(pyridin-3-yl)-1H-1,2,3-triazol-1-yl)heptyl)-[1,1'-biphényl]-2-sulfonamide
l'acide 2'-((7-(4-(pyridin-3-yl)-1H-1,2,3-triazol-1-yl)heptyl)carbamoyl)-[1,1-biphényl]-4-carboxylique
la 3-(2-((6-(4-(pyridin-3-yl)-1H-1,2,3-triazol-1-yl)hexyl)oxy)phényl)pyridine
le 2-(pyridin-3-yl)-N-(7-(4-(pyridin-3-yl)-1H-1,2,3-triazol-1-yl)heptyl)benzamide
la 3-(1-(8-([1,1'-biphényl]-2-yloxy)octyl)-1H-1,2,3-triazol-4-yl)pyridine
la 3-(1-(7-([1,1'-biphényl]-2-yloxy)heptyl)-1H-1,2,3-triazol-4-yl)pyridine
le N-(7-(4-(pyridin-3-yl)-1H-1,2,3-triazol-1-yl)heptyl)-[1,1'-biphényl]-2-carboxamide
la 3-(1-(6-([1,1'-biphényl]-2-yloxy)hexyl)-1H-1,2,3-triazol-4-yl)pyridine
le N-(6-(4-(pyridin-3-yl)-1H-1,2,3-triazol-1-yl)hexyl)-[1,1'-biphényl]-2-carboxamide
l'acide 2'-((7-(4-(pyridin-3-yl)-1H-1,2,3-triazol-1-yl)heptyl)carbamoyl)-[1,1'-biphényl]-2-carboxylique
la 3-(1-(6-((2'-méthyl-[1,1'-biphényl]-2-yl)oxy)hexyl)-1H-1,2,3-triazol-4-yl)pyridine
le 2'-méthyl-N-(7-(4-(pyridin-3-yl)-1H-1,2,3-triazol-1-yl)heptyl)-[1,1'-biphényl]-2-carboxamide
la N-([1,1'-biphényl]-2-ylméthyl)-7-(4-(pyridin-3-yl)-1H-1,2,3-triazol-1-yl)heptan-1-amine
la N-([1,1'-biphényl]-2-ylméthyl)-6-(4-(pyridin-3-yl)-1H-1,2,3-triazol-1-yl)hexan-1-amine
la 1-([1,1'-biphényl]-2-yl)-3-(6-(4-(pyridin-3-yl)-1H-1,2,3-triazol-1-yl)hexyl)urée
la 1-([1,1'-biphényl]-2-yl)-3-(7-(4-(pyridin-3-yl)-1H-1,2,3-triazol-1-yl)heptyl)urée
le [1,1'-biphényl]-2-ylcarbamate de 6-(4-(pyridin-3-yl)-1H-1,2,3-triazol-1-yl)hexyle
le [1,1'-biphényl]-2-ylcarbamate de 8-(4-(pyridin-3-yl)-1H-1,2,3-triazol-1-yl)octyle
le [1,1'-biphényl]-2-ylcarbamate de 7-(4-(pyridin-3-yl)-1H-1,2,3-triazol-1-yl)heptyle
la 1-([1,1'-biphényl]-2-yl)-3-(8-(4-(pyridin-3-yl)-1H-1,2,3-triazol-1-yl)octyl)urée
la N-([1,1'-biphényl]-2-ylméthyl)-8-(4-(pyridin-3-yl)-1H-1,2,3-triazol-1-yl)octan-1-amine
le 2-bromo-N-(8-(4-(pyridin-3-yl)-1H-1,2,3-triazol-1-yl)octyl)benzènesulfonamide
le N-(8-(4-(pyridin-3-yl)-1H-1,2,3-triazol-1-yl)octyl)-[1,1'-biphényl]-2-sulfonamide
la 3-(1-(6-(4-([1,1'-biphényl]-2-yl)-1H-1,2,3-triazol-1-yl)hexyl)-1H-1,2,3-triazol-4-yl)pyridine
la 3-(1-(7-(4-([1,1'-biphényl]-2-yl)-1H-1,2,3-triazol-1-yl)heptyl)-1H-1,2,3-triazol-4-yl)pyridine
la 3-(1-(8-(4-([1,1'-biphényl]-2-yl)-1H-1,2,3-triazol-1-yl)octyl)-1H-1,2,3-triazol-4-yl)pyridine
le N-(4-(2-(4-(pyridin-3-yl)-1H-1,2,3-triazol-1-yl)éthyl)benzyl)-[1,1'-biphényl]-2-carboxamide
le N-(4-((4-(pyridin-3-yl)-1H-1,2,3-triazol-1-yl)méthyl)phénéthyl)-[1,1'-biphényl]-2-carboxamide
la 3-(1-(4-(2-([1,1'-biphényl]-2-yloxy)éthyl)benzyl)-1H-1,2,3-triazol-4-yl)pyridine
la 3-(1-(4-(([1,1'-biphényl]-2-yloxy)méthyl)phénéthyl)-1H-1,2,3-triazol-4-yl)pyridine
le N-(4-(3-(4-(pyridin-3-yl)-1H-1,2,3-triazol-1-yl)propyl)phényl)-[1,1'-biphényl]-2-carboxamide
la 3-(1-(4-(3-([1,1'-biphényl]-2-yloxy)propyl)phényl)-1H-1,2,3-triazol-4-yl)pyridine
le N-(3-(4-(4-(pyridin-3-yl)-1H-1,2,3-triazol-1-yl)phényl)propyl)-[1,1'-biphényl]-2-carboxamide
la 1-([1,1'-biphényl]-2-yl)-3-(4-((4-(pyridin-3-yl)-1H-1,2,3-triazol-1-yl)méthyl)phénéthyl)urée
la 3-(1-(4-(2-(4-([1,1'-biphényl]-2-yl)-1H-1,2,3-triazol-1-yl)éthyl)benzyl)-1 H-1,2,3-triazol-4-yl)pyridine
le [1,1'-biphényl]-2-ylcarbamate de 4-((4-(pyridin-3-yl)-1H-1,2,3-triazol-1-yl)méthyl)phénéthyle
la N-([1,1'-biphényl]-2-ylméthyl)-2-(4-((4-(pyridin-3-yl)-1H-1,2,3-triazol-1-yl)méthyl)phényl)éthan-1-amine
le 2-bromo-N-(4-((4-(pyridin-3-yl)-1H-1,2,3-triazol-1-yl)méthyl)phénéthyl)benzènesulfonamide
le N-(4-((4-(pyridin-3-yl)-1H-1,2,3-triazol-1-yl)méthyl)phénéthyl)-[1,1'-biphényl]-2-sulfonamide.

4. Composé selon l'une quelconque des revendications 1 à 3, pour une utilisation en tant qu'agent thérapeutique.

5. Composé selon l'une quelconque des revendications 1 à 3, pour une utilisation dans le traitement d'une maladie auto-immune, de préférence choisie parmi le lupus érythémateux, le psoriasis, la polyarthrite rhumatoïde, la maladie de Crohn, l'encéphalite auto-immune, la réaction du greffon contre l'hôte.

6. Composé selon l'une quelconque des revendications 1 à 3, pour une utilisation dans le traitement d'un trouble inflammatoire aigu et/ou chronique, de préférence choisi parmi la rectocolite hémorragique, l'asthme, la bronchopneumopathie chronique obstructive (BPCO), le syndrome de détresse respiratoire aiguë (SDRA), la rhinite allergique, l'anaphylaxie, la fibrose kystique, l'infarctus du myocarde, l'insuffisance cardiaque, les maladies ischémiques et l'athérosclérose, une lésion pulmonaire aiguë, une lésion de la moelle épinière et la sepsie.

7. Composé selon l'une quelconque des revendications 1 à 3, pour une utilisation dans le traitement d'un trouble associé au métabolisme, de préférence choisi parmi l'obésité et le diabète de type II.

8. Composé selon l'une quelconque des revendications 1 à 3, pour une utilisation dans la prévention de la dégénérescence neuronale déterminée par une lésion, de préférence choisie parmi une lésion traumatique de la moelle épinière.

9. Composé selon l'une quelconque des revendications 1 à 3, pour une utilisation dans le traitement d'une tumeur solide, dans lequel ladite tumeur solide est de préférence choisie parmi le cancer de la prostate, le cancer de l'ovaire, le cancer du poumon, le cancer du sein, le cancer du côlon, l'adénocarcinome pancréatique, un mélanome et un neuroblastome.

10. Composé selon l'une quelconque des revendications 1 à 3, pour une utilisation dans le traitement d'une tumeur liquide, dans lequel ladite tumeur liquide est de préférence choisie parmi une leucémie et un lymphome.

11. Composition pharmaceutique comprenant au moins un composé selon l'une quelconque des revendications 1 à 3 et un support et/ou véhicule pharmaceutiquement acceptable(s).

12. Produit comprenant un composé selon l'une quelconque des revendications 1 à 3 et un agent anticancéreux en tant que préparation combinée pour une utilisation simultanée, séparée ou séquentielle dans le traitement d'un patient souffrant d'un cancer.

13. Produit pour une utilisation selon la revendication 12, dans lequel l'agent anticancéreux est choisi parmi des agents d'alkylation d'ADN, des agents intercalants d'ADN et des inhibiteurs de la topoisomérase.

14. Produit comprenant un composé selon l'une quelconque des revendications 1 à 3 et un inhibiteur de l'autophagie en tant que préparation combinée pour une utilisation simultanée, séparée ou séquentielle dans le traitement d'un patient souffrant d'un cancer.

15. Produit comprenant un composé selon l'une quelconque des revendications 1 à 3 et un médicament immunomodulateur ou un médicament anti-inflammatoire en tant que préparation combinée pour une utilisation simultanée, séparée ou séquentielle dans le traitement d'un patient souffrant d'un cancer ou d'une maladie inflammatoire chronique ou aiguë.

16. Produit pour une utilisation selon la revendication 14, dans lequel l'inhibiteur de l'autophagie est choisi parmi la chloroquine, l'hydroxychloroquine et des modulateurs de PI3K, tels que GDC-0941 et PI103.
